(19) Europäisches Patentamt
European Patent Office
Office européen des brevets

(11) EP 4 508 045 B1

(12) **EUROPEAN PATENT SPECIFICATION**

(45) Date of publication and mention of the grant of the patent:
**01.10.2025 Bulletin 2025/40**

(21) Application number: **23757194.8**

(22) Date of filing: **03.08.2023**

(51) International Patent Classification (IPC):
***C07D 401/14*** (2006.01) ***A61P 25/00*** (2006.01)
***A61P 25/04*** (2006.01) ***A61P 25/16*** (2006.01)
***A61P 25/18*** (2006.01) ***A61P 25/28*** (2006.01)
***A61K 31/454*** (2006.01)

(52) Cooperative Patent Classification (CPC):
**C07D 401/14; A61P 25/00; A61P 25/04; A61P 25/16; A61P 25/18; A61P 25/28**

(86) International application number:
**PCT/EP2023/071591**

(87) International publication number:
**WO 2024/028458 (08.02.2024 Gazette 2024/06)**

(54) **CITRATE SALT FORMS OF A MUSCARINIC RECEPTOR AGONIST**

CITRATSALZFORMEN EINES MUSKARINREZEPTORAGONISTEN

FORMES DE SEL CITRATE D’UN AGONISTE DU RÉCEPTEUR MUSCARINIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**MA MD TN**

(30) Priority: **04.08.2022 GB 202211399**
**13.04.2023 GB 202305444**
**26.06.2023 GB 202309615**

(43) Date of publication of application:
**19.02.2025 Bulletin 2025/08**

(60) Divisional application:
**25198643.6**

(73) Proprietor: **Nxera Pharma UK Limited**
**Cambridge, Cambridgeshire CB21 6DG (GB)**

(72) Inventors:
- **BROWN, Giles Albert**
  **Cambridge Cambridgeshire CB21 6DG (GB)**
- **MCGEE, Paul**
  **Cambridge Cambridgeshire CB21 6DG (GB)**
- **CANSFIELD, Julie**
  **Cambridge Cambridgeshire CB21 6DG (GB)**
- **PICKWORTH, Mark**
  **Cambridge Cambridgeshire CB21 6DG (GB)**

(74) Representative: **Mewburn Ellis LLP**
**Aurora Building**
**Counterslip**
**Bristol BS1 6BX (GB)**

(56) References cited:
**WO-A1-2014/025976 WO-A1-2015/118342**

- **HILFIKER R (EDITOR) ED - HILFIKER R: "Polymorphism in the Pharmaceutical Industry", 1 January 2006, 20060101, PAGE(S) 1 - 19, ISBN: 978-3-527-31146-0, XP002528052**
- **MINO R CAIRA ED - MONTCHAMP JEAN-LUC: "Crystalline Polymorphism of Organic Compounds", TOPICS IN CURRENT CHEMISTRY; [TOPICS IN CURRENT CHEMISTRY], SPRINGER, BERLIN, DE, vol. 198, 1 January 1998 (1998-01-01), pages 163 - 208, XP008166276, ISSN: 0340-1022, [retrieved on 19990226], DOI: 10.1007/3-540-69178-2_5**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 508 045 B1

- EVAN A THACKABERRY: "Non-clinical toxicological considerations for pharmaceutical salt selection", EXPERT OPINION ON DRUG METABOLISM & TOXICOLOGY, vol. 8, no. 11, 6 September 2012 (2012-09-06), GB, pages 1419 - 1433, XP055497267, ISSN: 1742-5255, DOI: 10.1517/17425255.2012.717614

## Description

### Technical Field

This invention relates to salts that are muscarinic receptor agonists and which are useful in the treatment of muscarinic receptor mediated diseases. Also provided are crystalline forms of the salts; pharmaceutical compositions containing the salts, or crystalline forms thereof; therapeutic uses of salts, or crystalline forms thereof; methods of synthesis thereof; and intermediates useful in said methods of synthesis.

### Background

Muscarinic acetylcholine receptors (mAChRs) are members of the G protein-coupled receptor superfamily which mediate the actions of the neurotransmitter acetylcholine in both the central and peripheral nervous system. Five mAChR subtypes have been cloned, M1 to M5. The M1 mAChR is predominantly expressed post-synaptically in the cortex, hippocampus, striatum and thalamus; M2 mAChRs are located predominantly in the brainstem and thalamus, though also in the cortex, hippocampus and striatum where they reside on cholinergic synaptic terminals (Langmead et al., 2008 Br J Pharmacol). However, M2 mAChRs are also expressed peripherally on cardiac tissue (where they mediate the vagal innervation of the heart) and in smooth muscle and exocrine glands. M3 mAChRs are expressed at relatively low level in the CNS but are widely expressed in smooth muscle and glandular tissues such as sweat and salivary glands (Langmead et al., 2008 Br J Pharmacol).

Muscarinic receptors in the central nervous system, especially the M1 mAChR, play a critical role in mediating higher cognitive processing. Diseases associated with cognitive impairments, such as Alzheimer's disease, are accompanied by loss of cholinergic neurons in the basal forebrain (Whitehouse et al., 1982 Science). In schizophrenia, which is also characterised by cognitive impairments, mAChR density is reduced in the pre-frontal cortex, hippocampus and caudate putamen of schizophrenic subjects (Dean et al., 2002 Mol Psychiatry). Furthermore, in animal models, blockade or lesion of central cholinergic pathways results in profound cognitive deficits and non-selective mAChR antagonists have been shown to induce psychotomimetic effects in psychiatric patients. Cholinergic replacement therapy has largely been based on the use of acetylcholinesterase inhibitors to prevent the breakdown of endogenous acetylcholine. These compounds have shown efficacy versus symptomatic cognitive decline in the clinic, but give rise to dose-limiting side effects resulting from stimulation of peripheral M2 and M3 mAChRs including disturbed gastrointestinal motility, bradycardia, nausea and vomiting (http://www.drugs.com/pro/donepezil.html; http://www.drugs.com/pro/rivastigmine.html).

Further discovery efforts have targeted the identification of direct M1 mAChR agonists to target increases in cognitive function. Such efforts resulted in the identification of a range of agonists, exemplified by compounds such as xanomeline, AF267B, sabcomeline, milameline and cevimeline. Many of these compounds have been shown to be highly effective in pre-clinical models of cognition in both rodents and / or non-human primates. Milameline has shown efficacy versus scopolamine-induced deficits in working and spatial memory in rodents; sabcomeline displayed efficacy in a visual object discrimination task in marmosets and xanomeline reversed mAChR antagonist-induced deficits in cognitive performance in a passive avoidance paradigm.

Alzheimer's disease (AD) is the most common neurodegenerative disorder (26.6 million people worldwide in 2006) that affects the elderly, resulting in profound memory loss and cognitive dysfunction. The aetiology of the disease is complex, but is characterised by two hallmark brain sequelae: aggregates of amyloid plaques, largely composed of amyloid-β peptide (Aβ), and neurofibrillary tangles, formed by hyperphosphorylated tau proteins. The accumulation of Aβ is thought to be the central feature in the progression of AD and, as such, many putative therapies for the treatment of AD are currently targeting inhibition of Aβ production. Aβ is derived from proteolytic cleavage of the membrane bound amyloid precursor protein (APP). APP is processed by two routes, non-amyloidgenic and amyloidgenic. Cleavage of APP by γ-secretase is common to both pathways, but in the former APP is cleaved by an α-secretase to yield soluble APPα. The cleavage site is within the Aβ sequence, thereby precluding its formation. However, in the amyloidgenic route, APP is cleaved by β-secretase to yield soluble APPβ and also Aβ. In vitro studies have shown that mAChR agonists can promote the processing of APP toward the soluble, non-amyloidogenic pathway. In vivo studies showed that the mAChR agonist, AF267B, altered disease-like pathology in the 3xTgAD transgenic mouse, a model of the different components of Alzheimer's disease (Caccamo et al., 2006 Neuron). Finally, the mAChR agonist cevimeline has been shown to give a small, but significant, reduction in cerebrospinal fluid levels of Aβ in Alzheimer's patients, thus demonstrating potential disease modifying efficacy (Nitsch et al., 2000 Neurol).

Furthermore, preclinical studies have suggested that mAChR agonists display an atypical antipsychotic-like profile in a range of pre-clinical paradigms. The mAChR agonist, xanomeline, reverses a number of dopamine driven behaviours, including amphetamine induced locomotion in rats, apomorphine induced climbing in mice, dopamine agonist driven turning in unilateral 6-OH-DA lesioned rats and amphetamine induced motor unrest in monkeys (without EPS liability). It also has been shown to inhibit A10, but not A9, dopamine cell firing and conditioned avoidance and induces c-fos expression in prefrontal cortex and nucleus accumbens, but not in striatum in rats. These data are all suggestive of an atypical antipsychotic-like profile (Mirza et al., 1999 CNS Drug Rev). Muscarinic receptors have also been implicated in the neurobiology of addiction. The reinforcing effects of cocaine and other addictive substances are mediated by the mesolimbic dopamine system where behavioural and neurochemical studies have shown that the cholinergic muscarinic receptor subtypes play important roles in regulation of dopaminergic neurotransmission. For example M(4) (-/-) mice demonstrated significantly enhanced reward driven behaviour as result of exposure to cocaine (Schmidt et al Psychopharmacology (2011) Aug;216(3):367-78). Furthermore, xanomeline has been demonstrated to block the effects of cocaine in these models.

Muscarinic receptors are also involved in the control of movement and potentially represent novel treatments for movement disorders such as Parkinson's disease, ADHD, Huntingdon's disease, Tourette's syndrome and other syndromes associated with dopaminergic dysfunction as an underlying pathogenetic factor driving disease.

Xanomeline, sabcomeline, milameline and cevimeline have all progressed into various stages of clinical development for the treatment of Alzheimer's disease and/or schizophrenia. Phase II clinical studies with xanomeline demonstrated its efficacy versus various cognitive symptom domains, including behavioural disturbances and hallucinations associated with Alzheimer's disease (Bodick et al., 1997 Arch Neurol). This compound was also assessed in a small Phase II study of schizophrenics and gave a significant reduction in positive and negative symptoms when compared to placebo control (Shekhar et al., 2008 Am J Psych). However, in all clinical studies xanomeline and other related mAChR agonists have displayed an unacceptable safety margin with respect to cholinergic side effects, including nausea, gastrointestinal pain, diarrhoea, diaphoresis (excessive sweating), hypersalivation (excessive salivation), syncope and bradycardia.

Muscarinic receptors are involved in central and peripheral pain. Pain can be divided into three different types: acute, inflammatory, and neuropathic. Acute pain serves an important protective function in keeping the organism safe from stimuli that may produce tissue damage however management of post-surgical pain is required. Inflammatory pain may occur for many reasons including tissue damage, autoimmune response, and pathogen invasion and is triggered by the action of inflammatory mediators such as neuropeptides and prostaglandins which result in neuronal inflammation and pain. Neuropathic pain is associated with abnormal painful sensations to non-painful stimuli. Neuropathic pain is associated with a number of different diseases/traumas such as spinal cord injury, multiple sclerosis, diabetes (diabetic neuropathy), viral infection (such as HIV or Herpes). It is also common in cancer both as a result of the disease or a side effect of chemotherapy. Activation of muscarinic receptors has been shown to be analgesic across a number of pain states through the activation of receptors in the spinal cord and higher pain centres in the brain. Increasing endogenous levels of acetylcholine through acetylcholinesterase inhibitors, direct activation of muscarinic receptors with agonists or allosteric modulators has been shown to have analgesic activity. In contrast blockade of muscarinic receptors with antagonists or using knockout mice increases pain sensitivity. Evidence for the role of the M1 receptor in pain is reviewed by D. F. Fiorino and M. Garcia-Guzman, 2012.

More recently, a small number of compounds have been identified which display improved selectivity for the M1 mAChR subtype over the peripherally expressed mAChR subtypes (Bridges et al., 2008 Bioorg Med Chem Lett; Johnson et al., 2010 Bioorg Med Chem Lett; Budzik et al., 2010 ACS Med Chem Lett). Despite increased levels of selectivity versus the M3 mAChR subtype, some of these compounds retain significant agonist activity at both this subtype and the M2 mAChR subtype. Herein we describe compounds which display high levels of selectivity for the M1 and/or M4 mAChR over the M2 and M3 receptor subtypes.

WO2015/118342 discloses muscarinic agonist compounds.

## Summary

A first aspect of the invention is a citrate salt of a compound according to formula (1):

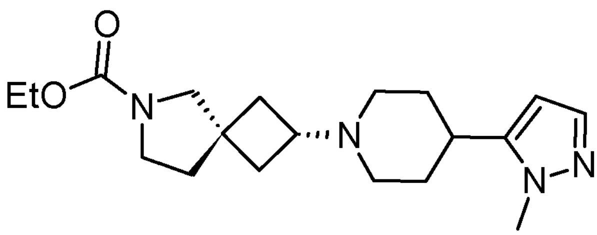

(1).

In one embodiment, the citrate salt is a citrate monohydrate salt of formula (4b):

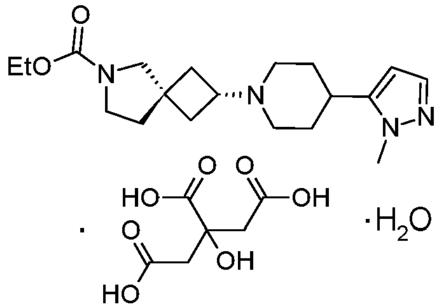

(4b).

In one embodiment, the citrate salt is a compound of formula (3b):

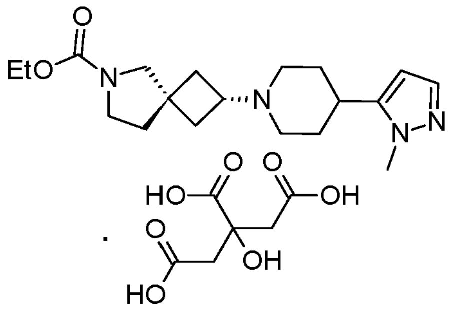

(3b).

A second aspect of the invention is a pharmaceutical composition comprising a citrate salt as defined in the first aspect, and a pharmaceutically acceptable excipient.

A third aspect of the invention is the citrate salt according to the first aspect; or the composition according to the second aspect for use in medicine.

A fourth aspect of the invention is the citrate salt according to the first aspect; or the composition according to the second aspect; for use in the treatment of a cognitive disorder or psychotic disorder or for the treatment or lessening the severity of acute, chronic, neuropathic, or inflammatory pain.

In one embodiment, the disorder is Alzheimer's disease, dementia with Lewy bodies or schizophrenia.

A fifth aspect of the invention is a crystalline form of a salt of ethyl cis-2-[4-(1-methyl-1H-pyrazol-5-yl)piperidin-1-yl]-6-azaspiro[3.4]octane-6-carboxylate, wherein:

said salt of ethyl cis-2-[4-(1-methyl-1H-pyrazol-5-yl)piperidin-1-yl]-6-azaspiro[3.4]octane-6-carboxylate is a citrate salt of ethyl cis-2-[4-(1-methyl-1H-pyrazol-5-yl)piperidin-1-yl]-6-azaspiro[3.4]octane-6-carboxylate.

In one embodiment, said citrate salt of ethyl cis-2-[4-(1-methyl-1H-pyrazol-5-yl)piperidin-1-yl]-6-azaspiro[3.4]octane-6-carboxylate is ethyl cis-2-[4-(1-methyl-1H-pyrazol-5-yl)piperidin-1-yl]-6-azaspiro[3.4]octane-6-carboxylate citrate hydrate.

In one embodiment, said citrate salt of ethyl cis-2-[4-(1-methyl-1H-pyrazol-5-yl)piperidin-1-yl]-6-azaspiro[3.4]octane-6-carboxylate is ethyl cis-2-[4-(1-methyl-1H-pyrazol-5-yl)piperidin-1-yl]-6-azaspiro[3.4]octane-6-carboxylate citrate monohydrate.

In one embodiment, the crystalline is characterised by three XRPD peaks, in terms of 2-theta, at:

$$11.3° \pm 0.2°,\ 19.2° \pm 0.2°,\ \text{and}\ 20.5° \pm 0.2°.$$

In one embodiment, the crystalline is characterised by three XRPD peaks, in terms of 2-theta, at:

$$11.3° \pm 0.2°,\ 18.4° \pm 0.2°,\ \text{and}\ 20.5° \pm 0.2°.$$

In one embodiment, the crystalline is characterised by three XRPD peaks, in terms of 2-theta, at:

$$11.3° \pm 0.2°,\ 18.4° \pm 0.2°,\ \text{and}\ 19.2° \pm 0.2°.$$

In one embodiment, the crystalline is characterised by three XRPD peaks, in terms of 2-theta, at:

18.4°± 0.2°, 19.2°± 0.2°, and 20.5°± 0.2°.

In one embodiment, the crystalline is characterised by four XRPD peaks, in terms of 2-theta, at 11.3°± 0.2°, 18.4°± 0.2°, 19.2°± 0.2°, and 20.5°± 0.2°.
In one embodiment, the crystalline is characterised by an XRPD spectrum as shown in Figure 25.
In one embodiment, the crystalline is characterised by a DSC thermogram having an endothermic peak with onset at 176 °C.
In one embodiment, the crystalline is characterised by a DSC thermogram as shown in Figure 26.
In one embodiment, the crystalline is characterised by a TGA thermogram with a weight loss of 3.3% loss from room temperature to 110 °C.
In one embodiment, the crystalline is characterised by a TGA thermogram as shown in Figure 26.
In one embodiment, said citrate salt of ethyl cis-2-[4-(1-methyl-1*H*-pyrazol-5-yl)piperidin-1-yl]-6-azaspiro[3.4]octane-6-carboxylate is ethyl cis-2-[4-(1-methyl-1*H*-pyrazol-5-yl)piperidin-1-yl]-6-azaspiro[3.4]octane-6-carboxylate citrate.
In one embodiment, the crystalline form has:
at least one characterisation selected from the group consisting of:

(1) an XRPD spectrum as shown in Figure 1;
(2) a DTA thermogram having an endothermic peak with onset at 174 °C; and
(3) a TGA thermogram with a weight loss of 33% loss from 140 °C to 220 °C.

In one embodiment, the crystalline form has:
at least one characterisation selected from the group consisting of:

(1) an XRPD spectrum as shown in Figure 1;
(2) a DTA thermogram as shown in Figure 2; and
(3) a TGA thermogram as shown in Figure 2.

In one embodiment, the crystalline form has characterisation (1).
In one embodiment, the crystalline form has characterisation (2).
In one embodiment, the crystalline form has characterisation (3).
In one embodiment, the crystalline form has characterisations (1) and (2).
In one embodiment, the crystalline form has characterisations (1) and (3).
In one embodiment, crystalline form has characterisations (2) and (3).
In one embodiment, the crystalline form has characterisations (1), (2) and (3).
A sixth aspect of the invention is a pharmaceutical composition comprising a crystalline form according to the fifth aspect, and a pharmaceutically acceptable excipient.
A seventh aspect of the invention is a citrate salt according to the first aspect, or a crystalline form according to the fifth aspect, or a pharmaceutical composition according to the second aspect or sixth aspect, for use in the treatment of a cognitive disorder or psychotic disorder.
In one embodiment, the cognitive disorder or psychotic disorder is, comprises, arises from or is associated with a condition selected from:
cognitive impairment, Mild Cognitive Impairment (MCI), frontotemporal dementia, vascular dementia, dementia with Lewy bodies, presenile dementia, senile dementia, Friedreich's ataxia, Down's syndrome, Huntington's chorea, hyperkinesia, mania, Tourette's syndrome, Alzheimer's disease , progressive supranuclear palsy, impairment of cognitive functions including attention, orientation, learning disorders, memory and language function; cognitive impairment as a result of stroke, Huntington's disease, Pick disease, AIDS-related dementia or other dementia states such as multi-infarct dementia, alcoholic dementia, hypothyroidism-related dementia, and dementia associated to other degenerative disorders such as cerebellar atrophy and amyotrophic lateral sclerosis; other acute or sub-acute conditions that may cause cognitive decline such as delirium or depression, trauma, head trauma, age related cognitive decline, stroke, neurodegeneration, drug-induced states, neurotoxic agents, age related cognitive impairment, autism related cognitive impairment, Down's syndrome, cognitive deficit related to psychosis, and post-electroconvulsive treatment related cognitive disorders; cognitive disorders due to drug abuse or drug withdrawal including nicotine, cannabis, amphetamine, cocaine, Attention Deficit Hyperactivity Disorder (ADHD) and dyskinetic disorders such as Parkinson's disease, neuroleptic-induced parkinsonism, and tardive dyskinesias, schizophrenia, schizophreniform diseases, psychotic depression, mania, acute mania, paranoid, hallucinogenic and delusional disorders, personality disorders, obsessive compulsive disorders, schizotypal disorders, delusional disorders, psychosis due to malignancy, metabolic disorder, endocrine disease or narcolepsy, psychosis due to drug abuse or drug withdrawal, bipolar disorders, schizo-affective disorder, and Alzheimer's

psychosis.

In one embodiment, the treatment is treatment of Schizophrenia.

In one embodiment, the treatment is treatment of bipolar disorders.

In one embodiment, the treatment is treatment of Alzheimer's psychosis.

## Brief Description

Described herein are compounds having selective activity as muscarinic M1 and/or M4 receptor agonists. More particularly, described herein are compounds that exhibit selectivity for the M4 receptor relative to the M2 and M3 receptor subtypes.

Accordingly, provided herein is a citrate salt of a compound of the formula (1):

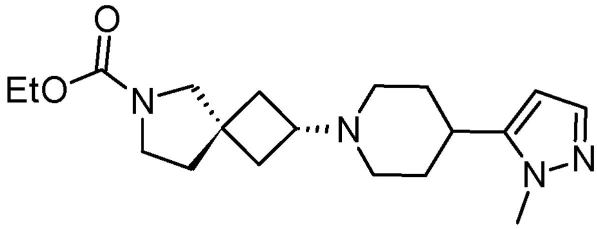

(1).

Further provided is a compound of the formula (3b):

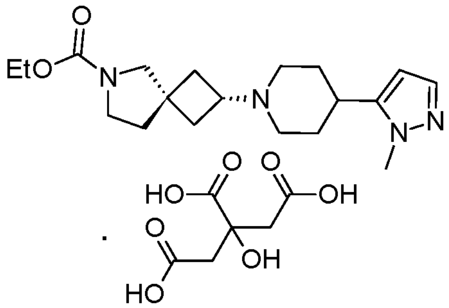

(3b).

Further provided is a compound of the formula (4b):

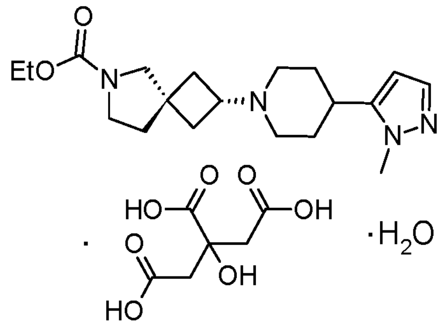

(4b).

The compound of formula (1) can form a citrate monohydrate salt.

The compound can be ethyl (2r,4s)-2-[4-(1-methyl-1*H*-pyrazol-5-yl)piperidin-1-yl]-6-azaspiro[3.4]octane-6-carboxylate citrate.

The compound can be ethyl (2r,4s)-2-[4-(1-methyl-1*H*-pyrazol-5-yl)piperidin-1-yl]-6-azaspiro[3.4]octane-6-carboxylate citrate monohydrate.

Also described herein are methods of synthesis, including methods of synthesis which preferentially favour formation of compounds and intermediates with desired stereochemistry. For example, the method may employ stereoselective reduction of a ketone to afford an alcohol or stereoselective reductive amination of a ketone.

Described herein is a method of synthesis according to the following general scheme:

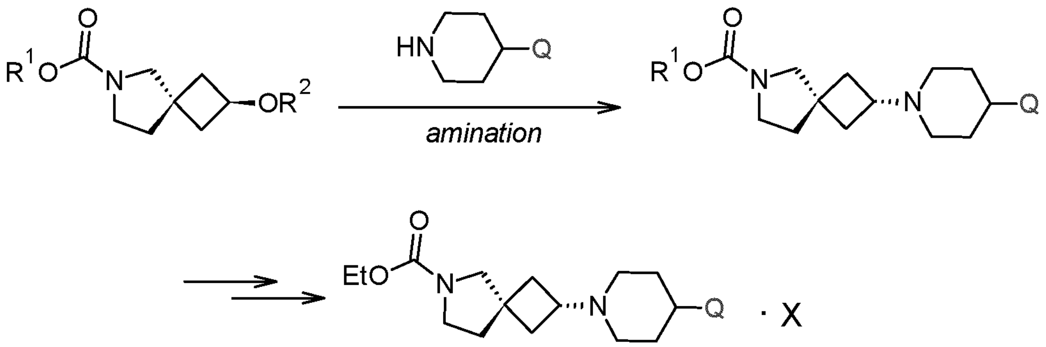


wherein Q is selected from:

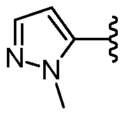; 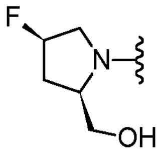; and 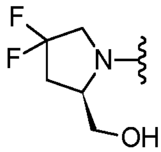;

$R^1$ is a $C_{1-6}$ linear or branched alkyl group or a $C_{3-6}$ cycloalkyl group;
$R^2$ together with the oxygen atom to which it is attached represents any suitable leaving group;
and X denotes a salt.

Compounds described herein may be prepared according to the following general scheme:

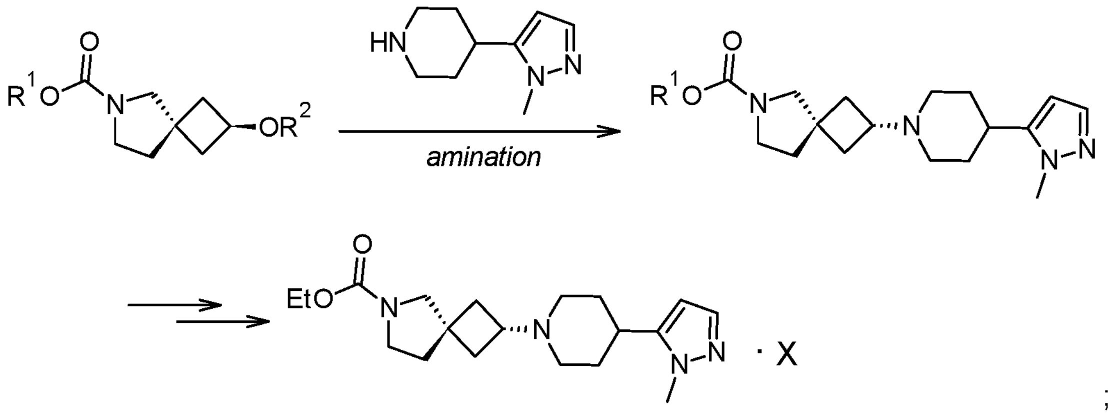


;

wherein $R^1$ is a $C_{1-6}$ linear or branched alkyl group or a $C_{3-6}$ cycloalkyl group, and $R^2$ together with the oxygen atom to which it is attached represents any suitable leaving group.

Also described herein is a method of synthesis according to the following general scheme:

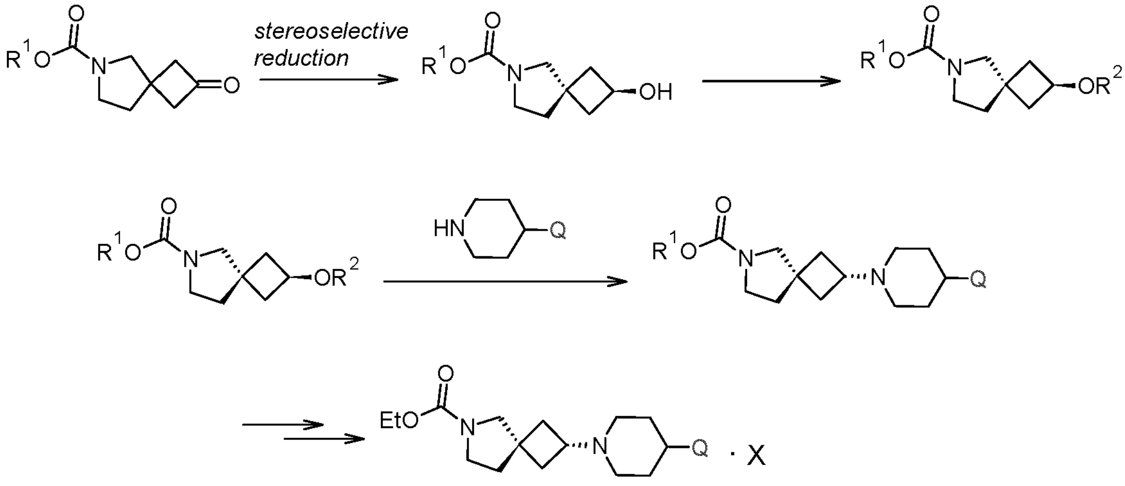


wherein Q is selected from:

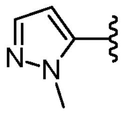 ; 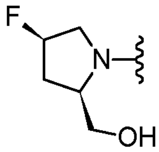 ; and 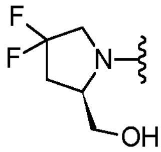 ;

$R^1$ is a $C_{1-6}$ linear or branched alkyl group or a $C_{3-6}$ cycloalkyl group;
$R^2$ together with the oxygen atom to which it is attached represents any suitable leaving group;
and X denotes a salt.

Compounds described herein may be prepared according to the following general scheme:

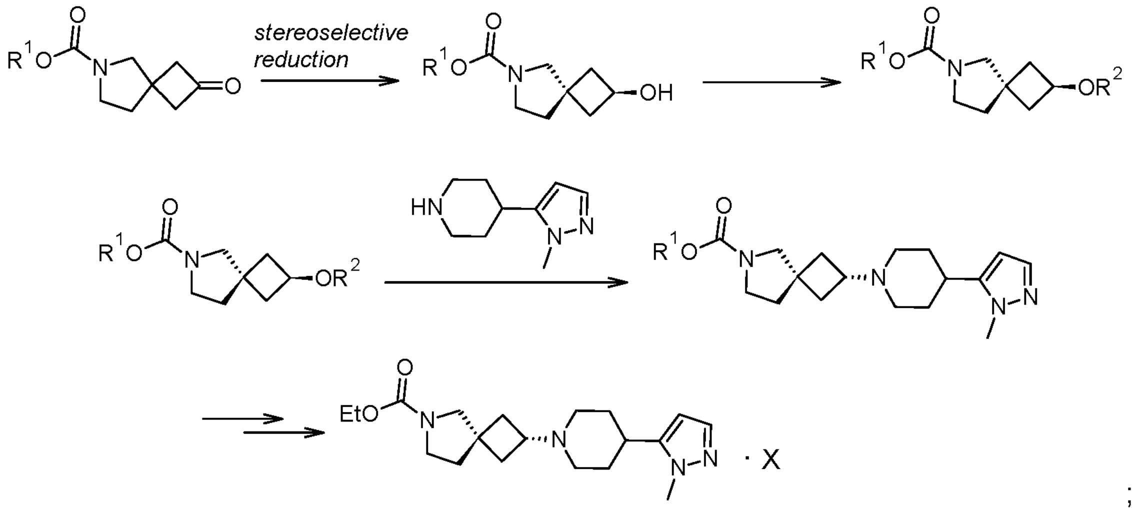


;

wherein $R^1$ is a $C_{1-6}$ linear or branched alkyl group or a $C_{3-6}$ cycloalkyl group;

$R^2$ together with the oxygen atom to which it is attached represents any suitable leaving group;
and X denotes a salt.

Also described herein is a method of synthesis according to the following general scheme:

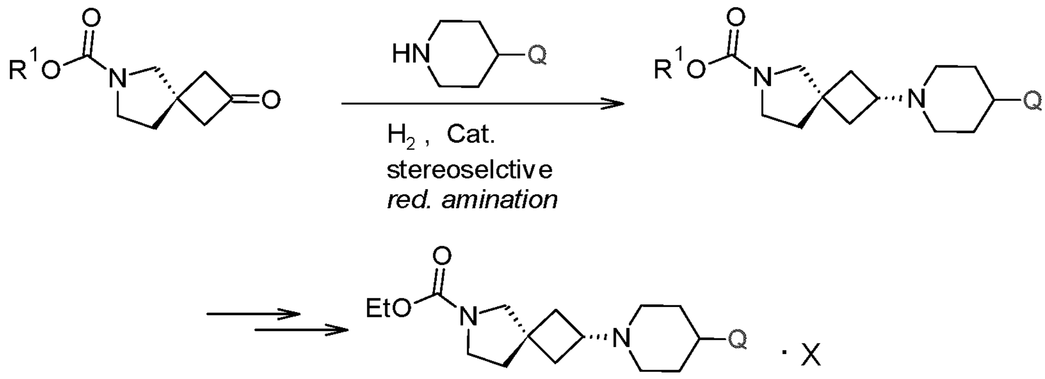


wherein Q is selected from:

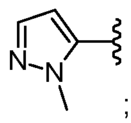 ; 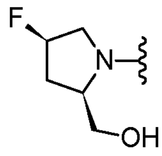 ; and 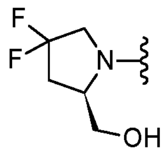 ;

$R^1$ is a $C_{1-6}$ linear or branched alkyl group or a $C_{3-6}$ cycloalkyl group;
Cat. is a hydrogenation catalyst;
and X denotes a salt.

Compounds described herein may be prepared according to the following general scheme:

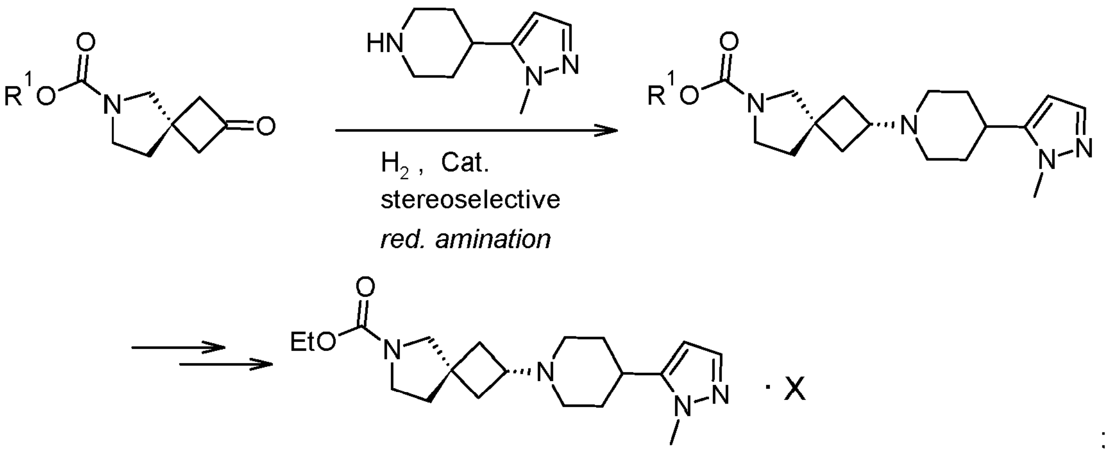


:

wherein $R^1$ is a $C_{1-6}$ linear or branched alkyl group or a $C_{3-6}$ cycloalkyl group;

Cat. is a hydrogenation catalyst;
and X denotes a salt.

In the methods described hererin, Q can be

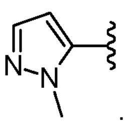

.

Q can be

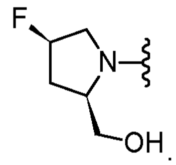

Q can be

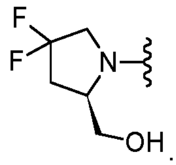

Particular methods include compounds where Q is

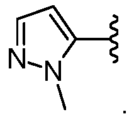

In the methods described herein, $R^1$ is a $C_{1-6}$ linear or branched alkyl group or a $C_{3-6}$ cycloalkyl group. $R^1$ may be ethyl or t-butyl. $R^1$ may be t-butyl. $R^1$ may be ethyl.

In the methods described herein $R^2$ together with the oxygen atom to which it is attached represents any suitable leaving group. $R^2$ may be tosyl.

In the methods described herein Cat. represents a hydrogenation catalyst. Cat. may be any hydrogenation catalyst capable of catalysing the transformation indicated in the relevant synthetic scheme. Cat. may be $Pd/BaSO_4$. Cat. may be $Pd/BaSO_4$ (10 mol%).

***JBN/008686743 - EP* 23757194.8 *- Amended Description filed 31 Mar 2025***

In the methods described herein, X denotes a salt. X can be a pharmaceutically acceptable salt. X can be an acid addition salt. X can be a citrate salt. X can be citrate monohydrate salt.

The compound described herein may be prepared according to the following scheme:

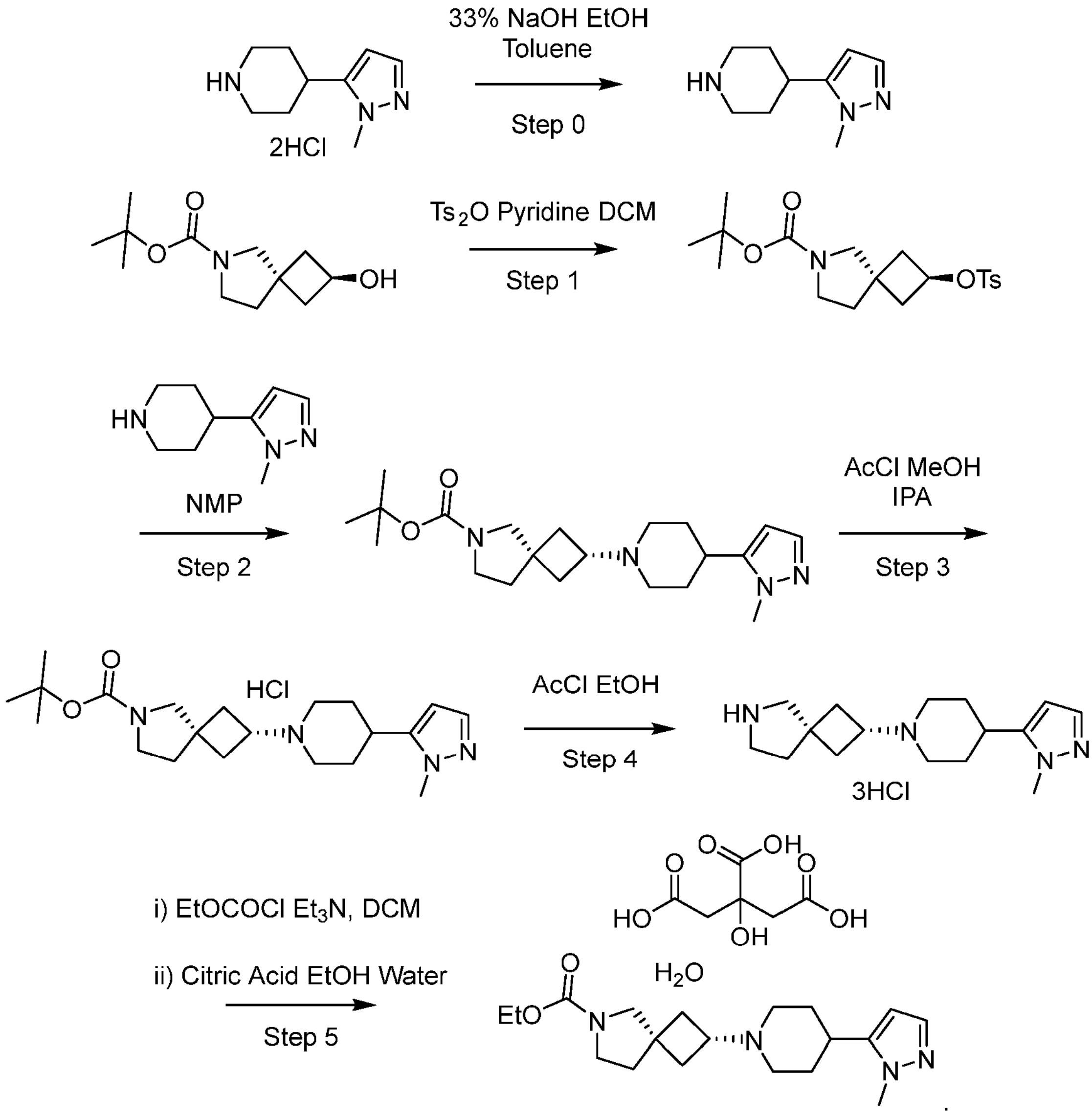


The compound described herein may be prepared according to the following scheme:

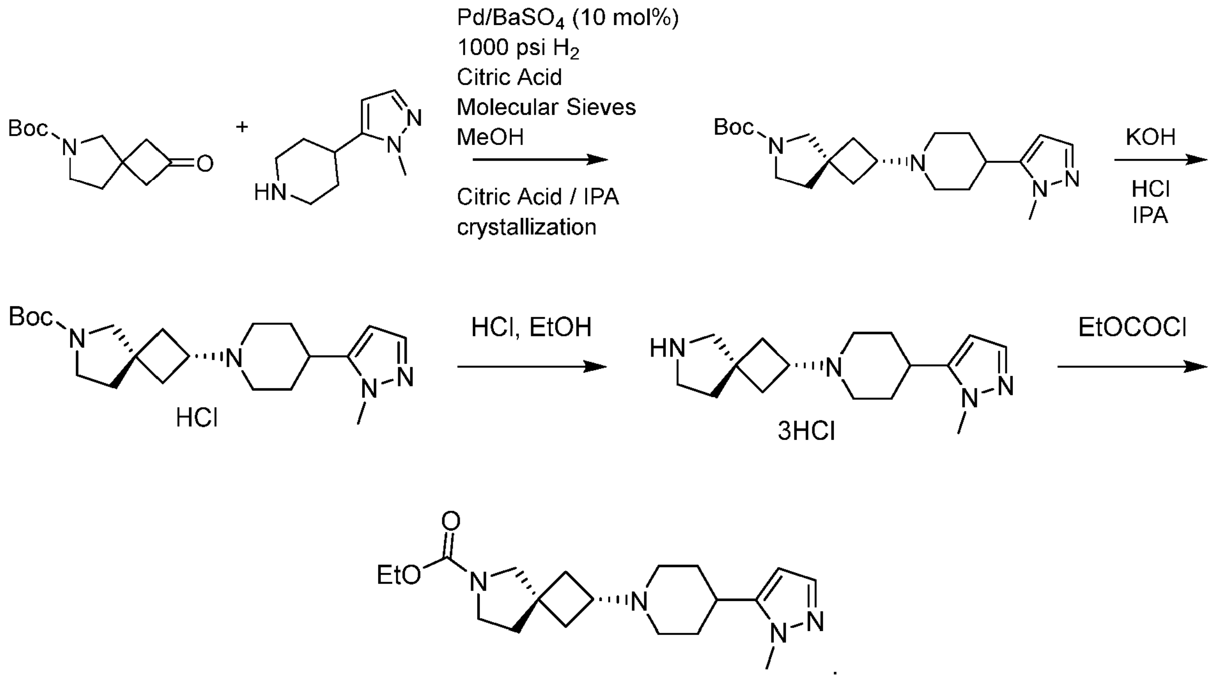


The compound described herein may be prepared according to the following scheme:

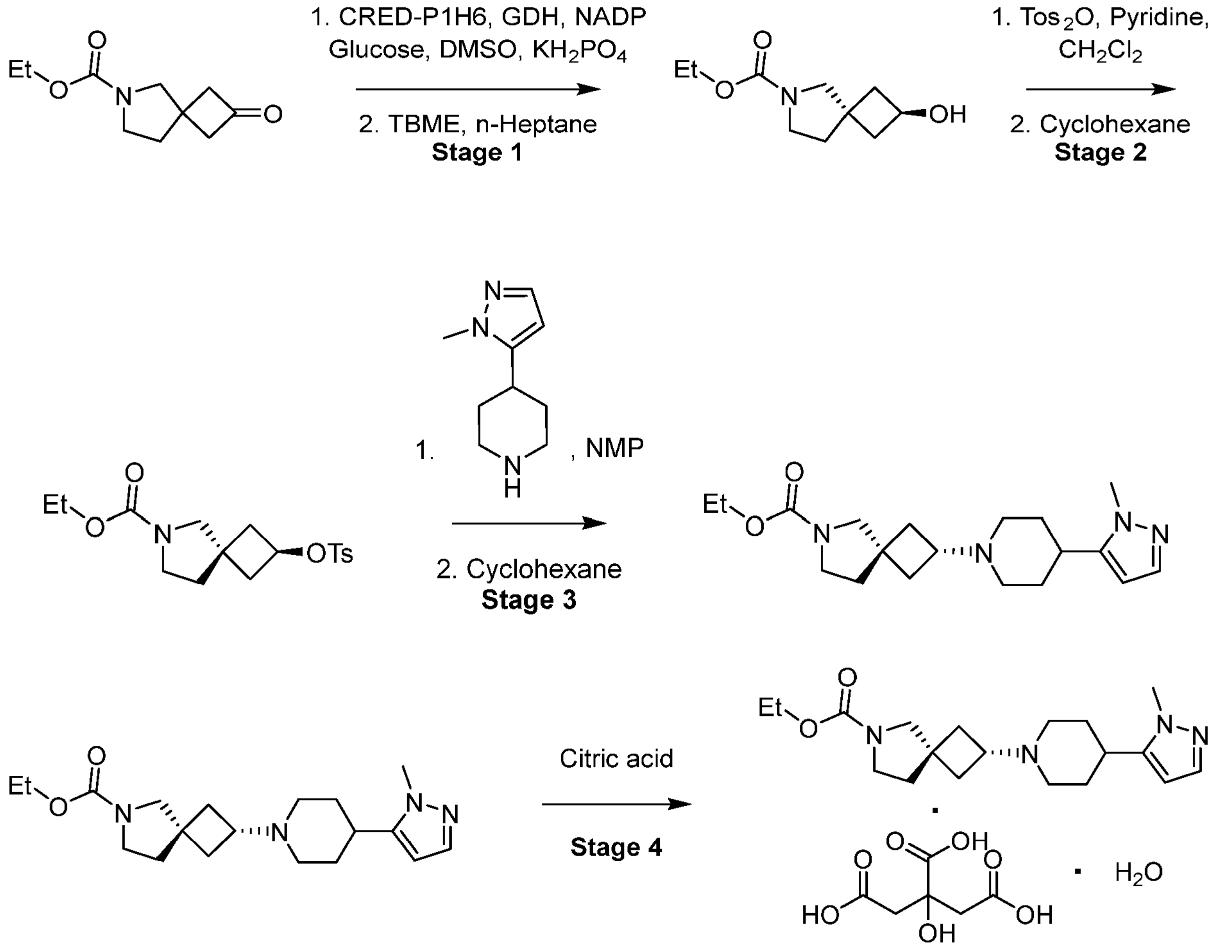


The compound described herein may be prepared according to the following scheme:

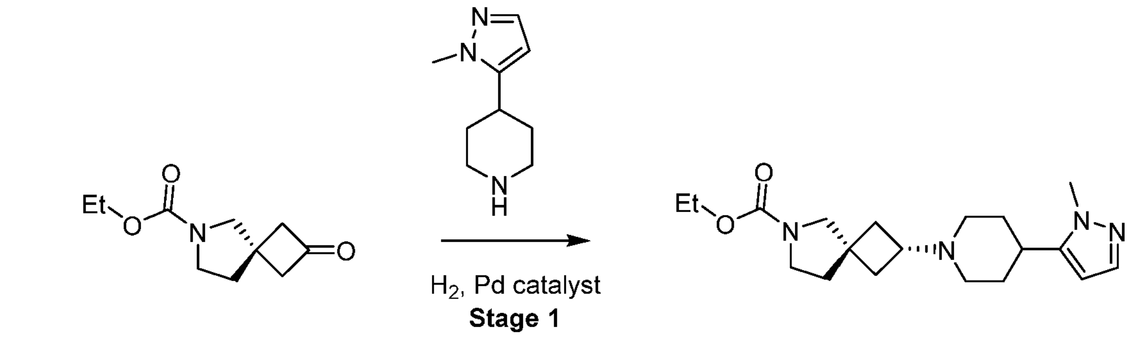


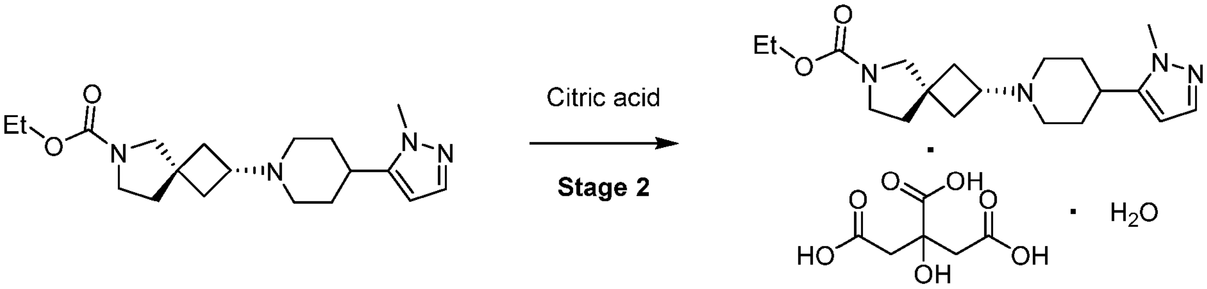


**Brief Description of the Figures**

**Fig. 1:** XRPD pattern of ethyl cis-2-[4-(1-methyl-1H-pyrazol-5-yl)piperidin-1-yl]-6-azaspiro[3.4]octane-6-carboxylate citrate exemplified, e.g., in Synthetic Example 6.

**Fig. 2:** TG/DTA thermogram for ethyl cis-2-[4-(1-methyl-1H-pyrazol-5-yl)piperidin-1-yl]-6-azaspiro[3.4]octane-6-carboxylate citrate exemplified, e.g., in Synthetic Example 6analysed from 25-300 °C at 10 °C per minute.

**Fig. 3:** Photomicrograph of ethyl cis-2-[4-(1-methyl-1H-pyrazol-5-yl)piperidin-1-yl]-6-azaspiro[3.4]octane-6-carboxylate citrate exemplified, e.g., in Synthetic Example 6.

**Fig. 4:** $^1$H NMR spectrum of ethyl cis-2-[4-(1-methyl-1H-pyrazol-5-yl)piperidin-1-yl]-6-azaspiro[3.4]octane-6-carboxylate citrate exemplified, e.g., in Synthetic Example 6.

**Fig. 5:** DVS isotherm of ethyl cis-2-[4-(1-methyl-1H-pyrazol-5-yl)piperidin-1-yl]-6-azaspiro[3.4]octane-6-carboxylate citrate exemplified, e.g., in Synthetic Example 6.

**Fig. 6** XRPD trace showing (top) ethyl cis-2-[4-(1-methyl-1H-pyrazol-5-yl)piperidin-1-yl]-6-azaspiro[3.4]octane-6-carboxylate, (middle) ethyl cis-2-[4-(1-methyl-1H-pyrazol-5-yl)piperidin-1-yl]-6-azaspiro[3.4]octane-6-carboxylate citrate exemplified, e.g., in Synthetic Example 6 and (bottom) ethyl cis-2-[4-(1-methyl-1H-pyrazol-5-yl)piperidin-1-yl]-6-azaspiro[3.4]octane-6-carboxylate citrate exemplified, e.g., in Synthetic Example 6 Post DVS.

**Fig. 7:** XRPD pattern of reference compound: ethyl cis-2-[4-(1-methyl-1H-pyrazol-5-yl)piperidin-1-yl]-6-azaspiro[3.4]octane-6-carboxylate hydrochloride exemplified, e.g., in Synthetic Example 7.

**Fig. 8:** TG/DTA thermogram for reference compound: ethyl cis-2-[4-(1-methyl-1H-pyrazol-5-yl)piperidin-1-yl]-6-azaspiro[3.4]octane-6-carboxylate hydrochloride exemplified, e.g., in Synthetic Example 7 analysed from 25-300 °C at 10 °C per minute.

**Fig. 9:** DSC thermogram for reference compound: ethyl cis-2-[4-(1-methyl-1H-pyrazol-5-yl)piperidin-1-yl]-6-azaspiro[3.4]octane-6-carboxylate hydrochloride exemplified, e.g., in Synthetic Example 7 analysed from 25-300 °C at 10 °C per minute.

**Fig. 10:** Photomicrograph of reference compound: ethyl cis-2-[4-(1-methyl-1H-pyrazol-5-yl)piperidin-1-yl]-6-azaspiro[3.4]octane-6-carboxylate hydrochloride exemplified, e.g., in Synthetic Example 7.

**Fig. 11:** $^1$H NMR spectrum of reference compound: ethyl cis-2-[4-(1-methyl-1H-pyrazol-5-yl)piperidin-1-yl]-6-azaspiro[3.4]octane-6-carboxylate hydrochloride exemplified, e.g., in Synthetic Example 7.

**Fig. 12:** DVS isotherm of reference compound: ethyl cis-2-[4-(1-methyl-1H-pyrazol-5-yl)piperidin-1-yl]-6-azaspiro[3.4]octane-6-carboxylate hydrochloride exemplified, e.g., in Synthetic Example 7.

**Fig. 13:** XRPD trace showing (top) reference compound: ethyl cis-2-[4-(1-methyl-1H-pyrazol-5-yl)piperidin-1-yl]-6-azaspiro[3.4]octane-6-carboxylate hydrochloride exemplified, e.g., in Synthetic Example 7 post-DVS, (middle) reference compound: ethyl cis-2-[4-(1-methyl-1H-pyrazol-5-yl)piperidin-1-yl]-6-azaspiro[3.4]octane-6-carboxylate hydrochloride exemplified, e.g., in Synthetic Example 7 pre-DVS and (bottom) known alternative reference HCl salt form.

**Fig. 14:** XRPD pattern of reference compound: ethyl cis-2-[4-(1-methyl-1H-pyrazol-5-yl)piperidin-1-yl]-6-azaspiro[3.4]octane-6-carboxylate fumarate exemplified, e.g., in Synthetic Example 8.

**Fig. 15:** TG/DTA thermogram for reference compound: ethyl cis-2-[4-(1-methyl-1H-pyrazol-5-yl)piperidin-1-yl]-6-azaspiro[3.4]octane-6-carboxylate fumarate exemplified, e.g., in Synthetic Example 8 analysed from 25-300 °C at 10 °C per minute.

**Fig. 16:** Photomicrograph of reference compound: ethyl cis-2-[4-(1-methyl-1H-pyrazol-5-yl)piperidin-1-yl]-6-azaspiro[3.4]octane-6-carboxylate fumarate exemplified, e.g., in Synthetic Example 8.

**Fig. 17:** $^1$H NMR spectrum of reference compound: ethyl cis-2-[4-(1-methyl-1H-pyrazol-5-yl)piperidin-1-yl]-6-azaspiro[3.4]octane-6-carboxylate fumarate exemplified, e.g., in Synthetic Example 8.

**Fig. 18:** DVS isotherm of reference compound: ethyl cis-2-[4-(1-methyl-1H-pyrazol-5-yl)piperidin-1-yl]-6-azaspiro[3.4]octane-6-carboxylate fumarate exemplified, e.g., in Synthetic Example 8.

**Fig. 19:** XRPD trace showing (top) reference compound: ethyl cis-2-[4-(1-methyl-1H-pyrazol-5-yl)piperidin-1-yl]-6-azaspiro[3.4]octane-6-carboxylate (Free base), (middle) ethyl cis-2-[4-(1-methyl-1H-pyrazol-5-yl)piperidin-1-yl]-6-azaspiro[3.4]octane-6-carboxylate fumarate exemplified, e.g., in Synthetic Example 8 and (bottom) reference compound: ethyl cis-2-[4-(1-methyl-1H-pyrazol-5-yl)piperidin-1-yl]-6-azaspiro[3.4]octane-6-carboxylate fumarate exemplified, e.g., in Synthetic Example 8 - Post DVS.

**Fig. 20:** XRPD trace showing (top) reference compound: ethyl cis-2-[4-(1-methyl-1H-pyrazol-5-yl)piperidin-1-yl]-6-azaspiro[3.4]octane-6-carboxylate fumarate exemplified, e.g., in Synthetic Example 8 prior to humidity stressing and (bottom) post-humidity stressed reference sample.

**Fig. 21:** XRPD trace showing (top) ethyl cis-2-[4-(1-methyl-1H-pyrazol-5-yl)piperidin-1-yl]-6-azaspiro[3.4]octane-6-carboxylate citrate exemplified, e.g., in Synthetic Example 6 prior to humidity stressing and (bottom) post-humidity stressed sample.

**Fig. 22:** XRPD trace showing (top) reference compound: ethyl cis-2-[4-(1-methyl-1H-pyrazol-5-yl)piperidin-1-yl]-6-azaspiro[3.4]octane-6-carboxylate hydrochloride exemplified, e.g., in Synthetic Example 7 prior to humidity stressing, (middle) post-humidity stressed reference sample and (bottom) known alternative reference HCl salt form.

**Fig. 23:** XRPD trace showing (top) reference compound: ethyl cis-2-[4-(1-methyl-1H-pyrazol-5-yl)piperidin-1-yl]-6-azaspiro[3.4]octane-6-carboxylate fumarate exemplified, e.g., in Synthetic Example 8, (middle) reference compound: ethyl cis-2-[4-(1-methyl-1H-pyrazol-5-yl)piperidin-1-yl]-6-azaspiro[3.4]octane-6-carboxylate fumarate exemplified, e.g., in Synthetic Example 8 aqueous solubility sample and (bottom) fumaric acid.

**Fig. 25:** XRPD pattern of ethyl cis-2-[4-(1-methyl-1H-pyrazol-5-yl)piperidin-1-yl]-6-azaspiro[3.4]octane-6-carboxylate citrate monohydrate exemplified in, e.g., Synthetic Examples 1-3.

**Fig. 26:** TGA/DSC thermogram for ethyl cis-2-[4-(1-methyl-1H-pyrazol-5-yl)piperidin-1-yl]-6-azaspiro[3.4]octane-6-carboxylate citrate monohydrate exemplified in, e.g., Synthetic Examples 1-3, analysed from 25-300 °C at 10 °C per minute.

**Fig. 27:** DVS isotherm plot for ethyl cis-2-[4-(1-methyl-1*H*-pyrazol-5-yl)piperidin-1-yl]-6-azaspiro[3.4]octane-6-carboxylate citrate monohydrate exemplified in, e.g., Synthetic Examples 1-3.

**Fig. 28:** Photomicrograph of ethyl cis-2-[4-(1-methyl-1H-pyrazol-5-yl)piperidin-1-yl]-6-azaspiro[3.4]octane-6-carboxylate citrate monohydrate exemplified in, e.g., Synthetic Examples 1-3.

**Fig. 29:** SEM micrographs of ethyl cis-2-[4-(1-methyl-1H-pyrazol-5-yl)piperidin-1-yl]-6-azaspiro[3.4]octane-6-carboxylate citrate monohydrate exemplified in, e.g., Synthetic Examples 1-3. Top: aggregate of particles; Bottom: single crystal. Scale bar shows 50 μm.

**Fig. 30:** 1H NMR spectrum of ethyl cis-2-[4-(1-methyl-1H-pyrazol-5-yl)piperidin-1-yl]-6-azaspiro[3.4]octane-6-carboxylate citrate monohydrate exemplified in, e.g., Synthetic Examples 1-3.

**Fig. 31:** Zoomed sections of $^1$H NMR spectrum of ethyl cis-2-[4-(1-methyl-1*H*-pyrazol-5-yl)piperidin-1-yl]-6-azaspiro[3.4]octane-6-carboxylate citrate monohydrate exemplified in, e.g., Synthetic Examples 1-3.

**Fig. 34:** $^1$H NMR Spectrum for tert-butyl trans-2-(tosyloxy)-6-azaspiro[3.4]octane-6-carboxylate.

**Fig. 35:** $^1$H NMR Spectrum for tert-butyl cis-2-(tosyloxy)-6-azaspiro[3.4]octane-6-carboxylate.

**Fig. 36:** Assignment of tert-butyl trans-2-(tosyloxy)-6-azaspiro[3.4]octane-6-carboxylate by $^1$H-$^1$H NOESY. Top: Assignment of NOE cross-peaks; Bottom: zoomed section of 2D-NOESY spectrum.

**Fig. 37:** Assignment of tert-butyl cis-2-(tosyloxy)-6-azaspiro[3.4]octane-6-carboxylate by $^1$H-$^1$H NOESY. Top: Assignment of NOE cross-peaks; Bottom: zoomed section of 2D-NOESY spectrum.

## Detailed Description

References to methods of treatment by therapy or surgery or in vivo diagnosis methods in this description are to

be interpreted as references to compounds, pharmaceutical compositions and/or medicaments for use in those methods.

Described herein are salts having selective activity as muscarinic M1 and/or M4 receptor agonists. More particularly, described herein are salts that exhibit selectivity for the M4 receptor relative to the M2 and M3 receptor subtypes.

The following compounds are muscarinic M1 and/or M4 receptor agonists: ethyl *cis*-2-[4-(1-methyl-1*H*-pyrazol-5-yl)piperidin-1-yl]-6-azaspiro[3.4]octane-6-carboxylate (structure shown below); ethyl cis-2-{4-[(2R)-4,4-difluoro-2-(hydroxymethyl)pyrrolidin-1-yl]piperidin-1-yl}-6-azaspiro[3.4]octane-6-carboxylate (structure shown below); and ethyl cis-2-{4-[(2R,4R)-4-fluoro-2-(hydroxymethyl)pyrrolidin-1-yl]piperidin-1-yl}-6-azaspiro[3.4]octane-6-carboxylate (structure shown below).

Each of these compounds, and salts thereof, is useful, for example in the treatment of muscarinic receptor mediated diseases of diseases and conditions, such as schizophrenia, Alzheimer's disease, Alzheimer's psychosis, and bipolar disorders (see, e.g., WO2015/118342 A1).

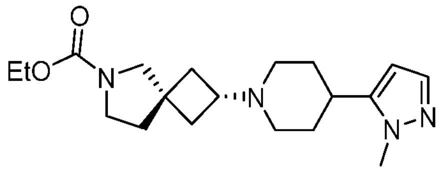

ethyl cis-2-[4-(1-methyl-1*H*-pyrazol-5-yl)piperidin-1-yl]-6-azaspiro[3.4]octane-6-carboxylate

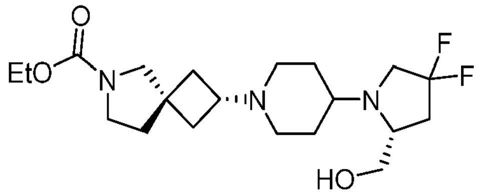

ethyl cis-2-{4-[(2R)-4,4-difluoro-2-(hydroxymethyl)pyrrolidin-1-yl]piperidin-1-yl}-6-azaspiro[3.4]octane-6-carboxylate

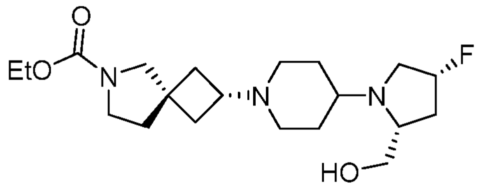

ethyl cis-2-{4-[(2R,4R)-4-fluoro-2-(hydroxymethyl)pyrrolidin-1-yl]piperidin-1-yl}-6-azaspiro[3.4]octane-6-carboxylate

***Processes for Preparing Compounds***

Described herein are processes for preparing compounds described herein, and salts thereof.

**Ethyl *cis*-2-[4-(1-methyl-1*H*-pyrazol-5-yl)piperidin-1-yl]-6-azaspiro[3.4]octane-6-carboxylate (compounds of formulae (1), (2b), (3b), (4b))**

Described herein is a process of preparing a compound of formula (1), or a salt thereof, or a crystalline form thereof, wherein the process comprises the step of reacting a compound of the following formula (I):

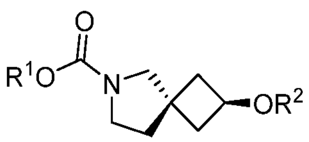

(I);

with a compound of the following formula (IIa), or a salt thereof:

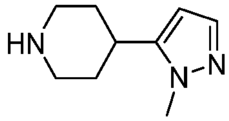

(IIa);

to provide a compound of the following formula (IIIa), or a salt thereof:

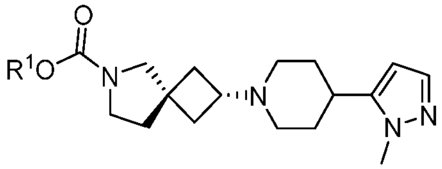

(IIIa);

wherein:

$R^1$ is a $C_{1-6}$ linear or branched alkyl group or a $C_{3-6}$ cycloalkyl group;
$R^2$ together with the oxygen atom to which it is attached represents any suitable leaving group.

Also described herein is a process of preparing a compound of formula (1), or a salt thereof, or a crystalline form thereof, wherein the process comprises the step of reacting a compound of the following formula (IV):

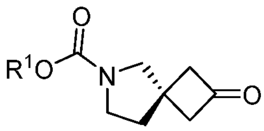

(IV);

with a compound of the following formula (IIa), or a salt thereof:

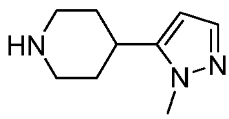

(IIa);

under a reductive amination condition to provide a compound of the following formula (IIIa), or a salt thereof:

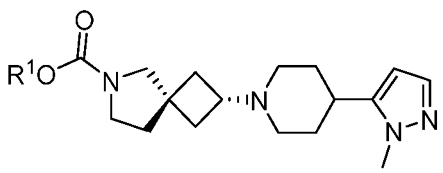

(IIIa);

wherein, $R^1$ is a $C_{1-6}$ linear or branched alkyl group or a $C_{3-6}$ cycloalkyl group.

Also described herein is a process of preparing a compound of formula (1), or a salt thereof, or a crystalline form thereof, wherein the process comprises the step of reacting a compound of the following formula (IIIa), or a salt thereof:

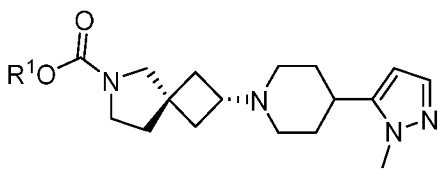

(IIIa);

with a deprotection acid, to provide a compound of formula (V), or a salt thereof:

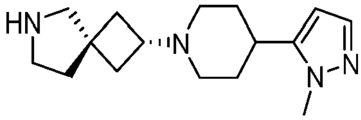

(V);

wherein, $R^1$ is a $C_{1-6}$ linear or branched alkyl group or a $C_{3-6}$ cycloalkyl group.

Also described herein is a process of preparing a compound of formula (1), or a salt thereof, or a crystalline form thereof, wherein the process comprises the step of reacting a compound of the following formula (V), or a salt thereof:

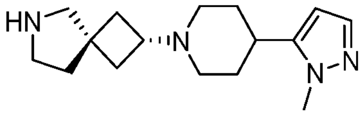

(V);

with ethyl chloroformate, to provide a compound of formula (1), or a salt thereof:

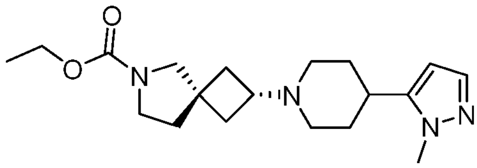

(1).

In some embodiments, the compound of formula (1) prepared by a process described hereinabove is a compound of formula (2b), or a crystalline form thereof:

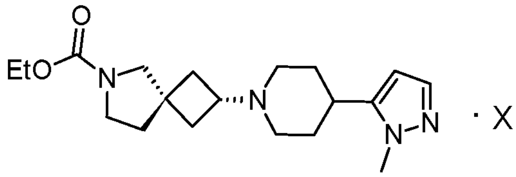

(2b);

wherein X denotes a salt.

In some embodiments, the compound of formula (1) prepared by a process described hereinabove is a compound of formula (3b), or a crystalline form thereof:

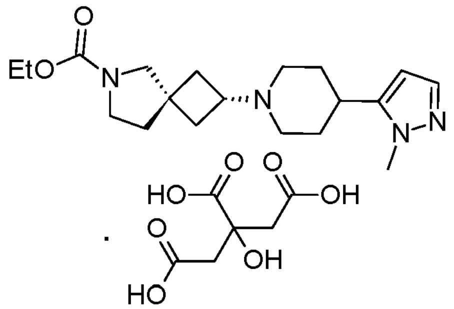

(3b).

In some embodiments, the compound of formula (1) prepared by a process described hereinabove is a compound of formula (4b), or a crystalline form thereof:

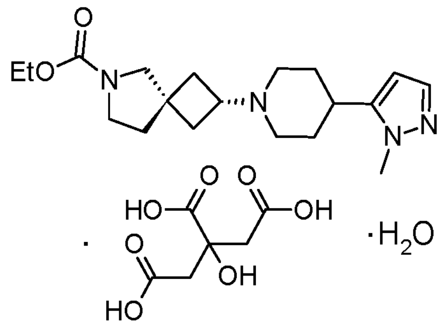

(4b).

Also described herein is a process for preparing a compound of formula (4b), or a crystalline form thereof, wherein the process comprises the step of forming a compound of formula (4b):

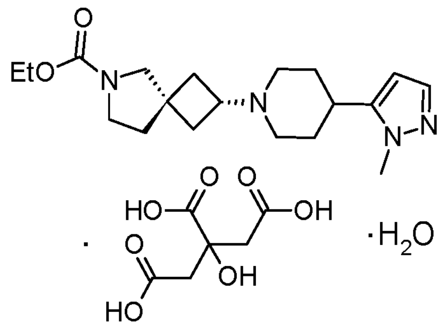

(4b);

from a crystallisation solution comprising ethanol, water, citric acid, and a compound of formula (1):

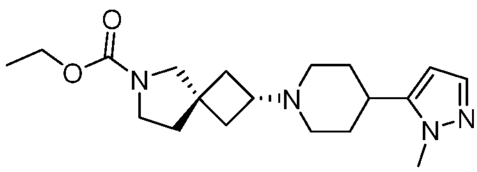

(1).

Also described herein is a process for preparing a compound of formula (4b), or a crystalline form thereof, wherein the process comprises:

(i.a) the step of reacting a compound of the following formula (I):

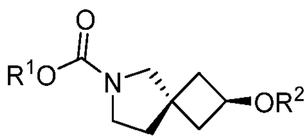

(I);

with a compound of the following formula (IIa), or a salt thereof:

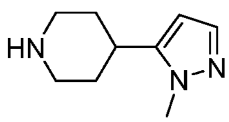

(IIa);

to provide a compound of the following formula (IIIa), or a salt thereof:

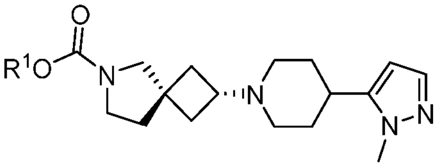

(IIIa);

wherein:

$R^1$ is a $C_{1-6}$ linear or branched alkyl group or a $C_{3-6}$ cycloalkyl group;
$R^2$ together with the oxygen atom to which it is attached represents any suitable leaving group; or

(i.b) the step of reacting a compound of the following formula (IV):

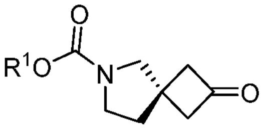

(IV);

with a compound of the following formula (IIa), or a salt thereof:

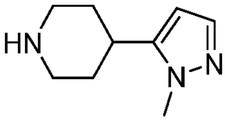

(IIa);

under a reductive amination condition to provide a compound of the following formula (IIIa), or a salt thereof:

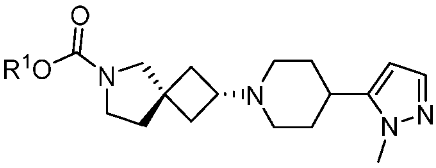

(IIIa);

wherein, $R^1$ is a $C_{1-6}$ linear or branched alkyl group or a $C_{3-6}$ cycloalkyl group; then
(ii) the step of reacting a compound of the following formula (IIIa), or a salt thereof:

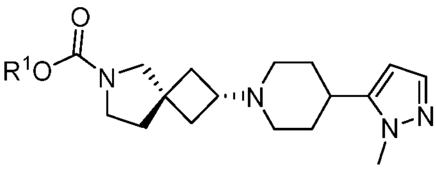

(IIIa);

with a deprotection acid, to provide a compound of formula (V), or a salt thereof:

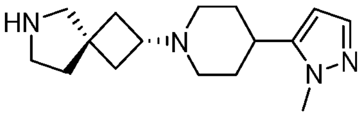

(V);

then
(iii) the step of reacting a compound of the following formula (V), or a salt thereof:

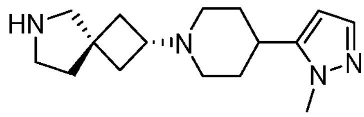

(V);

with ethyl chloroformate, to provide a compound of formula (1), or a salt thereof:

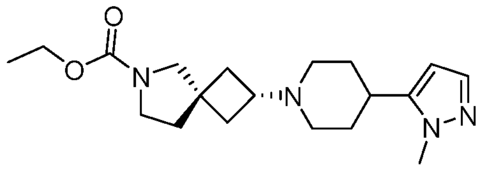

(1);

then

(iv) the step of forming a compound of formula (4b):

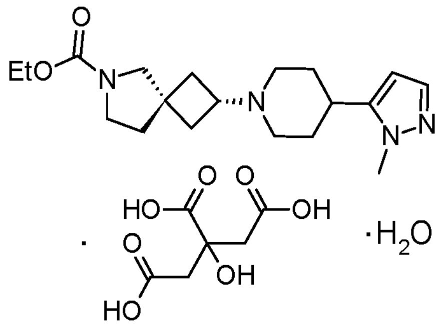

(4b);

from a solution comprising ethanol, water, citric acid, and a compound of formula (1):

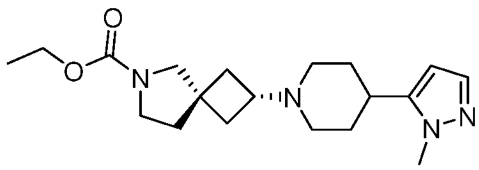

(1).

In some embodiments $R^1$ is a $C_{1-6}$ linear or branched alkyl group. In some embodiments, $R^1$ is ethyl or t-butyl. In some embodiments, $R^1$ is t-butyl. In some embodiments, $R^1$ is ethyl.

In some embodiments, $R^2$ together with the oxygen atom to which it is attached represents a sulfonate leaving group. In some embodiments, $R^2$ together with the oxygen atom to which it is attached represents an aryl sulfonate group. In some embodiments, $R^2$ together with the oxygen atom to which it is attached represents a tosylate group.

In some embodiments, the reductive amination condition comprises a reduction acid and a reductant.

In some embodiments, the reductive amination condition comprises $H_2$ and palladium. In some embodiments, the reductive amination condition comprises $H_2$, palladium on barium sulfate, and molecular sieves. In some embodiments, the the reductive amination condition comprises $H_2$ at about 1000 psi, palladium on barium sulfate (reduced), and molecular sieves.

In some embodiments, the reductive amination condition comprises a reduction acid having a pKa of from about 2.0 to about 3.5. In some embodiments, the reductive amination comprises a reduction acid selected from citric acid, tartaric acid, pivalic acid, 2,2-dimethylsuccinic acid, acetic acid, di-p-toluoyltartaric acid, difluoroacetic acid, phosphoric acid, an mixtures thereof. In some embodiments, the reductive amination comprises a reduction acid that is citric acid. In some embodiments, the reductive amination comprises a reduction acid that is tartaric acid. In some embodiments, the reductive amination comprises a reduction acid that is a mixture of citric acid and tartaric acid.

In some embodiments, the reductive amination condition comprises reacting at a temperature of less than about 40 °C. In some embodiments, the reductive amination condition comprises reacting for a first time period, at a first temperature, followed by reacting for a second time period, at a second temperature. In some embodiments, the second temperature is higher than the first temperature. In some embodiments, the reductive amination condition comprises

reacting for about 1 day, at a room temperature (about 23 °C), followed by reacting for about 1 day, at about 35 °C.

In some embodiments, the compound of formula (IIa) is a salt. In some embodiments, the compound of formula (IIa) is a tartaric acid salt.

In some embodiments, the deprotection acid is hydrochloric acid. In some embodiments, the deprotection acid is hydrochloric acid, generated in situ by reaction between acetyl chloride and a protic solvent. In some embodiments, the deprotection acid is hydrochloric acid, generated in situ by reaction between acetyl chloride and ethanol.

In some embodiments, the compound of formula (V) is a salt. In some embodiments, the compound of formula (V) is a tri-acid salt. In some embodiments, the compound of formula (V) is a hydrochloride salt. In some embodiments, the compound of formula (V) is a trihydrochloride salt.

In some embodiments, the crystallisation solution comprises ethanol and water in a ratio of from about 100:1 to about 100:10 by volume. the crystallisation solution comprises ethanol and water in a ratio of from about 100:2 to about 100:5 by volume. In some embodiments, the crystallisation solution comprises ethanol and water in a ratio of about 100:2.7 by volume. In some embodiments, the crystallisation solution comprises ethanol and water in a ratio of about 100:5 by volume.

In some embodiments, the crystallisation solution comprises citric acid and the compound of formula (1) in a ratio of from about 1:1 to about 1.3:1. In some embodiments, the crystallisation solution comprises citric acid and the compound of formula (1) in a ratio of from about 1:1 to about 1.2:1. In some embodiments, the crystallisation solution comprises citric acid and the compound of formula (1) in a ratio of from about 1:1 to about 1.1:1. In some embodiments, the crystallisation solution comprises citric acid and the compound of formula (1) in a ratio of about 1:1. In some embodiments, the crystallisation solution comprises citric acid and the compound of formula (1) in a ratio of about 1.08:1.

In some embodiments, seeds of a crystalline form of a compound of formula (4b) are added to the crystallisation solution.

## Intermediate Compounds

Also described herein are intermediate compounds which are useful in the production of the compounds and salts described herein.

Accordingly, the also described herein is a compund of formula (I):

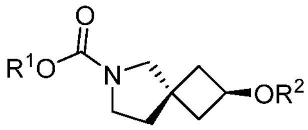
(I);

wherein:

- $R^1$ is a $C_{1-6}$ linear or branched alkyl group or a $C_{3-6}$ cycloalkyl group;
- $R^2$ together with the oxygen atom to which it is attached represents any suitable leaving group.

In some embodiments $R^1$ is a $C_{1-6}$ linear or branched alkyl group. In some embodiments, $R^1$ is ethyl or t-butyl. In some embodiments, $R^1$ is t-butyl. In some embodiments, $R^1$ is ethyl.

In some embodiments, $R^2$ together with the oxygen atom to which it is attached represents a sulfonate leaving group. In some embodiments, $R^2$ together with the oxygen atom to which it is attached represents an aryl sulfonate group. In some embodiments, $R^2$ together with the oxygen atom to which it is attached represents a tosylate group.

Also described herein is a compund of formula (III), or a salt thereof:

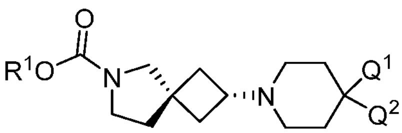
(III);

wherein:
$R^1$ is methyl or a $C_{3-6}$ linear or branched alkyl group, or a $C_{3-6}$ cycloalkyl group;

(a) $Q^1$ is -H and $Q^2$ is selected from:

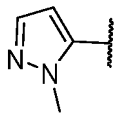, 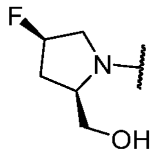, and 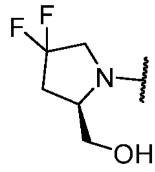;

or

(b) $Q^1$ is $-OR^3$ and $Q^2$ is $-OR^3$, and $R^3$ is a $C_{1-6}$ linear or branched alkyl group; or

(c) together $Q^1$ and $Q^2$ form a group $-O-(CH_2)_m-O-$ and m is an integer selected from 2, 3, and 4.

Also described herein is a compund of formula (IIIa), or a salt thereof:

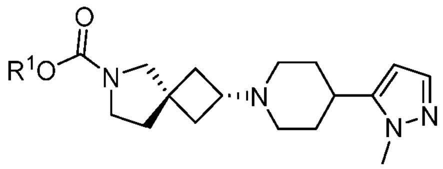
(IIIa);

wherein $R^1$ is methyl, or a $C_{3-6}$ linear or branched alkyl group or a $C_{3-6}$ cycloalkyl group.

In some embodiments $R^1$ is methyl, or a $C_{3-6}$ linear or branched alkyl group. In some embodiments, $R^1$ is t-butyl.

In some embodiments, the compound of formula (IIIa) is a salt. In some embodiments, the compound of formula (IIIa) is a citric acid salt. In some embodiments, the compound of formula (IIIa) is a hydrochloride salt.

Also described herein is a compund of formula (V), or a salt thereof:

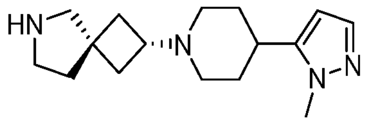
(V).

In some embodiments, the compound of formula (V) is a salt. In some embodiments, the compound of formula (V) is a tri-acid salt. In some embodiments, the compound of formula (V) is a hydrochloride salt. In some embodiments, the compound of formula (V) is a trihydrochloride salt.

## *Crystalline Forms*

Also described herein are crystalline forms of the salts described herein.

## Ethyl *cis*-2-[4-(1-methyl-1*H*-pyrazol-5-yl)piperidin-1-yl]-6-azaspiro[3.4]octane-6-carboxylate

In some embodiments, the present application provides at least one crystalline form of a citrate salt ethyl cis-2-[4-(1-methyl-1H-pyrazol-5-yl)piperidin-1-yl]-6-azaspiro[3.4]octane-6-carboxylate. In some embodiments, the present application provides at least one crystalline form of ethyl cis-2-[4-(1-methyl-1H-pyrazol-5-yl)piperidin-1-yl]-6-azaspiro[3.4]octane-6-carboxylate citrate. In some embodiments, the present application provides at least one crystalline form of ethyl cis-2-[4-(1-methyl-1H-pyrazol-5-yl)piperidin-1-yl]-6-azaspiro[3.4]octane-6-carboxylate citrate hydrate. In some embodiments, the present application provides at least one crystalline form of ethyl cis-2-[4-(1-methyl-1H-pyrazol-5-yl) piperidin-1-yl]-6-azaspiro[3.4]octane-6-carboxylate citrate monohydrate.

### *Ethyl cis-2-[4-(1-methyl-1H-pyrazol-5-yl)piperidin-1-yl]-6-azaspiro[3.4]octane-6-carboxylate citrate monohydrate*

In some embodiments, the crystalline form of a salt of ethyl cis-2-[4-(1-methyl-1H-pyrazol-5-yl)piperidin-1-yl]-6-azaspiro[3.4]octane-6-carboxylate is characterised by a DSC thermogram having an endothermic peak with onset at about 176 °C. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by a DSC thermogram substantially as shown in Figure 26.

In some embodiments, the crystalline form of a salt of ethyl cis-2-[4-(1-methyl-1H-pyrazol-5-yl)piperidin-1-yl]-6-azaspiro[3.4]octane-6-carboxylate is characterised by a TGA thermogram with a weight loss of about 3.3% loss from room temperature to about 110 °C. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by a TGA thermogram with a weight loss of about 27.4% from about 170 °C to 210 °C. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by a TGA thermogram substantially as shown in Figure 26.

In some embodiments, the crystalline form of a salt of ethyl cis-2-[4-(1-methyl-1H-pyrazol-5-yl)piperidin-1-yl]-6-azaspiro[3.4]octane-6-carboxylate is characterised by at least one XRPD peak, in terms of 2-theta, selected from 11.3°± 0.2°, 15.0°± 0.2°, 17.6°± 0.2°, 18.4°± 0.2°, 19.2°± 0.2°, 20.5°± 0.2°, 22.3°± 0.2°, 22.7°± 0.2°, and 24.1°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by at least two XRPD peaks, in terms of 2-theta, selected from 11.3°± 0.2°, 15.0°± 0.2°, 17.6°± 0.2°, 18.4°± 0.2°, 19.2°± 0.2°, 20.5°± 0.2°, 22.3°± 0.2°, 22.7°± 0.2°, and 24.1°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by at least three XRPD peaks, in terms of 2-theta, selected from 11.3°± 0.2°, 15.0°± 0.2°, 17.6°± 0.2°, 18.4°± 0.2°, 19.2°± 0.2°, 20.5°± 0.2°, 22.3°± 0.2°, 22.7°± 0.2°, and 24.1°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by at least four XRPD peaks, in terms of 2-theta, selected 11.3°± 0.2°, 15.0°± 0.2°, 17.6°± 0.2°, 18.4°± 0.2°, 19.2°± 0.2°, 20.5°± 0.2°, 22.3°± 0.2°, 22.7°± 0.2°, and 24.1°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by at least five XRPD peaks, in terms of 2-theta, selected from 11.3°± 0.2°, 15.0°± 0.2°, 17.6°± 0.2°, 18.4°± 0.2°, 19.2°± 0.2°, 20.5°± 0.2°, 22.3°± 0.2°, 22.7°± 0.2°, and 24.1°± 0.2°. In some embodiments, the crystalline form is characterised by at least six XRPD peaks, in terms of 2-theta, selected from 11.3°± 0.2°, 15.0°± 0.2°, 17.6°± 0.2°, 18.4°± 0.2°, 19.2°± 0.2°, 20.5°± 0.2°, 22.3°± 0.2°, 22.7°± 0.2°, and 24.1°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by at least seven XRPD peaks, in terms of 2-theta, selected from 11.3°± 0.2°, 15.0°± 0.2°, 17.6°± 0.2°, 18.4°± 0.2°, 19.2°± 0.2°, 20.5°± 0.2°, 22.3°± 0.2°, 22.7°± 0.2°, and 24.1°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by at least eight XRPD peaks, in terms of 2-theta, selected from 11.3°± 0.2°, 15.0°± 0.2°, 17.6°± 0.2°, 18.4°± 0.2°, 19.2°± 0.2°, 20.5°± 0.2°, 22.3°± 0.2°, 22.7°± 0.2°, and 24.1°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by XRPD peaks, in terms of 2-theta, at 11.3°± 0.2°, 15.0°± 0.2°, 17.6°± 0.2°, 18.4°± 0.2°, 19.2°± 0.2°, 20.5°± 0.2°, 22.3°± 0.2°, 22.7°± 0.2°, and 24.1°± 0.2°.

In some embodiments, the crystalline form of a salt of ethyl cis-2-[4-(1-methyl-1H-pyrazol-5-yl)piperidin-1-yl]-6-azaspiro[3.4]octane-6-carboxylate is characterised by at least one XRPD peak, in terms of 2-theta, selected from 11.3°± 0.2°, 18.4°± 0.2°, 19.2°± 0.2°, and 20.5°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by at least two XRPD peaks, in terms of 2-theta, selected from 11.3°± 0.2°, 18.4°± 0.2°, 19.2°± 0.2°, and 20.5°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by at least three XRPD peaks, in terms of 2-theta, selected from 11.3°± 0.2°, 18.4°± 0.2°, 19.2°± 0.2°, and 20.5°± 0.2°.

In some embodiments, the crystalline form of a salt of ethyl cis-2-[4-(1-methyl-1H-pyrazol-5-yl)piperidin-1-yl]-6-azaspiro[3.4]octane-6-carboxylate is characterised by three XRPD peaks, in terms of 2-theta, at 11.3°± 0.2°, 19.2°± 0.2°, and 20.5°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by three XRPD peaks, in terms of 2-theta, at 11.3°± 0.2°, 18.4°± 0.2°, and 20.5°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by three XRPD peaks, in terms of 2-theta, at 11.3°± 0.2°, 18.4°± 0.2°, and 19.2°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by three XRPD peaks, in terms of 2-theta, at 18.4°± 0.2°, 19.2°± 0.2°, and 20.5°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by four XRPD peaks, in terms of 2-theta, at 11.3°± 0.2°, 18.4°± 0.2°, 19.2°± 0.2°, and 20.5°± 0.2°.

In some embodiments, the crystalline form of a salt of ethyl cis-2-[4-(1-methyl-1H-pyrazol-5-yl)piperidin-1-yl]-6-azaspiro[3.4]octane-6-carboxylate is characterised by six XRPD peaks, in terms of 2-theta, at 11.3°± 0.2°, 15.0°± 0.2°, 17.6°± 0.2°, 18.4°± 0.2°, 19.2°± 0.2°, and 20.5°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by six XRPD peaks, in terms of 2-theta, at 11.3°± 0.2°, 15.0°± 0.2°, 18.4°± 0.2°, 19.2°± 0.2°, 20.5°± 0.2°, and 22.3°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by six XRPD peaks, in terms of 2-theta, at 11.3°± 0.2°, 15.0°± 0.2°, 18.4°± 0.2°, 19.2°± 0.2°, 20.5°± 0.2°, and 22.7°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by six XRPD peaks, in terms

of 2-theta, at 11.3°± 0.2°, 15.0°± 0.2°, 18.4°± 0.2°, 19.2°± 0.2°, 20.5°± 0.2°, and 24.1°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by six XRPD peaks, in terms of 2-theta, at 11.3°± 0.2°, 17.6°± 0.2°, 18.4°± 0.2°, 19.2°± 0.2°, 20.5°± 0.2°, and 22.3°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by six XRPD peaks, in terms of 2-theta, at 11.3°± 0.2°, 17.6°± 0.2°, 18.4°± 0.2°, 19.2°± 0.2°, 20.5°± 0.2°, and 22.7°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by six XRPD peaks, in terms of 2-theta, at 11.3°± 0.2°, 17.6°± 0.2°, 18.4°± 0.2°, 19.2°± 0.2°, 20.5°± 0.2°, and 24.1°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by six XRPD peaks, in terms of 2-theta, at 11.3°± 0.2°, 18.4°± 0.2°, 19.2°± 0.2°, 20.5°± 0.2°, 22.3°± 0.2°, and 22.7°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by six XRPD peaks, in terms of 2-theta, at 11.3°± 0.2°, 18.4°± 0.2°, 19.2°± 0.2°, 20.5°± 0.2°, 22.3°± 0.2°, and 24.1°± 0.2°. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by six XRPD peaks, in terms of 2-theta, at 11.3°± 0.2°, 18.4°± 0.2°, 19.2°± 0.2°, 20.5°± 0.2°, 22.7°± 0.2°, and 24.1°± 0.2°.

In some embodiments, the crystalline form of a salt of ethyl cis-2-[4-(1-methyl-1H-pyrazol-5-yl)piperidin-1-yl]-6-azaspiro[3.4]octane-6-carboxylate is characterised by an XRPD spectrum substantially as shown in Figure 25.

In any one of the above embodiments, the crystalline form may be prepared by any one of the processes described herein. In any one of the above embodiments, the crystalline form may be substantially isolated.

In any one of the above embodiments, the salt is a citrate salt of ethyl cis-2-[4-(1-methyl-1H-pyrazol-5-yl)piperidin-1-yl]-6-azaspiro[3.4]octane-6-carboxylate. In any one of the above embodiments, the salt is ethyl cis-2-[4-(1-methyl-1H-pyrazol-5-yl)piperidin-1-yl]-6-azaspiro[3.4]octane-6-carboxylate citrate hydrate. In any one of the above embodiments, the salt is ethyl cis-2-[4-(1-methyl-1H-pyrazol-5-yl)piperidin-1-yl]-6-azaspiro[3.4]octane-6-carboxylate citrate monohydrate.

*Ethyl cis-2-[4-(1-methyi-1H-pyrazol-5-yl)piperidin-1-yl]-6-azaspiro[3.4]octane-6-carboxylate citrate*

In some embodiments, the crystalline form of a salt of ethyl cis-2-[4-(1-methyl-1H-pyrazol-5-yl)piperidin-1-yl]-6-azaspiro[3.4]octane-6-carboxylate is characterised by a DTA thermogram having an endothermic peak with onset at about 174 °C. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by a DTA thermogram substantially as shown in Figure 2. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by a TGA thermogram with a weight loss of about 33% loss from about 140 °C to about 220 °C. In some embodiments, the crystalline form of a salt of ethyl cis-2-[4-(1-methyl-1H-pyrazol-5-yl)piperidin-1-yl]-6-azaspiro[3.4]octane-6-carboxylate is characterised by a TGA thermogram substantially as shown in Figure 2.

In some embodiments, the crystalline form of a salt of ethyl cis-2-[4-(1-methyl-1H-pyrazol-5-yl)piperidin-1-yl]-6-azaspiro[3.4]octane-6-carboxylate has an XRPD spectrum substantially as shown in Figure 1.

In any one of the above embodiments, the crystalline form may be prepared by any one of the processes described herein. In any one of the above embodiments, the crystalline form may be substantially isolated.

In any one of the above embodiments, the salt is a citrate salt of ethyl cis-2-[4-(1-methyl-1H-pyrazol-5-yl)piperidin-1-yl]-6-azaspiro[3.4]octane-6-carboxylate. In any one of the above embodiments, the salt is ethyl cis-2-[4-(1-methyl-1H-pyrazol-5-yl)piperidin-1-yl]-6-azaspiro[3.4]octane-6-carboxylate citrate.

Alternative Names

As used herein, the compound of the structural formula shown below:

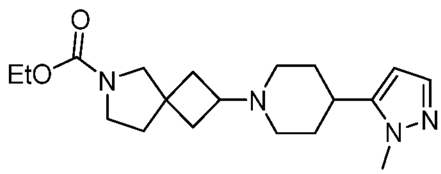

;

having a molecular formula of $C_{19}H_{30}N_4O_2$ and a molecular weight of 346.5 g/mol, may be referred to by the following names and identifiers:

ethyl 2-[4-(1-methyl-1H-pyrazol-5-yl)-1-piperidinyl]-6-azaspiro[3.4]octane-6-carboxylate;
ethyl 2-[4-(1-methyl-1H-pyrazol-5-yl)piperidin-1-yl]-6-azaspiro[3.4]octane-6-carboxylate; and
ethyl 2-(4-(1-methyl-1H-pyrazol-5-yl)piperidin-1-yl)-6-azaspiro[3.4]octane-6-carboxylate (as named by ChemDraw Professional v. 21.0.0.28, PerkinElmer Informatics, Inc.).

As used herein, the compound of formula (1), as shown below:

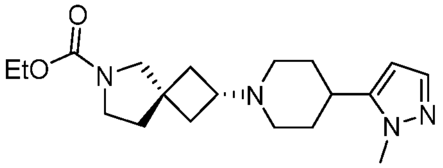

(1);

having a molecular formula of $C_{19}H_{30}N_4O_2$ and a molecular weight of 346.5 g/mol, may be referred to by the following names and identifiers:

ethyl cis-2-[4-(1-methyl-1H-pyrazol-5-yl)-1-piperidinyl]-6-azaspiro[3.4]octane-6-carboxylate;
ethyl cis-2-[4-(1-methyl-1H-pyrazol-5-yl)piperidin-1-yl]-6-azaspiro[3.4]octane-6-carboxylate;
ethyl (2r,4s)-2-[4-(1-methyl-1H-pyrazol-5-yl)piperidin-1-yl]-6-azaspiro[3.4]octane-6-carboxylate;
ethyl (2r,4s)-2-(4-(1-methyl-1H-pyrazol-5-yl)piperidin-1-yl)-6-azaspiro[3.4]octane-6-carboxylate (as named by ChemDraw Professional v. 21.0.0.28, PerkinElmer Informatics, Inc.); and
CAS RN: 1803346-98-6.

As used herein, the compound of formula (3b) shown below:

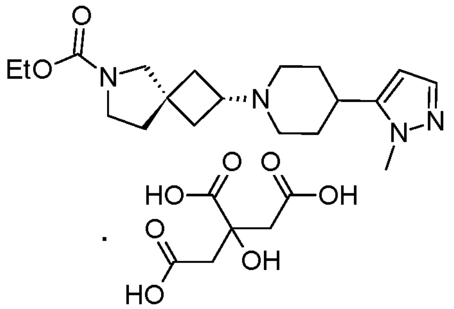

(3b).

wherein the molar ratio between ethyl cis-2-[4-(1-methyl-1H-pyrazol-5-yl)piperidin-1-yl]-6-azaspiro[3.4]octane-6-carboxylate and citric acid is 1:1; and having a molecular formula of $C_{25}H_{38}N_4O_9$ ($C_{19}H_{30}N_4O_2 \cdot C_6H_8O_7$) and a molecular weight of 538.6 g/mol; may be referred to by the following names and identifiers:

ethyl cis-2-[4-(1-methyl-1H-pyrazol-5-yl)-1-piperidinyl]-6-azaspiro[3.4]octane-6-carboxylate citrate;
ethyl cis-2-[4-(1-methyl-1H-pyrazol-5-yl)piperidin-1-yl]-6-azaspiro[3.4]octane-6-carboxylate citrate;
ethyl (2r,4s)-2-[4-(1-methyl-1H-pyrazol-5-yl)piperidin-1-yl]-6-azaspiro[3.4]octane-6-carboxylate citrate;
ethyl cis-2-[4-(1-methyl-1H-pyrazol-5-yl)-1-piperidinyl]-6-azaspiro[3.4]octane-6-carboxylate, citrate (1:1); and
ethyl (2r,4s)-2-[4-(1-methyl-1H-pyrazol-5-yl)piperidin-1-yl]-6-azaspiro[3.4]octane-6-carboxylate, citrate (1:1).

As used herein, the compound of formula (4b) shown below:

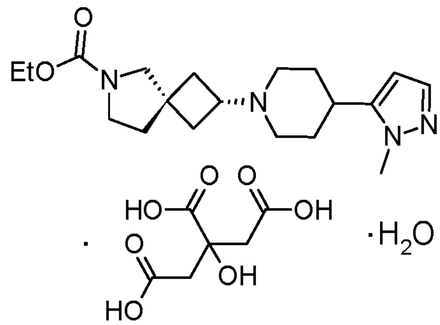

(4b).

wherein the molar ratio between ethyl cis-2-[4-(1-methyl-1H-pyrazol-5-yl)piperidin-1-yl]-6-azaspiro[3.4]octane-6-carboxylate, citric acid, and water is 1:1:1; and having a molecular formula of $C_{25}H_{40}N_4O_{10}$ ($C_{19}H_{30}N_4O_2 \cdot C_6H_8O_7 \cdot H_2O$) and a molecular weight of 556.6 g/mol; may be referred to by the following names and identifiers:

ethyl cis-2-[4-(1-methyl-1H-pyrazol-5-yl)-1-piperidinyl]-6-azaspiro[3.4]octane-6-carboxylate citrate monohydrate;
ethyl cis-2-[4-(1-methyl-1H-pyrazol-5-yl)piperidin-1-yl]-6-azaspiro[3.4]octane-6-carboxylate citrate monohydrate;
ethyl (2r,4s)-2-[4-(1-methyl-1H-pyrazol-5-yl)piperidin-1-yl]-6-azaspiro[3.4]octane-6-carboxylate citrate monohydrate;
ethyl cis-2-[4-(1-methyl-1H-pyrazol-5-yl)-1-piperidinyl]-6-azaspiro[3.4]octane-6-carboxylate, citrate (1:1), hydrate;
ethyl (2r,4s)-2-[4-(1-methyl-1H-pyrazol-5-yl)piperidin-1-yl]-6-azaspiro[3.4]octane-6-carboxylate, citrate (1:1), hydrate; and
CAS RN: 2920742-36-3.

As used herein, the compound of formula (5b) shown below:

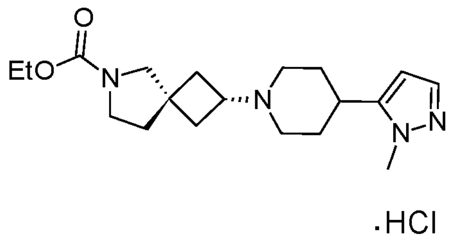

(5b).

wherein the molar ratio between ethyl cis-2-[4-(1-methyl-1H-pyrazol-5-yl)piperidin-1-yl]-6-azaspiro[3.4]octane-6-carboxylate and hydrochloric acid is 1:1; and having a molecular formula of $C_{19}H_{31}ClN_4O_2$ ($C_{19}H_{30}N_4O_2 \cdot HCl$) and a molecular weight of 382.9 g/mol; may be referred to by the following names and identifiers:

ethyl cis-2-[4-(1-methyl-1H-pyrazol-5-yl)-1-piperidinyl]-6-azaspiro[3.4]octane-6-carboxylate hydrochloride;
ethyl cis-2-[4-(1-methyl-1H-pyrazol-5-yl)piperidin-1-yl]-6-azaspiro[3.4]octane-6-carboxylate hydrochloride;
ethyl (2r,4s)-2-[4-(1-methyl-1H-pyrazol-5-yl)piperidin-1-yl]-6-azaspiro[3.4]octane-6-carboxylate hydrochloride;
ethyl cis-2-[4-(1-methyl-1H-pyrazol-5-yl)-1-piperidinyl]-6-azaspiro[3.4]octane-6-carboxylate, hydrochloride (1:1); and
ethyl (2r,4s)-2-[4-(1-methyl-1H-pyrazol-5-yl)piperidin-1-yl]-6-azaspiro[3.4]octane-6-carboxylate, hydrochloride (1:1).

As used herein, the compound of formula (6b) shown below:

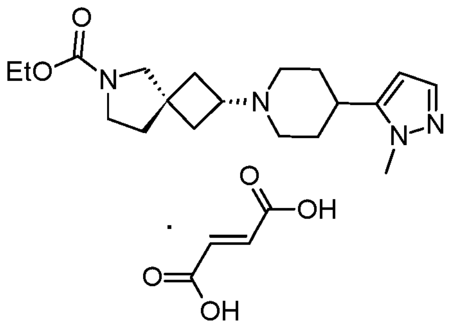

(6b).

wherein the molar ratio between ethyl cis-2-[4-(1-methyl-1H-pyrazol-5-yl)piperidin-1-yl]-6-azaspiro[3.4]octane-6-carboxylate and fumaric acid is 1:1; and having a molecular formula of $C_{23}H_{34}N_4O_6$ ($C_{19}H_{30}N_4O_2 \cdot C_4H_4O_4$) and a molecular weight of 462.6 g/mol; may be referred to by the following names and identifiers:

ethyl cis-2-[4-(1-methyl-1H-pyrazol-5-yl)-1-piperidinyl]-6-azaspiro[3.4]octane-6-carboxylate fumarate;
ethyl cis-2-[4-(1-methyl-1H-pyrazol-5-yl)piperidin-1-yl]-6-azaspiro[3.4]octane-6-carboxylate fumarate;
ethyl (2r,4s)-2-[4-(1-methyl-1H-pyrazol-5-yl)piperidin-1-yl]-6-azaspiro[3.4]octane-6-carboxylate fumarate;
ethyl cis-2-[4-(1-methyl-1H-pyrazol-5-yl)-1-piperidinyl]-6-azaspiro[3.4]octane-6-carboxylate, fumarate (1:1); and
ethyl (2r,4s)-2-[4-(1-methyl-1H-pyrazol-5-yl)piperidin-1-yl]-6-azaspiro[3.4]octane-6-carboxylate, fumarate (1:1).

Definitions

In this application, the following definitions apply, unless indicated otherwise.

The term "treatment", in relation to the uses of the compounds of the formula (1), formula (3b), or formula (4b) is used to describe any form of intervention where a compound is administered to a subject suffering from, or at risk of suffering from, or potentially at risk of suffering from the disease or disorder in question. Thus, the term "treatment" covers both preventative (prophylactic) treatment and treatment where measurable or detectable symptoms of the disease or disorder are being displayed.

The term "effective therapeutic amount" as used herein (for example in relation to methods of treatment of a disease or condition) refers to an amount of the compound which is effective to produce a desired therapeutic effect. For example, if the condition is pain, then the effective therapeutic amount is an amount sufficient to provide a desired level of pain relief. The desired level of pain relief may be, for example, complete removal of the pain or a reduction in the severity of the pain.

The term "suitable leaving group" describes a group suitable to act as such in a given reaction. For example, the leaving groups described herein are suitable for use in bimolecular nucleophilic substitution ($S_{N2}$) reactions. The term leaving group is defined in: IUPAC. Compendium of Chemical Terminology, 2nd ed. (the "Gold Book"). Compiled by A. D. McNaught and A. Wilkinson. Blackwell Scientific Publications, Oxford (1997). Online version (2019-) created by S. J. Chalk. ISBN 0-9678550-9-8. https://doi.org/10.1351/goldbook. Suitable leaving groups may include: sulfonate leaving groups (e.g., arylsulfonates, alkylsulfonates, haloalkylsulfonates), halogens (in particular -I, - Br, -Cl), trialkylamines, dinitrogen, dialkylethers, aryliodonium salts, and arylsulfonium salts. In some embodiments, the leaving group is a nucleofuge. In some embodiments, the leaving group is an arylsulfonate. In some embodiments, the leaving group is a tosylate group.

Salts

Compounds described herein can exist in the form of salts, for example acid addition salts or, in certain cases salts of organic and inorganic bases such as carboxylate, sulfonate and phosphate salts. All such salts are contemplated, and references to compounds include the salt forms of the compounds as defined herein.

The salts are typically acid addition salts.

The salts described herein can be synthesized from the parent compound that contains a basic or acidic moiety by conventional chemical methods such as methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002. Generally, such salts can be prepared by reacting the free acid or base forms of these compounds with the appropriate base or acid in water or in an organic solvent, or in a mixture of the two; generally, nonaqueous media such as ether, ethyl acetate, ethanol, isopropanol, or acetonitrile are used.

Acid addition salts may be formed with a wide variety of acids, both inorganic and organic. Examples of acid

addition salts contemplated herein include mono- or di-salts formed with an acid selected from the group consisting of acetic, 2,2-dichloroacetic, adipic, alginic, ascorbic (e.g. L-ascorbic), L-aspartic, benzenesulfonic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, D-gluconic, glucuronic (e.g. D-glucuronic), glutamic (e.g. L-glutamic), $\alpha$-oxoglutaric, glycolic, hippuric, hydrohalic acids (e.g. hydrobromic, hydrochloric, hydriodic), isethionic, lactic (e.g. (+)-L-lactic, ($\pm$)-DL-lactic), lactobionic, maleic, malic, (-)-L-malic, malonic, ($\pm$)-DL-mandelic, methanesulfonic, naphthalene-2-sulfonic, naphthalene-1,5-disulfonic, 1-hydroxy-2-naphthoic, nicotinic, nitric, oleic, orotic, oxalic, palmitic, pamoic, phosphoric, propionic, pyruvic, L-pyroglutamic, salicylic, 4-amino-salicylic, sebacic, stearic, succinic, sulfuric, tannic, (+)-L-tartaric, thiocyanic, p-toluenesulfonic, undecylenic and valeric acids, as well as acylated amino acids and cation exchange resins.

In particular, acid addition salts may be formed with an acid selected from: 2,5-dihydroxybenzoic (gentisic), 2-furoic, acetic, butandioic (succinic), citric, ethansulfonic (ESA), fumaric, gluconic (D), glucuronic (D), hydroxyacetic (glycolic), hydrochloric (HCl), maleic, malic (L), malonic, N-acetylglycine (aceturic), nicotinic, orthophosphoric (phosphoric), oxoglutaric (ketoglutaric), p-toluenesulfonic (p-TSA), pyroglutamic (L), sulphuric and tartaric (L).

Amine functions in the compounds described herein may form quaternary ammonium salts, for example by reaction with an alkylating agent according to methods well known to the skilled person. Such quaternary ammonium compounds are contemplated herein.

The compounds described herein may exist as mono- or di-salts depending upon the pKa of the acid from which the salt is formed.

The salt forms of the compounds described herein are typically pharmaceutically acceptable salts, and examples of pharmaceutically acceptable salts are discussed in Berge et al., 1977, "Pharmaceutically Acceptable Salts," J. Pharm. Sci., Vol. 66, pp. 1-19. However, salts that are not pharmaceutically acceptable may also be prepared as intermediate forms which may then be converted into pharmaceutically acceptable salts. Such non-pharmaceutically acceptable salts forms, which may be useful, for example, in the purification or separation of the compounds described herein, also contemplated herein.

Optical Isomers

Where compounds described herein contain one or more chiral centres, and can exist in the form of two or more optical isomers, references to the compounds include all optical isomeric forms thereof (e.g. enantiomers, epimers, diastereoisomers, *cis-trans* geomteric isomers of cyclic functional groups), either as individual optical isomers, or mixtures (e.g. racemic mixtures) or two or more optical isomers, unless the context requires otherwise.

The optical isomers may be characterised and identified by their optical activity (i.e. as + and - isomers, or d and *l* isomers) or they may be characterised in terms of their absolute stereochemistry using the "R and S" nomenclature developed by Cahn, Ingold and Prelog, see Advanced Organic Chemistry by Jerry March, 4th Edition, John Wiley & Sons, New York, 1992, pages 109-114, and see also Cahn, Ingold & Prelog, Angew. Chem. Int. Ed. Engl., 1966, 5, 385-415. Compounds featuring cyclic moieties may be characterised using *"cis-trans"* nomenclature. In the case of Optical isomers can be separated by a number of techniques including chiral chromatography (chromatography on a chiral support) and such techniques are well known to the person skilled in the art. As an alternative to chiral chromatography, optical isomers can be separated by forming diastereoisomeric salts with chiral acids such as (+)-tartaric acid, (-)-pyroglutamic acid, (-)-di-toluoyl-L-tartaric acid, (+)-mandelic acid, (-)-malic acid, and (-)-camphorsulphonic, separating the diastereoisomers by preferential crystallisation, and then dissociating the salts to give the individual enantiomer of the free base.

Where compounds described herein exist as two or more optical isomeric forms, one enantiomer in a pair of enantiomers may exhibit advantages over the other enantiomer, for example, in terms of biological activity. Thus, in certain circumstances, it may be desirable to use as a therapeutic agent only one of a pair of enantiomers, or only one of a plurality of diastereoisomers.

Accordingly, described herein are compositions containing a compound having one or more chiral centres, wherein at least 55% (e.g., at least 60%, 65%, 70%, 75%, 80%, 85%, 90% or 95%) of the compound is present as a single optical isomer (e.g., enantiomer or diastereoisomer).

In one general embodiment, 99% or more (e.g., substantially all) of the total amount of the compound (or compound for use) is present as a single optical isomer.

For example, in one embodiment the compound is present as a single enantiomer. In another embodiment the compound is present as a single diastereoisomer.

Alse described herein are mixtures of optical isomers, which may be racemic or non-racemic. Thus, described herein is:

A compound which is in the form of a racemic mixture of optical isomers.

A compound which is in the form of a non-racemic mixture of optical isomers.

*Cis-trans* isomerism

Additionally, described herein are compositions containing a compound having one or more areas of restricted rotation giving rise to *cis-trans* isomerism, wherein at least 55% (e.g., at least 60%, 65%, 70%, 75%, 80%, 85%, 90% or 95%) of the compound is present as a single stereoisomer of the *cis-trans* pair.
In one general embodiment, 99% or more (e.g., substantially all) of the total amount of the compound (or compound for use) is present as a single *cis-trans* isomer.
For example, in one embodiment the compound is present as a cis-isomer. In another embodiment the compound is present as a trans-isomer (for example, an intermediate compound).
Also described herein are mixtures of *cis-trans* isomers.

Isotopes

The compounds described herein may contain one or more isotopic substitutions, and a reference to a particular element includes within its scope all isotopes of the element. For example, a reference to hydrogen includes within its scope $^{1}H$, $^{2}H$ (D), and $^{3}H$ (T). Similarly, references to carbon and oxygen include within their scope respectively $^{12}C$, $^{13}C$ and $^{14}C$ and $^{16}O$ and $^{18}O$.
In an analogous manner, a reference to a particular functional group also includes within its scope isotopic variations, unless the context indicates otherwise. For example, a reference to an alkyl group such as an ethyl group also covers variations in which one or more of the hydrogen atoms in the group is in the form of a deuterium or tritium isotope, e.g. as in an ethyl group in which all five hydrogen atoms are in the deuterium isotopic form (a perdeuteroethyl group).
The isotopes may be radioactive or non-radioactive. The compounds may contain no radioactive isotopes. Such compounds are preferred for therapeutic use. However, the compound may contain one or more radioisotopes. Compounds containing such radioisotopes may be useful in a diagnostic context.

Solvates

Compounds described herein may form solvates. Preferred solvates are solvates formed by the incorporation into the solid-state structure (e.g. crystal structure) of the compounds described herein of molecules of a non-toxic pharmaceutically acceptable solvent (referred to below as the solvating solvent). Examples of such solvents include water, alcohols (such as ethanol, isopropanol and butanol) and dimethylsulfoxide. Solvates can be prepared by recrystalising the compounds described herein with a solvent or mixture of solvents containing the solvating solvent. Whether or not a solvate has been formed in any given instance can be determined by subjecting crystals of the compound to analysis using well known and standard techniques such as thermogravimetric analysis (TGE), differential scanning calorimetry (DSC) and X-ray crystallography. The solvates can be stoichiometric or non-stoichiometric solvates. Particularly preferred solvates are hydrates, and examples of hydrates include hemihydrates, monohydrates and dihydrates.
Accordingly, also described herein is:
A compound in the form of a solvate.
A compound wherein the solvate is a hydrate.
For a more detailed discussion of solvates and the methods used to make and characterise them, see Bryn et al., Solid-State Chemistry of Drugs, Second Edition, published by SSCI, Inc of West Lafayette, IN, USA, 1999, ISBN 0-967-06710-3.
Alternatively, rather than existing as a hydrate, the compounds described herein may be anhydrous. Therefore, also described herein is a compound described herein in an anhydrous form (e.g., anhydrous crystalline form).

Crystalline and amorphous forms

The compounds may exist in a crystalline or non-crystalline (e.g., amorphous) state. Whether or not a compound exists in a crystalline state can readily be determined by standard techniques such as X-ray powder diffraction (XRPD). Crystals and their crystal structures can be characterised using a number of techniques including single crystal X-ray crystallography, X-ray powder diffraction (XRPD), differential scanning calorimetry (DSC) and infra-red spectroscopy, e.g., Fourier Transform infra-red spectroscopy (FTIR). The behaviour of the crystals under conditions of varying humidity can be analysed by gravimetric vapour sorption studies and also by XRPD. Determination of the crystal structure of a compound can be performed by X-ray crystallography which can be carried out according to conventional methods such as those described herein and as described in Fundamentals of Crystallography, C. Giacovazzo, H. L. Monaco, D. Viterbo, F. Scordari, G. Gilli, G. Zanotti and M. Catti, (International Union of Crystallography/Oxford University Press, 1992 ISBN 0-19-855578-4 (p/b), 0-19-85579-2 (h/b)). This technique involves the analysis and interpretation of the X-ray diffraction of single crystal. In an amorphous solid, the three dimensional structure that normally exists in a crystalline form does not

exist and the positions of the molecules relative to one another in the amorphous form are essentially random, see for example Hancock et al. J. Pharm. Sci. (1997), 86, 1).

Other characterisation methods such as differential scanning calorimetry (DSC), differential thermal analysis (DTA), thermogravimetric analysis (TGA), dynamic vapor sorption (DVS), and the like, may help to further identify the form, as well as help determine stability and solvent/water content.

An XRPD pattern of reflections (peaks) is typically considered a fingerprint of a particular crystalline form. It is well known that the relative intensities of the XRPD peaks can widely vary depending on, inter alia, the sample preparation technique, crystal size distribution, various filters used, the sample mounting procedure, and the particular instrument employed. In some instances, new peaks may be observed or existing peaks may disappear, depending on the type of the instrument or the settings. As used herein, the term "peak" refers to a reflection having a relative height/intensity of at least about 4% of the maximum peak height/intensity. Moreover, instrument variation and other factors can affect the 2-theta values. Thus, peak assignments, such as those reported herein, can vary by plus or minus about 0.2° (2-theta), and the term "substantially" and "about" as used in the context of XRPD herein is meant to encompass the above-mentioned variations.

In the same way, temperature readings in connection with DSC, TGA, or other thermal experiments can vary about ±3 °C depending on the instrument, particular settings, sample preparation, etc. Accordingly, a crystalline form provided herein a DSC thermogram "substantially" as shown in any of the Figures or the term "about" is understood to accommodate such variation. As used herein, the term "no substantial weight loss" means weight loss which is less than 0.25%.

Different forms of the same substance may have different bulk properties relating to, for example, hygroscopicity, solubility, stability, and the like. Forms with high melting points may have good thermodynamic stability which is advantageous in prolonging shelf-life drug formulations containing the solid form. Forms with lower melting points may be less thermodynamically stable, but may be advantageous in that they have increased water solubility, which may translate to increased drug bioavailability. Forms that are weakly hygroscopic may be desirable for their stability to heat and humidity and may be resistant to degradation during long storage.

Accordingly, also described herein is:
A compound in a crystalline form.

A compound which is:

(a) from 50% to 100% crystalline, and more particularly is at least 50% crystalline, or at least 60% crystalline, or at least 70% crystalline, or at least 80% crystalline, or at least 90% crystalline, or at least 95% crystalline, or at least 98% crystalline, or at least 99% crystalline, or at least 99.5% crystalline, or at least 99.9% crystalline, for example 100% crystalline.

A compound which is in an amorphous form.

In some embodiments, a crystalline form provided herein is substantially isolated. By "substantially isolated" is meant that the compound or crystalline form is at least partially or substantially separated from the environment in which it was formed or detected. Partial separation can include, for example, a composition enriched in the salts described herein. Substantial separation can include compositions containing at least about 50%, at least about 60%, at least about 70%, at least about 80%, at least about 90%, at least about 95%, at least about 97%, or at least about 99% by weight of the crystalline forms described herein, or salt thereof.

In some embodiments, a crystalline form of a compound or salt described herein comprises at least about 50%, at least about 60%, at least about 70%, at least about 80%, at least about 90%, at least about 95%, at least about 97%, or at least about 99% by weight of the crystalline form of that compound or salt described herein.

## Biological activity and therapeutic uses

Again, references to methods of treatment by therapy or surgery or in vivo diagnosis methods in this description are to be interpreted as references to compounds, pharmaceutical compositions and/or medicaments for use in those methods.

The compounds described herein have activity as muscarinic $M_4$ receptor agonists (WO2015/118342).

A significant advantage of certain compounds described herein is that they are highly selective for the $M_4$ receptor relative to the $M_1$, $M_2$ and $M_3$ receptor subtypes. For example, whereas compounds described herein typically have $pEC_{50}$ values of at least 6 and $E_{max}$ values of greater than 70 against the $M_4$ receptor in the functional assay described in Example A of WO2015/118342, they may have $pEC_{50}$ values of less than 5 and $E_{max}$ values of less than 20% when tested against the $M_1$, $M_2$ and $M_3$ subtypes in the functional assay of Example A of WO2015/118342.

With respect to the compounds described herein, also described herein is:
A compound for use in medicine.

A compound for use as a muscarinic $M_4$ receptor agonist.

A compound which is a muscarinic $M_4$ receptor agonist having a $pEC_{50}$ greater than 6 and an $E_{max}$ of at least 70 against the $M_4$ receptor in the assay of Example A herein or an assay substantially similar thereto.

A compound which is a muscarinic $M_4$ receptor agonist having a $pEC_{50}$ greater than 7.0.

A compound having an $E_{max}$ of at least 70 against the $M_4$ receptor.

A compound which is selective for the $M_4$ receptor compared to the muscarinic $M_1$, $M_2$ and $M_3$ receptors.

A compound which has a $pEC_{50}$ of less than 5 and an $E_{max}$ of less than 30 against the muscarinic $M_1$, $M_2$ and $M_3$ receptor subtypes.

A compound for use in the treatment of a disease or condition mediated by the muscarinic $M_4$ receptor.

A compound which is a muscarinic $M_1$ receptor agonist having a $pEC_{50}$ greater than 7.0.

A compound having an $E_{max}$ of at least 70 against the $M_1$ receptor.

A compound which is selective for the $M_1$ receptor compared to the muscarinic $M_2$ and $M_3$ receptors.

A compound which has a $pEC_{50}$ of less than 5 and an $E_{max}$ of less than 30 against the muscarinic $M_2$ and $M_3$ receptor subtypes.

A compound for use in the treatment of a disease or condition mediated by the muscarinic $M_1$ receptor.

A compound for use in the treatment of a disease or condition mediated by the muscarinic $M_1$ receptor or muscarinic $M_4$ receptor.

By virtue of their muscarinic $M_4$ receptor agonist activity, compounds described herein can be used in the treatment of Alzheimer's disease, dementia with Lewy bodies, schizophrenia and other psychotic disorders, cognitive disorders and other diseases mediated by the muscarinic $M_4$ receptor, and can also be used in the treatment of various types of pain.

Accordingly, with respect to the compounds described herein, also described herein is:
A compound for use in the treatment of a cognitive disorder or psychotic disorder.

A compound for use in the treatment of a cognitive disorder or psychotic disorder, wherein the cognitive disorder or psychotic disorder comprises, arises from or is associated with a condition selected from cognitive impairment, Mild Cognitive Impairment (MCI), (including amnestic MCI and nonamnestic MCI, and including mild cognitive impairment due to Alzheimer's disease and/or prodromal Alzheimer's disease), frontotemporal dementia, vascular dementia, dementia with Lewy bodies, presenile dementia, senile dementia, Friederich's ataxia, Down's syndrome, Huntington's chorea, hyperkinesia, mania, Tourette's syndrome, Alzheimer's disease (including prodromal Alzheimer's disease and stages 1, 2, and 3 early Alzheimer's disease as defined by the US Food and Drug Administration's "Early Alzheimer's disease: Developing Drugs for Treatment" available at fda.gov/downloads/Drugs/GuidanceComplianceRegulatoryInformation/-Guidances/UCM5967 28.pdf), progressive supranuclear palsy, impairment of cognitive functions including attention, orientation, learning disorders, memory (i.e. memory disorders, amnesia, amnesic disorders, transient global amnesia syndrome and age-associated memory impairment) and language function; cognitive impairment as a result of stroke, Huntington's disease, Pick disease, AIDS-related dementia or other dementia states such as multi-infarct dementia, alcoholic dementia, hypothyroidism-related dementia, and dementia associated to other degenerative disorders such as cerebellar atrophy and amyotrophic lateral sclerosis; other acute or sub-acute conditions that may cause cognitive decline such as delirium or depression (pseudodementia states) trauma, head trauma, age related cognitive decline, stroke, neurodegeneration, drug-induced states, neurotoxic agents, age related cognitive impairment, autism related cognitive impairment, Down's syndrome, cognitive deficit related to psychosis, and post-electroconvulsive treatment related cognitive disorders; cognitive disorders due to drug abuse or drug withdrawal including nicotine, cannabis, amphetamine, cocaine, Attention Deficit Hyperactivity Disorder (ADHD) and dyskinetic disorders such as Parkinson's disease, neuroleptic-induced parkinsonism, and tardive dyskinesias, schizophrenia, schizophreniform diseases, psychotic depression, mania, acute mania, paranoid, hallucinogenic and delusional disorders, personality disorders, obsessive compulsive disorders, schizotypal disorders, delusional disorders, psychosis due to malignancy, metabolic disorder, endocrine disease or narcolepsy, psychosis due to drug abuse or drug withdrawal, bipolar disorders and schizo-affective disorder.

A compound for use in the treatment of a cognitive disorder or psychotic disorder, wherein the cognitive disorder or psychotic disorder is, comprises, arises from or is associated with a condition selected from cognitive impairment, Mild Cognitive Impairment (MCI), (including amnestic MCI and nonamnestic MCI, and including mild cognitive impairment due to Alzheimer's disease and/or prodromal Alzheimer's disease), frontotemporal dementia, vascular dementia, dementia with Lewy bodies, presenile dementia, senile dementia, Friederich's ataxia, Down's syndrome, Huntington's chorea, hyperkinesia, mania, Tourette's syndrome, Alzheimer's disease (including prodromal Alzheimer's disease and stages 1, 2, and 3 early Alzheimer's disease as defined by the US Food and Drug Administration's "Early Alzheimer's disease: Developing Drugs for Treatment" available at fda.gov/downloads/Drugs/GuidanceComplianceRegulatoryInformation/-Guidances/UCM5967 28.pdf), progressive supranuclear palsy, impairment of cognitive functions including attention, orientation, learning disorders, memory (i.e. memory disorders, amnesia, amnesic disorders, transient global amnesia syndrome and age-associated memory impairment) and language function; cognitive impairment as a result of stroke, Huntington's disease, Pick disease, AIDS-related dementia or other dementia states such as multi-infarct dementia,

alcoholic dementia, hypothyroidism-related dementia, and dementia associated to other degenerative disorders such as cerebellar atrophy and amyotrophic lateral sclerosis; other acute or sub-acute conditions that may cause cognitive decline such as delirium or depression (pseudodementia states) trauma, head trauma, age related cognitive decline, stroke, neurodegeneration, drug-induced states, neurotoxic agents, age related cognitive impairment, autism related cognitive impairment, Down's syndrome, cognitive deficit related to psychosis, and post-electroconvulsive treatment related cognitive disorders; cognitive disorders due to drug abuse or drug withdrawal including nicotine, cannabis, amphetamine, cocaine, Attention Deficit Hyperactivity Disorder (ADHD) and dyskinetic disorders such as Parkinson's disease, neuroleptic-induced parkinsonism, and tardive dyskinesias, schizophrenia, schizophreniform diseases, psychotic depression, mania, acute mania, paranoid, hallucinogenic and delusional disorders, personality disorders, obsessive compulsive disorders, schizotypal disorders, delusional disorders, psychosis due to malignancy, metabolic disorder, endocrine disease or narcolepsy, psychosis due to drug abuse or drug withdrawal, bipolar disorders, schizo-affective disorder, and Alzheimer's psychosis.

A compound for use in the treatment of schizophrenia, Alzheimer's disease, Alzheimer's psychosis, or bipolar disorders.

A compound for use in the treatment of schizophrenia, Alzheimer's psychosis, or bipolar disorders.

A compound for use in the treatment of Alzheimer's disease.

A compound for use in the treatment of dementia with Lewy bodies.

A compound for use in the treatment of Schizophrenia.

A compound for use in the treatment of bipolar disorders.

A compound for use in the treatment of Alzheimer's psychosis.

A method of treatment of a cognitive disorder in a subject (e.g., a mammalian patient such as a human, e.g. a human in need of such treatment), which method comprises the administration of a therapeutically effective dose of a compound described herein.

A method of treatment of a cognitive disorder in a subject (e.g., a mammalian patient such as a human, e.g., a human in need of such treatment), which method comprises the administration of a therapeutically effective dose of a compound described herein, wherein the cognitive disorder comprises, arises from or is associated with a condition as defined in above.

A method of treatment of a cognitive disorder in a subject (e.g., a mammalian patient such as a human, e.g., a human in need of such treatment), which method comprises the administration of a therapeutically effective dose of a compound described herein, wherein the cognitive disorder arises from or is associated with Alzheimer's disease.

A method of treatment of a cognitive disorder in a subject (e.g., a mammalian patient such as a human, e.g., a human in need of such treatment), which method comprises the administration of a therapeutically effective dose of a compound described herein, wherein the cognitive disorder is dementia with Lewy bodies.

A method of treatment of a cognitive disorder in a subject (e.g., a mammalian patient such as a human, e.g., a human in need of such treatment), which method comprises the administration of a therapeutically effective dose of a compound described herein, wherein the cognitive disorder is Schizophrenia.

A method of treatment of a cognitive disorder in a subject (e.g., a mammalian patient such as a human, e.g., a human in need of such treatment), which method comprises the administration of a therapeutically effective dose of a compound described herein, wherein the cognitive disorder is a bipolar disorder.

A method of treatment of a cognitive disorder in a subject (e.g., a mammalian patient such as a human, e.g., a human in need of such treatment), which method comprises the administration of a therapeutically effective dose of a compound described herein, wherein the cognitive disorder is Alzheimer's psychosis.

A method of treatment of a psychotic disorder in a subject (e.g., a mammalian patient such as a human, e.g. a human in need of such treatment), which method comprises the administration of a therapeutically effective dose of a compound described herein.

A method of treatment of a psychotic disorder in a subject (e.g., a mammalian patient such as a human, e.g., a human in need of such treatment), which method comprises the administration of a therapeutically effective dose of a compound described herein, wherein the psychotic disorder comprises, arises from or is associated with a condition as defined in above.

A method of treatment of a psychotic disorder in a subject (e.g., a mammalian patient such as a human, e.g., a human in need of such treatment), which method comprises the administration of a therapeutically effective dose of a compound described herein, wherein the psychotic disorder arises from or is associated with Alzheimer's disease.

A method of treatment of a psychotic disorder in a subject (e.g., a mammalian patient such as a human, e.g., a human in need of such treatment), which method comprises the administration of a therapeutically effective dose of a compound described herein, wherein the psychotic disorder is dementia with Lewy bodies.

A method of treatment of a psychotic disorder in a subject (e.g., a mammalian patient such as a human, e.g., a human in need of such treatment), which method comprises the administration of a therapeutically effective dose of a compound described herein, wherein the psychotic disorder is Schizophrenia.

A method of treatment of a psychotic disorder in a subject (e.g., a mammalian patient such as a human, e.g., a human in need of such treatment), which method comprises the administration of a therapeutically effective dose of a compound described herein, wherein the psychotic disorder is a bipolar disorder.

A method of treatment of a psychotic disorder in a subject (e.g., a mammalian patient such as a human, e.g., a human in need of such treatment), which method comprises the administration of a therapeutically effective dose of a compound described herein, wherein the psychotic disorder is Alzheimer's psychosis.

A compound for use in the treatment or lessening the severity of acute, chronic, neuropathic, or inflammatory pain, arthritis, migraine, cluster headaches, trigeminal neuralgia, herpetic neuralgia, general neuralgias, visceral pain, osteoarthritis pain, postherpetic neuralgia, diabetic neuropathy, radicular pain, sciatica, back pain, head or neck pain, severe or intractable pain, nociceptive pain, breakthrough pain, postsurgical pain, or cancer pain.

A compound for use in the treatment of peripheral disorders such as reduction of intra ocular pressure in Glaucoma and treatment of dry eyes and dry mouth including Sjogren's Syndrome.

The use of a compound described herein for the treatment of skin lesions for example due to pemphigus vulgaris, dermatitis herpetiformis, pemphigoid and other blistering skin conditions.

The use of a compound described herein for treating, preventing, ameliorating or reversing conditions associated with altered gastro-intestinal function and motility such as functional dyspepsia, irritable bowel syndrome, gastroesophageal acid reflux (GER) and esophageal dysmotility, symptoms of gastroparesis and chronic diarrhea.

The use of a compound described herein for the treatment of olfactory dysfunction such as Bosma-Henkin-Christiansen syndrome, chemical poisoning (e.g. selenium and silver), hypopituitarism, Kallmann Syndrome, skull fractures, tumour therapy and underactive thyroid gland.

The use of a compound described herein for the treatment of addiction.

The use of a compound described herein for the treatment of movement disorders such as Parkinson's disease, ADHD, Huntingdon's disease, Tourette's syndrome and other syndromes associated with dopaminergic dysfunction as an underlying pathogenetic factor driving disease.

The use of a compound described herein for the treatment of behavioural and psychological symptoms of dementia (BPSD; including agitation, verbal aggressiveness, physical aggressiveness, depression, anxiety, abnormal motor behaviour, elated mood, irritability, apathy, disinhibition, impulsivity. delusions, hallucinations, sleep changes, and appetite changes).

## Methods for Preparation

Compounds described herein can be prepared in accordance with synthetic methods known to the skilled person and as well as the methods described herein. The compounds may also be prepared according to the methods described in WO2015/118342. Once formed, one compound described herein or a protected derivative thereof, can be converted into another compound described herein by methods well known to the skilled person. For example, a compound described herein may be converted into a salt form or alternative salt form of a compound described herein. Examples of such synthetic procedures are set out in standard texts such as *Advanced Organic Chemistry* and *Organic Syntheses* (see references above) or Fiesers' Reagents for Organic Synthesis, Volumes 1-17, John Wiley, edited by Mary Fieser (ISBN: 0-471-58283-2).

It may be necessary to protect one or more groups to prevent reaction from taking place at an undesirable location on the molecule. Examples of protecting groups, and methods of protecting and deprotecting functional groups, can be found in Protective Groups in Organic Synthesis (T. Greene and P. Wuts; 3rd Edition; John Wiley and Sons, 1999).

Compounds made by the foregoing methods may be isolated and purified by any of a variety of methods well known to those skilled in the art and examples of such methods include recrystallisation and chromatographic techniques such as column chromatography (e.g. flash chromatography) and HPLC.

As used herein, the term "reacting" is used as known in the art and generally refers to the bringing together of chemical reagents in such a manner so as to allow their interaction at the molecular level to achieve a chemical or physical transformation. In some embodiments, the reacting involves two reagents, wherein one or more equivalents of second reagent are used with respect to the first reagent. The reacting steps of the processes described herein can be conducted for a time and under conditions suitable for preparing the identified product.

The reactions of the processes described herein can be carried out in suitable solvents which can be readily selected by one of skill in the art of organic synthesis. Suitable solvents can be substantially nonreactive with the starting materials (reactants), the intermediates, or products at the temperatures at which the reactions are carried out, e.g., temperatures which can range from the solvent's freezing temperature to the solvent's boiling temperature. A given reaction can be carried out in one solvent or a mixture of more than one solvent. Depending on the particular reaction step, suitable solvents for a particular reaction step can be selected.

Suitable solvents can include halogenated solvents such as carbon tetrachloride, bromodichloromethane, dibromochloromethane, bromoform, chloroform, bromochloromethane, dibromomethane, butyl chloride, dichloromethane, tetrachloroethylene, trichloroethylene, 1,1,1-trichloroethane, 1,1,2-trichloroethane, 1,1-dichloroethane, 2-chloropropane, 1,2-dichloroethane, 1,2-dibromoethane, hexafluorobenzene, 1,2,4-trichlorobenzene, 1,2-dichlorobenzene, chlorobenzene, fluorobenzene, mixtures thereof and the like.

Suitable ether solvents include: dimethoxymethane, tetrahydrofuran, 1,3-dioxane, 1,4-dioxane, furan, diethyl ether, ethylene glycol dimethyl ether, ethylene glycol diethyl ether, diethylene glycol dimethyl ether, diethylene glycol diethyl ether, triethylene glycol dimethyl ether, anisole, t-butyl methyl ether, mixtures thereof and the like.

Suitable protic solvents can include, by way of example and without limitation, water, methanol, ethanol, 2-nitroethanol, 2-fluoroethanol, 2,2,2-trifluoroethanol, ethylene glycol, 1-propanol, 2-propanol, 2-methoxyethanol, 1-butanol, 2-butanol, i-butyl alcohol, t-butyl alcohol, 2-ethoxyethanol, diethylene glycol, 1-, 2-, or 3- pentanol, neo-pentyl alcohol, t-pentyl alcohol, diethylene glycol monomethyl ether, diethylene glycol monoethyl ether, cyclohexanol, benzyl alcohol, phenol, or glycerol.

Suitable aprotic solvents can include, by way of example and without limitation, tetrahydrofuran (THF), N,N-dimethylformamide (DMF), N,N-dimethylacetamide (DMA), 1,3-dimethyl-3,4,5,6-tetrahydro-2(1H)-pyrimidinone (DMPU), 1,3-dimethyl-2-imidazolidinone (DMI), N methylpyrrolidinone (NMP), formamide, N-methylacetamide, N-methylformamide, acetonitrile, dimethyl sulfoxide, propionitrile, ethyl formate, methyl acetate, hexachloroacetone, acetone, ethyl methyl ketone, ethyl acetate, sulfolane, N,N-dimethylpropionamide, tetramethylurea, nitromethane, nitrobenzene, or hexamethylphosphoramide.

Suitable hydrocarbon solvents include benzene, cyclohexane, pentane, hexane, toluene, cycloheptane, methylcyclohexane, heptane, ethylbenzene, m-, o-, or p-xylene, octane, indane, nonane, or naphthalene.

Suitable aqueous buffer solvents include phosphate buffers, tris buffers, barbital buffers, BES (N, N-Bis-(2-hydroxyethyl)-2-aminoethanesulphonic acid) buffers, and MOPS (3-(N-morpholino) propane sulphonic acid) buffers.

The reactions of the processes described herein can be carried out at appropriate temperatures which can be readily determined by the skilled artisan. Reaction temperatures will depend on, for example, the melting and boiling points of the reagents and solvent, if present; the thermodynamics of the reaction (e.g., vigorously exothermic reactions may need to be carried out at reduced temperatures); the kinetics of the reaction (e.g., a high activation energy barrier may need elevated temperatures); and the temperature range over which any enzymatic component is active / the temperature at which any enzymatic component is denaturated.

The expressions, "ambient temperature" and "room temperature" or "rt" as used herein, are understood in the art, and refer generally to a temperature, e.g., a reaction temperature, that is about the temperature of the room in which the reaction is carried out, for example, a temperature from about 20 °C to about 30 °C.

The reactions of the processes described herein can be carried out in air or under an inert atmosphere. Typically, reactions containing reagents or products that are substantially reactive with air can be carried out using air-sensitive synthetic techniques that are well known to the skilled artisan.

Ketoreductase Enzymes

Ketoreductases (KREDs, also called 'alcohol dehydrogenases' ADHs, or 'carbonyl reductases') catalyze the reduction of aldehydes and ketones to the corresponding primary and secondary alcohols, respectively.

The reduction catalyzed by KREDs requires a reduced cofactor as electron donor. Some KREDs use reduced nicotinamide adenine dinucleotide (NADH) as a cofactor, other KREDs use reduced nicotinamide adenine dinucleotide phosphate (NADPH) and some ketoreductases accept both, NADH and NADPH.

The in vitro use of KREDs in reduction processes requires a cofactor regeneration system to regenerate NADPH from $NADP^+$ or NADH from $NAD^+$. Common cofactor regeneration systems are glucose dehydrogenase (GDH) which takes glucose as a feedstock, or formate dehydrogenase which takes formate as a feedstock. These cofactor regeneration systems may be used in conjunction with KREDs.

KREDs are ubiquitous enzymes found in all kingdoms of life. Well-known, commercially available KREDs are derived from horse liver (HLADH), baker's yeast (YADH) and from bacteria, such as Thermoanaerobium brockii (TBADH) and Lactobacillus kefir (LKADH).

For industrial applications it is desirable to employ KREDs with a high specific activity and stereoselectivity. Another important criterion in the industrial use of KREDs is a long process stability, which often correlates with a high stability at elevated temperatures and a high solvent stability. It may also be desirable to employ KREDs with a high stereospecificity.

Enzyme A, described herein and used in the processes described herein is a ketoreductase enzyme (KRED) having an amino acid sequence of SEQ ID NO: 1:

MNSVQSQGTALITGASSGIGAIYAERLAARGFDLLLVARDKARLDSAASQLRDAHGVQVEVW
KADLTQKDDVIKLEQRLRSDSSISLLINNAGVAADGPLANADMDQLERLIQLNITAVTRLAS
AAAASFAKAGRGTIINIASVVALFPERFNATYTASKAYVLSLTQSLNAELEGSGVQIQAVLP
GVTRTEIWERSGIDASGIPAEMVMDAGEMVDAALAGLDQGELVTIPSLPDAGEWQSFVAARH
VMAPNLSRSAAAQRYKSGH.

Enzyme A may be produced by methods known in the art. For instance, methods for expression of enzymes in cellular (e.g., microbial) expression systems are well known and routine to those skilled in the art.

Enzyme A may be provided by or generated from a host cell (e.g., a microbial cell, such as *E. Coli*) comprising a polynucleotide and/or expression vector that encodes Enzyme A. The host cell can be TOP10 *E. Coli.* Enzyme A may be produced using the method disclosed in the Examples.

The polynucleotide may be DNA or RNA. The polynucleotide may be single-stranded or double-stranded. The polynucleotide may be provided in isolated/purified form, or within a host cell.

Because of the knowledge of the codons corresponding to the various amino acids, availability of a polypeptide sequence provides a description of all the polynucleotides capable of encoding the subject polypeptide. The degeneracy of the genetic code, where the same amino acids are encoded by alternative or synonymous codons allows an extremely large number of nucleic acids to be made, all of which encode a disclosed enzyme. Thus, having identified a particular amino acid sequence, those skilled in the art could make any number of different nucleic acids by simply modifying the sequence of one or more codons in a way which does not change the amino acid sequence of the protein. In this regard, the present disclosure specifically contemplates each and every possible variation of polynucleotides that could be made by selecting combinations based on the possible codon choices.

Enzyme A may be encoded by a polynucleotide and/or expression vector comprising a nucleotide sequence according to SEQ ID NO: 2:

ATGAATTCTGTGCAGTCTCAAGGTACGGCTCTGATCACTGGCGCCTCGTCCGGTATCGGTGC
GATTTACGCCGAGCGTCTGGCGGCGTGGTTTTGATCTGTTGCTGGTGGCCCGTGACAAGG
CCCGTCTGGACAGCGCCGCCAGCCAATTGCGCGACGCTCACGGTGTGCAGGTCGAGGTGTGG
AAAGCGGATCTGACCCAAAAGGACGACGTGATCAAACTCGAACAGCGCTTGCGCAGCGATTC
GAGCATCAGCCTGCTGATTAACAATGCCGGCGTGGCCGCCGACGGCCCGCTGGCCAATGCCG
ACATGGATCAACTGGAACGCCTGATCCAGTTGAACATCACCGCCGTCACGCGTCTGGCGTCG
GCCGCCGCTGCCAGTTTCGCCAAGGCAGGTCGCGGCACGATCATCAACATCGCCTCGGTCGT
GGCGCTGTTCCCCGAGCGTTTCAATGCGACCTACACCGCCAGCAAGGCCTATGTGTTGAGTC
TGACCCAATCGTTGAACGCGGAGCTCGAAGGCTCCGGCGTGCAGATCCAGGCCGTGCTGCCG
GGCGTGACCCGCACTGAAATCTGGGAGCGTTCGGGGATCGACGCCAGTGGCATTCCGGCGGA
AATGGTCATGGACGCCGGGGAGATGGTGGATGCCGCCCTGGCCGGTCTGGATCAGGGCGAAC
TGGTCACCATTCCATCGCTGCCCGATGCCGGCGAATGGCAGTCGTTTGTGGCGGCGCGCCAT
GTCATGGCGCCGAACCTTTCGCGCAGCGCTGCAGCCCAGCGCTACAAATCAGGTCATTGA.

The polynucleotide may consist of SEQ ID NO: 2.

The polynucleotide may be operatively linked to one or more heterologous regulatory or control sequences that control gene expression to create a recombinant polynucleotide capable of expressing the polypeptide. Expression constructs containing a heterologous polynucleotide encoding the engineered ketoreductase can be introduced into appropriate host cells to express the corresponding ketoreductase. The polynucleotides encoding the ketoreductase enzymes may be codon optimized for optimal production from the host organism selected for expression. For example, preferred codons used in bacteria are used to express the gene in bacteria; preferred codons used in yeast are used for expression in yeast; and preferred codons used in mammals are used for expression in mammalian cells.

A polynucleotide encoding a disclosed enzyme may be manipulated in a variety of ways to provide for expression of the polypeptide. Manipulation of the isolated polynucleotide prior to its insertion into an expression vector may be desirable or necessary depending on the expression vector. The techniques for modifying polynucleotides and nucleic acid sequences utilizing recombinant DNA methods are well known in the art. Guidance is provided in Sambrook et al., 2001, Molecular Cloning: A Laboratory Manual, 3rd Ed., Cold Spring Harbor Laboratory Press; and Current Protocols in Molecular Biology, Ausubel. F. ed., Greene Pub. Associates, 1998, updates to 2006.

The control sequence may be an appropriate promoter sequence, which can be obtained from genes encoding extracellular or intracellular polypeptides either homologous or heterologous to the host cell. For bacterial host cells, suitable promoters for directing transcription of the nucleic acid constructs of the present disclosure, include the promoters obtained from the E. coli lac operon, Streptomyces coelicolor agarase gene (dagA), Bacillus subtilis levansucrase gene (sacB), Bacillus licheniformis alpha-amylase gene (amyL), Bacillus stearothermophilus maltogenic amylase gene (amyM), Bacillus amyloliquefaciens alpha-amylase gene (amyQ), Bacillus licheniformis penicillinase gene (penP), Bacillus subtilis xylA and xylB genes, and prokaryotic beta-lactamase gene (Villa-Kamaroff et al., 1978, Proc. Natl Acad. Sci. USA 75: 3727-3731), as well as the tac promoter (DeBoer et al., 1983, Proc. Natl Acad. Sci. USA 80: 21-25). Further promoters are described in "Useful proteins from recombinant bacteria" in Scientific American, 1980, 242:74-94; and in Sambrook et al., supra.

The control sequence may also be a signal peptide coding region that codes for an amino acid sequence linked to the amino terminus of a polypeptide and directs the encoded polypeptide into the cell's secretory pathway. The 5' end of the coding sequence of the nucleic acid sequence may inherently contain a signal peptide coding region naturally linked in translation reading frame with the segment of the coding region that encodes the secreted polypeptide. Alternatively, the 5' end of the coding sequence may contain a signal peptide coding region that is foreign to the coding sequence. The foreign signal peptide coding region may be required where the coding sequence does not naturally contain a signal peptide coding region.

Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used.

Any suitable vectors, promoters, enhancers and termination codons known in the art may be used to express a polypeptide from a vector as described herein. The vector may be a plasmid, phage, MAC, virus, etc. The plasmid may be a pET28a plasmid (see Nature 2020; https://doi.org/10.1038/s42003-020-0939-8).

The polynucleotide and/or expression vector may be synthesised by conventional DNA synthesis techniques. Where the sequence of the engineered polypeptide is known, the polynucleotides encoding the enzyme can be prepared by standard solid-phase methods, according to known synthetic methods. Fragments of up to about 100 bases can be individually synthesized, then joined (e.g., by enzymatic or chemical litigation methods, or polymerase mediated methods) to form any desired continuous sequence. For example, the disclosed polynucleotides can be prepared by chemical synthesis using, e.g., the classical phosphoramidite method described by Beaucage et al., 1981, Tet Lett 22:1859-69, or the method described by Matthes et al., 1984, EMBO J. 3:801-05, e.g., as it is typically practiced in automated synthetic methods. According to the phosphoramidite method, oligonucleotides are synthesized, e.g., in an automatic DNA synthesizer, purified, annealed, ligated and cloned in appropriate vectors. In addition, essentially any nucleic acid can be obtained from any of a variety of commercial sources, such as The Midland Certified Reagent Company, Midland, TX, The Great American Gene Company, Ramona, CA, ExpressGen Inc. Chicago, IL, Operon Technologies Inc., Alameda, CA, and many others.

## Pharmaceutical Formulations

While it is possible for the active compound to be administered alone, it is preferable to present it as a pharmaceutical composition (e.g., formulation).

Accordingly, there is provided a pharmaceutical composition comprising at least one compound described herein together with at least one pharmaceutically acceptable excipient.

The composition may be a tablet composition. The composition may be a capsule composition.

The pharmaceutically acceptable excipient(s) can be selected from, for example, carriers (e.g. a solid, liquid or semi-solid carrier), adjuvants, diluents (e.g solid diluents such as fillers or bulking agents; and liquid diluents such as solvents and co-solvents), granulating agents, binders, flow aids, coating agents, release-controlling agents (e.g. release retarding or delaying polymers or waxes), binding agents, disintegrants, buffering agents, lubricants, preservatives, anti-fungal and antibacterial agents, antioxidants, buffering agents, tonicity-adjusting agents, thickening agents, flavouring agents, sweeteners, pigments, plasticizers, taste masking agents, stabilisers or any other excipients conventionally used in pharmaceutical compositions.

The term "pharmaceutically acceptable" as used herein means compounds, materials, compositions, and/or dosage forms which are, within the scope of sound medical judgment, suitable for use in contact with the tissues of a

subject (e.g. a human subject) without excessive toxicity, irritation, allergic response, or other problem or complication, commensurate with a reasonable benefit/risk ratio. Each excipient must also be "acceptable" in the sense of being compatible with the other ingredients of the formulation.

Pharmaceutical compositions containing compounds described herein can be formulated in accordance with known techniques, see for example, Remington's Pharmaceutical Sciences, Mack Publishing Company, Easton, PA, USA.

The pharmaceutical compositions can be in any form suitable for oral, parenteral, topical, intranasal, intrabronchial, sublingual, ophthalmic, otic, rectal, intra-vaginal, or transdermal administration. Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches. Tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here. Tablets may be designed to release the drug either upon contact with stomach fluids (immediate release tablets) or to release in a controlled manner (controlled release tablets) over a prolonged period of time or with a specific region of the GI tract.

The pharmaceutical compositions typically comprise from approximately 1% (w/w) to approximately 95%, preferably% (w/w) active ingredient and from 99% (w/w) to 5% (w/w) of a pharmaceutically acceptable excipient (for example as defined above) or combination of such excipients. Preferably, the compositions comprise from approximately 20% (w/w) to approximately 90% (w/w) active ingredient and from 80% (w/w) to 10% of a pharmaceutically excipient or combination of excipients. The pharmaceutical compositions comprise from approximately 1% to approximately 95%, preferably from approximately 20% to approximately 90%, active ingredient. Pharmaceutical compositions described herein may be, for example, in unit dose form, such as in the form of ampoules, vials, suppositories, prefilled syringes, dragées, powders, tablets or capsules.

Tablets and capsules may contain, for example, 0-20% disintegrants, 0-5% lubricants, 0-5% flow aids and/or 0-99% (w/w) fillers/ or bulking agents (depending on drug dose). They may also contain 0-10% (w/w) polymer binders, 0-5% (w/w) antioxidants, 0-5% (w/w) pigments. Slow release tablets would in addition typically contain 0-99% (w/w) release-controlling (e.g. delaying) polymers (depending on dose). The film coats of the tablet or capsule typically contain 0-10% (w/w) polymers, 0-3% (w/w) pigments, and/or 0-2% (w/w) plasticizers.

Parenteral formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils.

The pharmaceutical formulations may be presented to a patient in "patient packs" containing an entire course of treatment in a single package, usually a blister pack.

The compounds described herein will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular sub-ranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

For oral compositions, a unit dosage form may contain from 1 milligram to 2 grams, more typically 10 milligrams to 1 gram, for example 50 milligrams to 1 gram, e.g. 100 miligrams to 1 gram, of active compound.

The active compound will be administered to a patient in need thereof (for example a human or animal patient) in an amount sufficient to achieve the desired therapeutic effect (effective amount). The precise amounts of compound administered may be determined by a supervising physician in accordance with standard procedures.

## EXAMPLES

The invention will now be illustrated, but not limited, by reference to the specific embodiments described in the following example compounds and methods of synthesis. Biological activity of one isomer of ethyl 2-[4-(1-methyl-1H-pyrazol-5-yl)piperidin-1-yl]-6-azaspiro[3.4]octane-6-carboxylate is demonstrated in WO2015/118342.

*General Methods and Materials*

The following general methods and materials are exemplary for the methods and materials used during the processes described hereinbelow.

NMR

NMR analysis was performed on a Bruker 400 Avance III NMR spectrometer.

X-Ray Powder Diffraction (XRPD):

The XRPD analysis was performed on a Rigaku Powder X-Ray Diffractometer Miniflex 600 Serial Number BD70000351-01. For analysis, approximately 0.5-1 mg of sample was added to a PXRD zero-background sample holder. The powder was pressed down gently with a piece of weigh paper, and the sample holder was placed in the sample changer. Run Parameters: Miniflex Counter Detector, Kb Filter (x2), Scan Axis Theta/2-Theta, Mode Continuous, Start (deg) 2.0, Stop (deg) 45.0, Step (deg) 0.020, Speed (deg/min) 10.0, Spinyes, Voltage (kV) 40, Current (mA) 15.

It is understood that peak intensities can vary from one diffractogram to another for the same crystalline form based on any number of factors that are known to those skilled in the art, such as, preferred orientation effects, preparation technique, the sample mounting procedure, the instrument employed, etc. In some instances, peak intensities can vary rather dramatically. Accordingly, the diffraction peak intensities shown herein are illustrative and identical diffraction peak intensities are not necessarily required. One skilled in the art would readily be capable of comparing the diffractogram provided herein with a diffractogram generated for an unknown crystal form and confirm whether the diffractogram is characterising the same crystal form as provided herein or a different form.

Thermal Analysis

The Differential Scanning Calorimetry (DSC) and Thermal Gravimetric Analysis (TGA) analyses were performed on TA Instruments Discovery 2500 calorimeter with serial number: 2500-00547 (DSC) and Discovery 5500 with serial number: 5500-0126 (TGA).

For TGA analysis, a standard aluminum sample pan was placed into the platinum TGA pan and the blank was tared with the instrument. Approximately 1-5 mg of sample was added to the standard aluminum pan and analyzed at 10 °C/min up to 400°C.

For the DSC analysis, obtained and recorded the weight of a $T_{zero}$ pan and a $T_{zero}$ lid. ~1-3 mg of sample was weighed into the $T_{zero}$ Pan and the $T_{zero}$ lid was pressed on. The pan was transferred to the DSC autosampler for analysis. The method for analysis was a ramp at 10 °C/min to 225 °C. The reference pan was prepared with the same procedures, absent of the sample.

For DSC and TGA, it is known that the temperatures observed for thermal events will depend upon sample purity and may also depend on the rate of temperature change, as well as sample preparation technique, and the instrument employed. Thus, the values reported herein relating to DSC thermograms can vary by plus or minus 3 °C. For example, "about 176 °C" means 176 °C ± 3 °C. The values reported herein relating to DSC thermograms can also vary by plus or minus 10 joules per gram (*i.e.*, ± 10 joules per gram).

Hygroscopicity

Dynamic Vapor Sorption (DVS) analysis was performed on a Discovery SA-0180 vapor sorption analyzer (DVS) with serial number 958000.901. For DVS analysis, approximately 3-10 mg of sample was placed into a quartz crucible. The crucible was then transferred to the sorption analyzer for ~1 hour of drying at 60 °C, followed by analysis at 25°C with 10% RH steps not exceeding 240 minutes, from 0% RH up to 90% RH with stabilization criteria of 0.01%.

Microscopy

Polarized Light Microscopy (PLM ) and Scanning Electron Microscopy (SEM):

Imaging is performed on a Leica DM LM/P microscope equipped with a DMC2900 camera. For the analysis, approximately 1 mg of sample was added to a microscope slide. The slide was then positioned on the microscope where polarized images were captured.

Scanning Electron Microscopy (SEM) was used to assess the morphology and particle size. Images were taken using a Phenom ProX scanning electron microscope with serial number MVE052018-0480-S. The samples were added to

a section of an SEM mount using carbon conductive double coated tape. The mount was tapped to remove excess particles, then gold sputter coated with a Cressington 108. Imaging was performed at 5-15 kV with an SED detector.

Chiral RP-HPLC method:

System: Shimadzu LC-40
Column: Phenomenex Lux i-Amylose3 250 x 4.6mm 5μm
Mobile phase: water and acetonitrile mixture (60/40 water/acetonitrile) with 0.1% phosphoric acid.
Flow rate: 1 mL/min.
Column temperature: 40°C.
Run time: 15 min.
Detection: refractive index detector (RID) was used.
Retention times:

| Compound | Retention time |
|---|---|
| 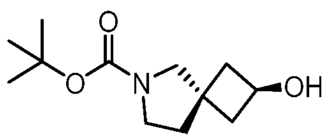 | 5.6 min |
| 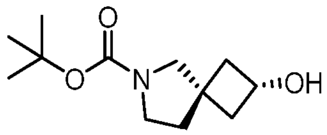 | 7.1 min |
| 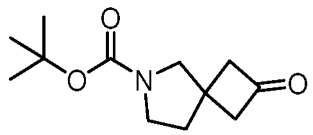 | 9.9 min |

Sample preparation: To 0.5 mL reaction sample 0.5 mL acetonitrile was added and mixed until homogeneity. The sample was placed into an ultrasonic bath for 5 min and then centrifuged at 12.000 x g for 5 min. An aliquot of the prepared sample was transferred to a glass vial and analyzed using the chiral RP-HPLC method.

Calculation of diastereomeric excess:

Diastereomeric excess (%de) was calculated using the following equation:

$$\%de = \frac{c_{trans\ isomer}}{(c_{trans\ isomer} + c_{cis\ isomer})} * 100$$

Where $C_{trans\,isomer}$ corresponds to the %Area of the peak in an HPLC chromatograph generated using the Chiral RP-HPLC method, and having a retention time of 5.6 min; and $C_{cis\ isomer}$ corresponds to the %Area of the peak in the HPLC chromatograph generated using the Chiral RP-HPLC method, and having a retention time of 7.1 min.

Screening of KRED enzymes

A photometric assay was used to screen a range of KRED enzymes for their utility in the transformation of Formula (B) to Formula (A). 288 diverse KRED enzymes were provided as dried enzyme formulation in 96 microtiter plates. The enzymes were solubilized in an appropriate buffer solution to generate a clear solution, necessary for photometric measurement and screening. The photometric measurement recorded the decrease of the absorption at 340 nm, which corresponds with an enzyme consuming the NADPH cofactor for a reductive reaction.

The collected data was analyzed and enzymes selected, which either showed a negative slope (lowest numbers

chosen first) over 10 min of reaction time or an overall absolute absorbance of lower than 0.7 and decreasing abruption over 10 min. A low starting absorbance is an indication for a highly active enzyme, that consumes the cofactor faster, than the technician can transfer the MTP to the MTP-reader.

Those enzymes meeting the selection criteria were submitted to a 0.5 mL biocatalysis reaction and HPLC verification using the chiral RP-HPLC method.

Biocatalysis conditions:

- Ketone 20 g/L (88 mM)
- NADP 5 mM
- DMSO 5% (v/v)
- $MgCl_2$ 2 mM
- Glucose 178 mM (2 equiv.)
- GDH (glucose dehydrogenase, CDX-901, Codexis Inc.) 1 g/L
- Potassium phosphate buffer 100 mM pH 7.0
- 0.5 mL, 30°C, 1000 rpm shaking, 24 h reaction time

As shown in Table 1, below, Enzyme A significantly out-performed the next best enzymes (Enzymes B-F) for conversion of starting material, while maintaining the highest level of stereoselectivity (diastereomeric excess) observed for the trans-alcohol (Formula (A)).

Table 1

| **Enzyme** | **conversion** | **%de** |
|---|---|---|
| Enzyme A | 100% (98.7*) | 97% (97*) |
| Enzyme B | 41.2% | 97% |
| Enzyme C | 37.0% | 94% |
| Enzyme D | 46.1% | 94% |
| Enzyme E | 42.8% | 94% |
| Enzyme F | 19.1% | 91% |

Production of synthetic DNA encoding Enzyme A

Synthetic DNA having SEQ ID NO: 2 and encoding Enzyme A was synthesised with codon optimisation for E. coli expression.

SEQ ID NO: 2

ATGAATTCTGTGCAGTCTCAAGGTACGGCTCTGATCACTGGCGCCTCGTCCGGTATCGGTGC
GATTTACGCCGAGCGTCTGGCGGCGTGGTTTTGATCTGTTGCTGGTGGCCCGTGACAAGG
CCCGTCTGGACAGCGCCGCCAGCCAATTGCGCGACGCTCACGGTGTGCAGGTCGAGGTGTGG
AAAGCGGATCTGACCCAAAAGGACGACGTGATCAAACTCGAACAGCGCTTGCGCAGCGATTC
GAGCATCAGCCTGCTGATTAACAATGCCGGCGTGGCCGCCGACGGCCCGCTGGCCAATGCCG
ACATGGATCAACTGGAACGCCTGATCCAGTTGAACATCACCGCCGTCACGCGTCTGGCGTCG
GCCGCCGCTGCCAGTTTCGCCAAGGCAGGTCGCGGCACGATCATCAACATCGCCTCGGTCGT
GGCGCTGTTCCCCGAGCGTTTCAATGCGACCTACACCGCCAGCAAGGCCTATGTGTTGAGTC
TGACCCAATCGTTGAACGCGGAGCTCGAAGGCTCCGGCGTGCAGATCCAGGCCGTGCTGCCG
GGCGTGACCCGCACTGAAATCTGGGAGCGTTCGGGGATCGACGCCAGTGGCATTCCGGCGGA
AATGGTCATGGACGCCGGGGAGATGGTGGATGCCGCCCTGGCCGGTCTGGATCAGGGCGAAC
TGGTCACCATTCCATCGCTGCCCGATGCCGGCGAATGGCAGTCGTTTGTGGCGGCGCGCCAT
GTCATGGCGCCGAACCTTTCGCGCAGCGCTGCAGCCCAGCGCTACAAATCAGGTCATTGA.

Production of Enzyme A

The synthetic DNA (SEQ ID No: 2) and pET28a were digested with *Nde*I and *Xho*I restriction endonucleases, purified, and ligated together employing T4 DNA ligase. The resultant recombinant molecule was subsequently transformed into E. coli strain TOP10 and the desired expression construct verified by Sanger DNA sequencing. After transforming sequence verified plasmid DNA into the E. coli protein expression host BL21(DE3), a single colony was used to inoculate 10 mL of LBP growth medium (10 g/L plant peptone, 10 g/L NaCl and 5 g/L yeast extract, supplemented with 35 μg/mL kanamycin), with culturing performed at 37 °C for 16 h with shaking at 200 rpm. This starter culture was then used to inoculate 1 L of TB growth medium (12 g/L plant peptone, 12 g/L glycerol, 9.96 g/L sodium phosphate dibasic, 20.4 g/L sodium phosphate monobasic, supplemented with 35 μg/mL kanamycin) in a 2 L baffled glass Erlenmeyer flask, which was incubated at 28 °C for 16 h to effect expression of Enzyme A. The culture was then transferred into 2 x 500 mL centrifuge pots (Nalgene®) and centrifuged at 6000 g / 4 °C in a SLA3000 rotor using a Sorvall RC-5C centrifuge. The bacterial pellets were then resuspended within their pots with 5 x pellet weight of 50 mM sodium phosphate buffer, pH 7.5. Subsequent to thorough re-suspension, the bacterial suspension was transferred into a 100 mL glass beaker for cell disruption, that was achieved using an MSE Soniprep 150 sonicator, configured with a 9.5mm sonication probe at 0 °C, for a total of 8 min (4 x 2 min intervals at 16 microns, with 4 min incubation time between cycles). The disrupted cell suspension was then transferred into a 50 mL (Nalgene®) centrifuge pot and centrifuged at 13000 x g in a Fiberlite™ F13-1 X60cy rotor for 45 min. Following centrifugation, the supernatant (approx. 40 mL), was carefully transferred into a 100 mL plastic container and placed at -20 °C for 16 h. The frozen Enzyme A solution was then subjected to lyophilisation by freeze drying (Edwards SuperModulo) over 2 days, maintaining a pressure below 1 mbar throughout. The dried enzyme cake was pulverised thoroughly by crushing with a steel spatula into a free-flowing homogenous powder and stored at -20 °C between use.

Preparation of Tert-butyl trans-2-hydroxy-6-azaspiro[3.4]octane-6-carboxylate

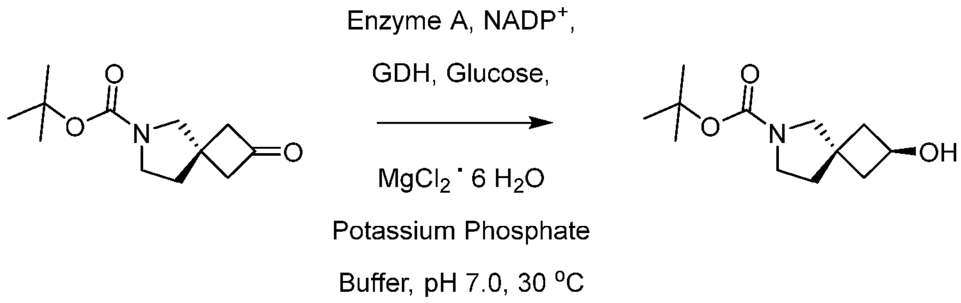


1 mg of Enzyme A was placed into a 2 mL reaction tube. Into the tube was added 0.5 mL of a reaction mix consisting of 5 mM nicotinamide adenine dinucleotide phosphate ($NADP^+$), 2 mM magnesium chloride hexahydrate, 178 mM glucose anhydrous, 1 g/L glucose dehydrogenase (CDX-901, Codexis Inc.), 5% (v/v) dimethyl sulfoxide (DMSO) and 20 g/L tert-butyl 2-oxo-6-azaspiro[3.4]octane-6-carboxylate in 100 mM potassium phosphate buffer. The reaction tube was shaken at 1000 rpm and 30°C for 24 h. The reaction was analyzed using the chiral HPLC method showing a conversion in a range of 98.7% - 100% and a diastereomeric excess of 97%de for tert-butyl trans-2-hydroxy-6-azaspiro [3.4]octane-6-carboxylate.

<u>Synthetic Methods</u>

The compounds of Examples 1 to 4 have been prepared according to the below methods.

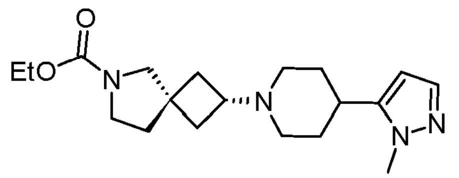

Example 1

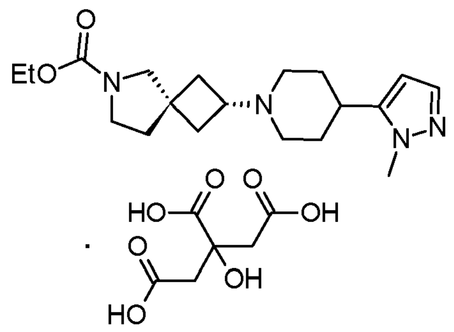

Example 2

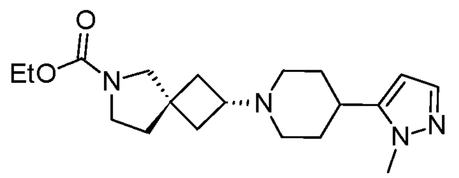

·HCl

Example 3

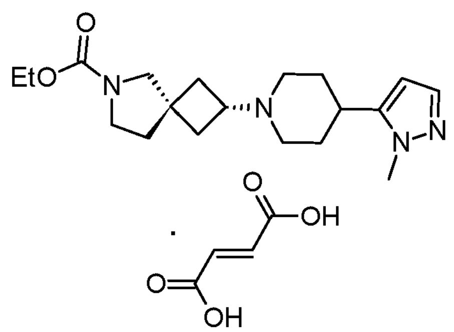

Example 4

<u>Synthetic Example 1: Synthesis of ethyl cis-2-[4-(1-methyl-1H-pyrazol-5-yl)piperidin-1-yl]-6-azaspiro[3.4]octane-6-carboxylate citrate monohydrate</u>

Ethyl cis-2-[4-(1-methyl-1H-pyrazol-5-yl)piperidin-1-yl]-6-azaspiro[3.4]octane-6-carboxylate citrate monohydrate may be prepared according to the following scheme. The process described was used to prepare approximately 5 kg (free base equivalent) of ethyl cis-2-[4-(1-methyl-1H-pyrazol-5-yl)piperidin-1-yl]-6-azaspiro[3.4]octane-6-carboxylate citrate monohydrate.

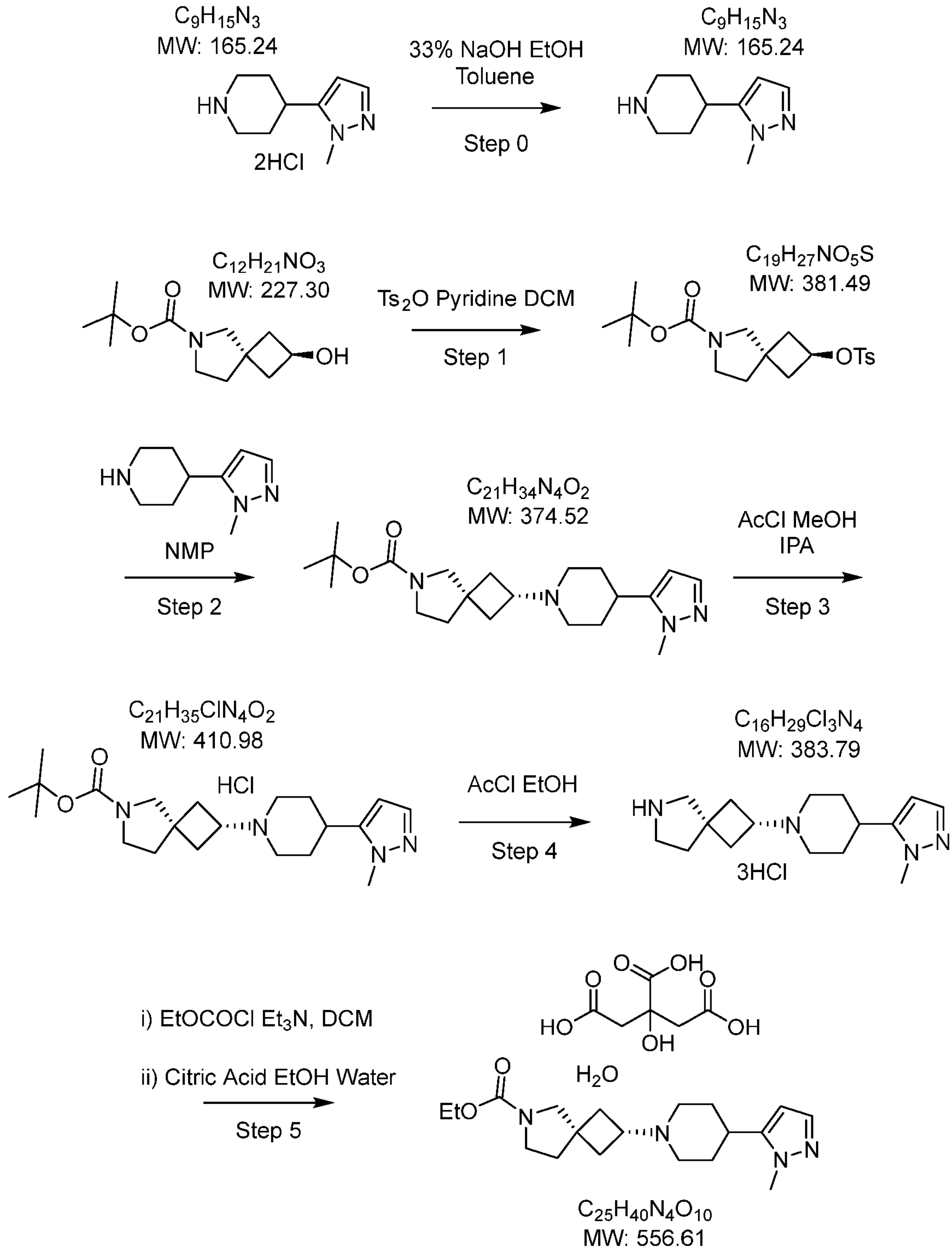


Process Description:

Step 0

1. Charge EtOH (7 vol) to reactor.
2. Charge Pyrazole piperidine dihydrochloride (1eq) to reactor.
3. Adjust temperature to 10 to 15 °C.
4. Charge 33% aqueous NaOH (1 vol) to reactor maintaining the temperature <30°C.

5. Agitate contents at 20 to 25°C for 2 to 3 hours.
6. Filter contents of reactor.
7. Charge EtOH (2 vol) to reactor and use to rinse cake.
8. Combined filtrates re-charged to reactor and concentrated in vacuo at 40 to 45°C to approx. 5 volumes.
9. Charge Toluene (5 vol) to reactor and reduce in vacuo at 40 to 45°C to approx. 4 to 5 volumes.
10. Charge Toluene (5 vol) to reactor and reduce in vacuo at 40 to 45°C to approx. 4 to 5 volumes.
11. Charge Toluene (5 vol) to reactor and reduce in vacuo at 40 to 45°C to approx. 4 to 5 volumes.
12. Charge Toluene (5 vol) to reactor.
13. Filter contents of reactor to clean receiver flask.
14. Charge Toluene (2 vol) to reactor and use to rinse cake.
15. Re-charge combined filtrates to clean reactor and reduce in vacuo at 40 to 45°C to a minimum volume.

Step 1

1. Charge DCM (10 vol) to reactor.
2. Charge Spiro Alcohol (1eq) to reactor.
3. Charge Pyridine (3 eq) to reactor.
4. Adjust internal temperature to 20 to 25°C.
5. Charge Tosyl anhydride (1.5 eq) to reactor maintaining the temperature <35°C. Target a temperature of approx. 30°C during addition.
6. Agitate contents at 20 to 25°C for at least 2 hours
8. Quench reaction with 10% Citric acid solution (5 vol) ensuring temperature does not exceed SOX [moderate exotherm],
9. Agitate for 15 minutes and allow to settle for 15 mins.
10. Discharge lower organic layer to hold container [Contains product].
11. Discharge upper aqueous layer to hold container.
12. Re-charge organic layer from point 10 to reactor.
13. Charge 10% Citric acid solution (5 vol) to reactor.
14. Agitate for 15 minutes and allow to settle for 15 mins.
15. Discharge lower organic layer to hold container [Contains product].
16. Discharge upper aqueous layer to hold container.
17. Re-charge organic layer from point 15 to reactor.
18. Charge saturated $NaHCO_3$ solution (5 vol) to reactor. [Caution: $CO_2$ evolution].
19. Agitate for 15 minutes and allow to settle for 15 mins.
20. Discharge lower organic layer to hold container [Contains product].
21. Discharge upper aqueous layer to hold container.
22. Re-charge organic layer from point 20 to reactor.
23. Charge saturated $NaHCO_3$ solution (5-vol) to reactor. [Caution: $CO_2$ evolution].
24. Agitate for 15 minutes and allow to settle for 15 mins.
25. Discharge lower organic layer to hold container [Contains product].
26. Discharge upper aqueous layer to hold container.
27. Re-charge organic portion [orange/brown solution] from point 25 to reactor and reduce to approx. 5 vol in vacuo at 35 to 40°C.
28. Charge Cyclohexane (5 vol) to reactor and reduce to approx. 5 vol in vacuo at 35 to 40°C.
29. Charge Cyclohexane (5 vol) to reactor and reduce to approx. 5 vol in vacuo at 35 to 40°C.
30. Charge Cyclohexane (5 vol) to reactor and reduce to approx. 5 vol in vacuo at 35 to 40°C.
31. Chemist Check 1: DCM content by NMR. Target: <0.2% w/w remaining wrt Cyclohexane peak. If fail, repeat Cyclohexane strip.
32. Charge Cyclohexane (5 vol) to reactor and adjust contents of reactor to 10 to 15°C at 6°C/hr and hold for 1 to 2 hours.
33. Chemist Check 2: Crystallization check. Target: Product recrystallized.
34. Filter contents of reactor.
35. Charge Cyclohexane (2 vol) to reactor and use to rinse cake.
36. Charge Cyclohexane (2 vol) to reactor and use to rinse cake.
37. Transfer solid to oven and dry at 35 to 40°C under vacuum with a nitrogen bleed for a minimum of 12 hours.

Step 2

1. Chemist check 1: Strip weight assay of piperidine compound Toluene solution.
2. Charge Toluene solution of piperidine compound (4 eq based result in point 1) to reactor.
3. Concentrate under vacuum at 40 to 45°C to approx. 4 to 5 volumes.
4. Charge NMP (2.5 vol) to reactor.
5. Concentrate under vacuum at 40 to 45°C to approx. 5 to 7 volumes
6. Charge Spiro alcohol tosylate (1 eq) to reactor.
7. Adjust contents to 97 to 102°C and agitate under vacuum of 180-200mbar under distillation conditions for at least 36 hours, [orange solution]
8. IPC 1: Reaction completion by HPLC. Target <3% area remaining Spiro alcohol tosylate.
9. Adjust contents to 20 to 25°C.
10. Charge EtOAc (4 vol) to reactor
11. Charge Water (4 vol) to reactor maintaining the temperature <25°C. [small exotherm]
12. Agitate for 15 minutes and allow to settle for 15 mins.
13. Discharge lower aqueous layer to hold container.
14. Discharge upper organic layer to hold container. [Contains product]
15. Re-charge aqueous layer from point 13 to reactor.
16. Charge EtOAc (4 vol) to reactor
17. Agitate for 15 minutes and allow to settle for 15 mins.
18. Discharge lower aqueous layer to hold container.
19. Discharge upper organic layer to hold container. [Contains product]
20. Re-charge aqueous layer from point 18 to reactor.
21. Charge EtOAc (4 vol) to reactor
22. Agitate for 15 minutes and allow to settle for 15 mins.
23. Discharge lower aqueous layer to hold container.
24. Discharge upper organic layer to hold container. [Contains product]
25. Re-charge aqueous layer from point 23 to reactor.
26. Charge EtOAc (4 vol) to reactor
27. Agitate for 15 minutes and allow to settle for 15 mins.
28. Discharge lower aqueous layer to hold container.
29. Re-charge organic layers from points 14, 19 and 24 to reactor.
30. Charge water (5 vol) to reactor.
31. Agitate for 15 minutes and allow to settle for 15 mins.
32. Discharge lower aqueous layer to hold container.
33. Charge water (5 vol) to reactor.
34. Agitate for 15 minutes and allow to settle for 15 mins.
35. Discharge lower aqueous layer to hold container.
36. Charge 2M NaCl (5 vol) to reactor.
37. Agitate for 15 minutes and allow to settle for 15 mins.
38. Discharge lower aqueous layer to hold container.
39. Charge 2M NaCl (5 vol) to reactor.
40. Agitate for 15 minutes and allow to settle for 15 mins.
41. Discharge lower aqueous layer to hold container.
42. Charge 5% Citric acid solution (5 vol) to reactor.
43. Agitate for 15 minutes and allow to settle for 15 mins.
44. Discharge lower aqueous layer to hold container. [Contains product]
45. Charge 5% Citric acid solution (5 vol) to reactor.
46. Agitate for 15 minutes and allow to settle for 15 mins.
47. Discharge lower aqueous layer to hold container. [Contains product]
48. Discharge upper organic layer to hold container.
49. Re-charge aqueous layers from points 44 and 47 to reactor.
50. Carefully charge Sodium carbonate (1 wt) to reactor [slight exotherm- easily controlled through addition rate] maintaining the temperature <30°C until pH >8. [Caution: $CO_2$ gas evolution, effervescence]
51. Chemist check 2: Confirm basic pH. Target: pH >8. Add further sodium carbonate if fail
52. Charge EtOAc (5 vol) to reactor

53. Agitate for 15 minutes and allow to settle for 15 mins.
54. Discharge lower aqueous layer to hold container.
55. Discharge organic layer to hold container. [Contains product]
56. Re-charge aqueous layer from point 54 to reactor.
57. Charge EtOAc (5 vol) to reactor.
58. Agitate for 15 minutes and allow to settle for 15 mins.
59. Discharge lower aqueous layer to hold container.
60. Discharge organic layer to hold container. [Contains product]
61. Re-charge aqueous layer from point 59 to reactor.
62. Charge EtOAc (5 vol) to reactor.
63. Agitate for 15 minutes and allow to settle for 15 mins.
64. Discharge lower aqueous layer to hold container.
65. Re-charge organic portions from points 55 and 60 to reactor.
66. Concentrate under vacuum at 35 to 40°C to approx. 4 to 5 volumes.
67. Charge Cyclohexane (5 vol) to reactor and reduce under vacuum at 35 to 40°C to approx. 4 to 5 volumes.
68. Charge Cyclohexane (5 vol) to reactor and reduce under vacuum at 35 to 40°C to approx. 4 to 5 volumes.
69. Charge Cyclohexane (5 vol) to reactor and reduce under vacuum at 35 to 40°C to approx. 4 to 5 volumes.
70. Chemist check 3: EtOAc content by NMR. Target: ^1% w/w. Repeat Cyclohexane strip if fail.
71. Charge Cyclohexane (5 vol) to reactor and adjust contents of reactor to 35 to 40°C before cooling to 10 to 15°C at 6°C/hr and hold for 1 to 2 hours.
72. Chemist check 4: Crystallization check. Target: Suspension observed.
73. Filter contents of reactor.
74. Charge Cyclohexane (2 vol) to reactor and use to rinse cake.
75. Charge Cyclohexane (2 vol) to reactor and use to rinse cake.
76. Transfer solid to oven and dry at 35 to 40°C under vacuum with a nitrogen bleed for a minimum of 12 hours.

(Step 3 was not performed due to process improvements)

Step 4

1. Charge Ethanol (5 vol) to reactor.
2. Adjust contents of reactor to -5 to 0°C.
3. Charge Acetyl Chloride (6 eq) to reactor maintaining the temperature <15°C [colourless solution]
4. Charge Step 2 Boc-product (1 eq) to reactor [eventually forms orange solution then a beige suspension].
5. Adjust contents of reactor to 20 to 25°C and stir for at least 18 hours.
6. IPC 1: Reaction completion by HPLC. Target <0.5% remaining SM.
7. Charge TBME (10 vol) to reactor over at least 15 minutes [Agitation may need to be increased temporarily during addition].
8. Agitate contents of reactor for 1 hour ± 15 mins.
9. Filter suspension. Solid is hygroscopic- filter under nitrogen flow and do not pull dry.
10. Charge TBME (5 vol) to reactor and use to wash filter cake. Filter under nitrogen flow and do not pull dry.
11. Charge TBME (5 vol) to reactor and use to wash filter cake. Filter under nitrogen flow and do not pull dry. Transfer to oven immediately.
12. Transfer to oven and dry at 35 to 40°C under vacuum with a nitrogen bleed for a minimum of 12 hours.

Step 5

1. Charge Dichloromethane (8 vol) to reactor.
2. Charge Triethylamine (4.5 eq) to reactor.
3. Adjust contents of reactor to -5 to 0°C.
4. Charge Step 4 product (1 eq) to reactor portion wise maintaining the temperature <10°C. [beige suspension]
5. Charge Ethyl chloroformate (1.5 eq) to reactor maintaining the temperature <10°C [strong exotherm, quickly dissipates, beige suspension].
6. Adjust contents to 20-25°C and agitate for at least 2 hours.

7. IPC 1: Reaction completion by HPLC. Target <0.5% remaining SM. Stir for a further 2h if fail.
8. Slowly charge water (3 vol) maintaining the temperature <25°C. [forms transparent biphasic layers, minimal exotherm]
9. Agitate for 15 minutes and allow to settle for 15 mins.
10. Discharge lower organic layer to hold container [contains product]
11. Discharge upper aqueous layer to hold container.
12. Re-charge organic layer from point 10 to reactor.
13. Charge 2M NaCl (4 vol) to reactor
14. Agitate for 15 minutes and allow to settle for 15 mins.
15. Discharge lower organic layer to hold container [contains product]
16. Discharge upper aqueous layer to hold container.
17. Charge organic layer from point 15 to a clean reactor and concentrate in vacuo at 40 to 45°C to approx. 4 volumes.
18. Charge EtOH (5 vol) to reactor and concentrate in vacuo at 40 to 45°C to approx. 4 volumes.
19. Charge EtOH (5 vol) to reactor and concentrate in vacuo at 40 to 45°C to approx. 4 volumes.
20. Chemist check 1: DCM content by NMR. Target <0.2% w/w DCM wrt Ethanol. Repeat Ethanol strip if fail.
21. Chemist check 2: Strip weight assay of ethanol solution used to determine amount of product present and therefore the citric acid, Ethanol, water and Citrate Seed charges in the next step.
22. Adjust reaction to 8 volumes (based on chemist check 2 result) of 5% Water in Ethanol.
23. Charge Citric acid (1 eq based on chemist check 2 result) to reactor.
24. Adjust contents to 20 to 25°C and agitate for 30 minutes to 1 hour [beige suspension will form]
25. Adjust contents to 55 to 60°C and agitate for at least 1 hour [dissolution usually at 50 to 55°C]
26. Chemist check 3: Dissolution. Target: Solution formed [orange solution]. If fail, add incremental 0.25 volume portions of 5% Water in Ethanol and hold for 30 minutes until dissolution is achieved.
27. Polish filter solution into a clean reactor using heated hoses [Warning: precipitation at 40°C]
28. Adjust contents to 55 to 60°C and agitate for at least 1 hour.
29. Adjust contents to 40 to 45°C over 3 hours, seed with 0.5% w/w product Citrate salt.
30. Agitate at 40 to 45°C for 2 hours.
31. Adjust contents to 0 to 5°C over at least 12 hours [beige suspension].
32. Chemist check 4: Precipitation. Target: Suspension formed.
33. IRC 2: Form by XRD. Target: Form 2. If fail, seed with a further portion of 0.5%. w/w Citrate Form 2, stir for at least 18h and resample for IPC 2.
34. Filter suspension.
35. Charge Ethanol (2 vol) to reactor.
36. Adjust temperature to 0 to 5°C and use to wash cake.
37. Charge Ethanol (2 vol) to reactor.
38. Adjust temperature to 0 to 5°C and use to wash cake.
39. Charge TBME (5 vol) to reactor and use to wash cake.
40. Charge TBME (5 vol) to reactor and use to wash cake.
41. IPC 3: Wetcake purity by HPLC. Target: >97.0% area. RRT 0.56 < 0.29%, RRT 0.89 < 0.56%, RRT 0.92 < 0.37%, RRT 0.95 < 3.6%, RRT 1.23 < 0.20%. All other individual impurities < 0.15%
42. Transfer solid to a vacuum oven and dry at 20 to 25°C for at least 8 hours up to a maximum to 114h.

Synthetic Example 2: Alterative Synthesis of ethyl cis-2-[4-(1-methyl-1H-pyrazol-5-yl)piperidin-1-yl]-6-azaspiro[3.4]octane-6-carboxylate citrate monohydrate

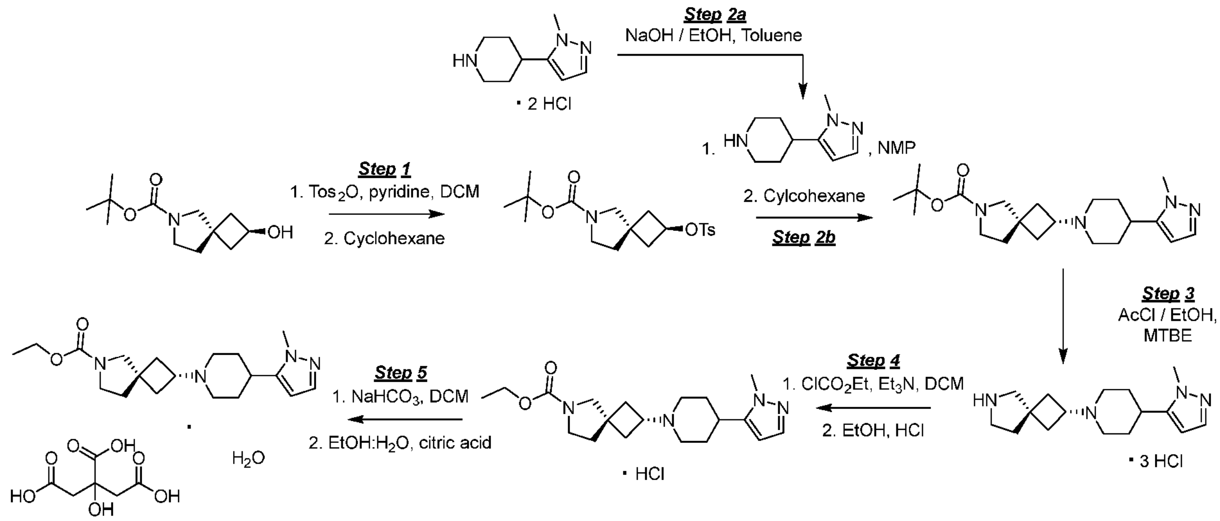


*Step 1. Tert-butyl trans-2-(tosyloxy)-6-azaspiro[3.4]octane-6-carboxylate*

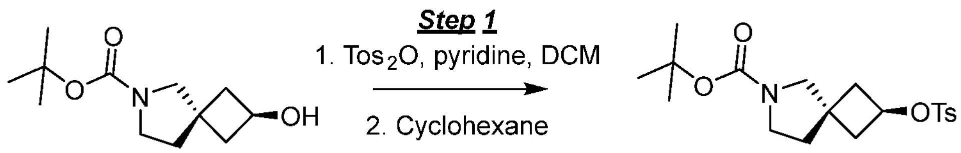


Tert-butyl trans-2-hydroxy-6-azaspiro[3.4]octane-6-carboxylate is dissolved in dichloromethane. Pyridine is added and the tosyl anhydride is added in portions. The reaction is stirred until complete and the organic and aqueous layers allowed to settle. The organic phase (containing tert-butyl trans-2-(tosyloxy)-6-azaspiro[3.4]octane-6-carboxylate) is sequentially washed with aqueous citric acid and aqueous sodium bicarbonate. The solution containing tert-butyl trans-2-(tosyloxy)-6-azaspiro[3.4]octane-6-carboxylate is solvent swapped to cyclohexane. The organic layer is then cooled to precipitate the product and the crystallized material is isolated on a filter. The cake is then washed with cyclohexane and dried.

*Alternative route to tert-butyl trans-2-(tosyloxy)-6-azaspiro[3.4]octane-6-carboxylate*

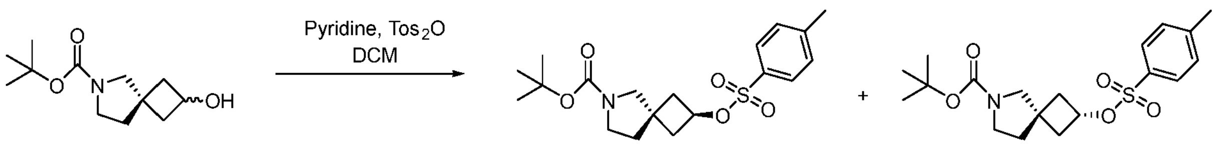


*trans* *cis*

tert-butyl 2-hydroxy-6-azaspiro[3.4]octane-6-carboxylate is dissovled in DCM (10 V), and pyridine (3 eqv) is added. The solution is stirred at RT for 15 min. Tosyl anhydride (1.5 eqv) is added and the reaction mixture stirred at RT for 2 h. A further portion of tosyl anhydride (0.12 eqv) is added and the reaction mixture stirred at RT for 18 h. The reaction is quenced with 10% aqueous citric acid (5 V) and the organic and aqueous phases allowed to settle. The organic phase is separated and washed with 10% aqueous citric acid (5 V) and saturated solution of $NaHCO_3$ (2 x 5 V). The organics were removed by distillation under vacuum to afford a crude product.

The crude product purified by flash column chromatography (silica gel) 0-50% EtOAc/hexane, monitoring the elution at wavelength 254 nm. The flow rate was reduced four-fold once peaks were visible on the chromatogram and two fractions were collected (top and bottom, named by the relative position of their corresponding spots on thin layer chromatography) corresponding to the cis and trans products. 1H NMR spectra for the products are shown in Figure 34

(trans-tosylate, "top" fraction), and Figure 35 (cis-tosylate, "bottom" fraction).

The top fraction was assigned as the desired trans isomer, and the bottom fraction was assigned as the undesired cis isomer by $^{1}H$-$^{1}H$ Nuclear Overhauser Effect Spectroscopy (NOESY). NOESY spectra and assignments are shown in Figure 36 (trans-tosylate, "top" fraction), and Figure 37 (cis-tosylate, "bottom" fraction).

*Step 2. Tert-butyl cis-2-(4-(1-methyl-1H-pyrazol-5-yl)piperidin-1-yl)-6-azaspiro[3.4]octane-6-carboxylate*

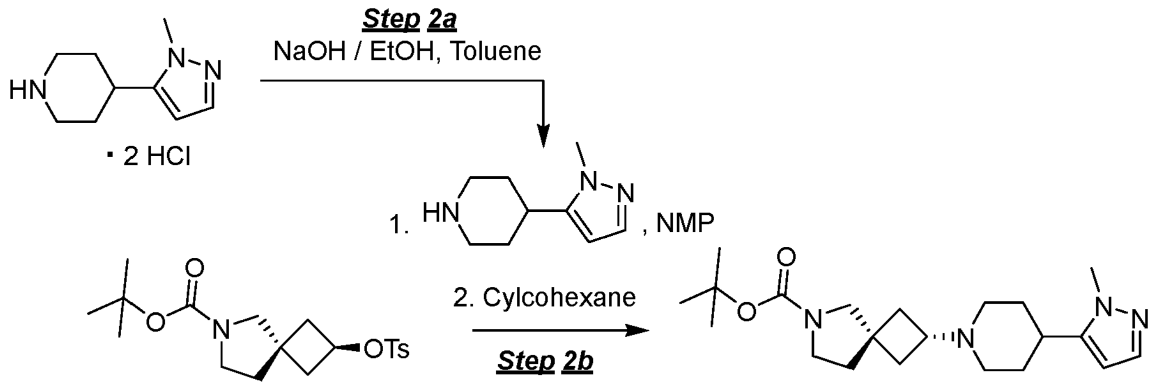


4-(1-methyl-1H-pyrazol-5-yl)piperidine dihydrochloride is suspended in ethanol before aqueous sodium hydroxide is added. The suspension is stirred to break the HCl salt before the mixture is filtered. The cake is washed with ethanol and the product solution displaced into toluene. The toluene layer (containing 4-(1-methyl-1H-pyrazol-5-yl)piperidine free base) is then filtered and concentrated. To the solution of 4-(1-methyl-1H-pyrazol-5-yl)piperidine free base in toluene is added NMP and tert-butyl (2s,4r)-2-(tosyloxy)-6-azaspiro[3.4]octane-6-carboxylate. The solution is heated, and residual toluene is distilled *in vacuo.* The reaction continues to be heated until reaction is deemed complete. The product is then diluted with ethyl acetate and the organics (containing tert-butyl cis-2-(4-(1-methyl-1H-pyrazol-5-yl)piperidin-1-yl)-6-azaspiro[3.4]octane-6-carboxylate) are washed with water and brine. The product is extracted from the organic layer into a solution of citric acid. The pH of the aqueous layer (containing product) is adjusted, and the product extracted into ethyl acetate. The product is displaced into cyclohexane, cooled to induce crystallization, and filtered. The cake is washed with cyclohexane and dried to yield tert-butyl cis-2-(4-(1-methyl-1H-pyrazol-5-yl)piperidin-1-yl)-6-azaspiro[3.4]octane-6-carboxylate.

*Step 3. cis-2-(4-(1-Methyl-1H-pyrazol-5-yl)piperidin-1-yl)-6-azaspiro[3.4]octane trihydrochloride*

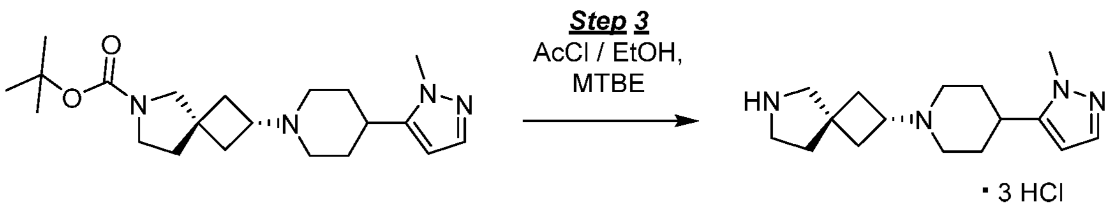


Acetyl chloride is charged to cold ethanol and stirred to generate HCl *in-situ.* To this solution is added tert-butyl cis-2-(4-(1-methyl-1H-pyrazol-5-yl)piperidin-1-yl)-6-azaspiro[3.4]octane-6-carboxylate. The resulting suspension is stirred until deprotection is achieved. MTBE is added to the resulting suspension and the solids stirred before being collected by filtration. The cake is washed with MTBE and dried *in vacuo* to yield cis-2-(4-(1-methyl-1H-pyrazol-5-yl)piperidin-1-yl)-6-azaspiro[3.4]octane as the trihydrochloride salt.

*Step 4. Ethyl cis-2-[4-(1-methyl-1H-pyrazol-5-yl)piperidin-1-yl]-6-azaspiro[3.4]octane-6-carboxylate hydrochloride*

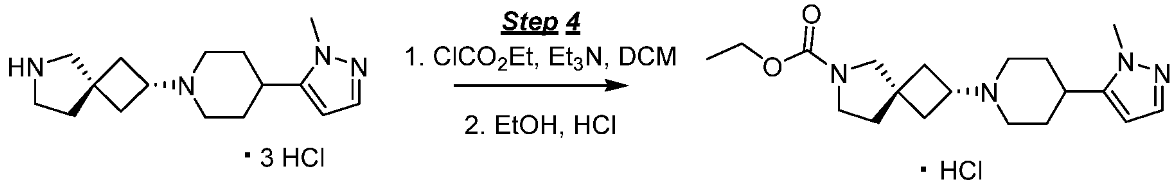


Triethylamine is dissolved in dichloromethane and cooled. To the solution is added cis-2-(4-(1-methyl-1H-pyrazol-5-yl)piperidin-1-yl)-6-azaspiro[3.4]octane trihydrochloride in portions. The mixture is stirred and ethyl chloroformate is added. The mixture is heated and stirred until completion of the reaction. The organic layer (containing product) is washed sequentially with water and a solution of sodium chloride. The product is displaced into ethanol before anhydrous HCl is charged. The resulting suspension is heated until dissolution and then cooled to initiate crystallization. The solids are stirred and then isolated *via* filtration, washed with MTBE, and then dried.

*Step 5. Ethyl cis-2-[4-(1-methyl-1H-pyrazol-5-yl)piperidin-1-yl]-6-azaspiro[3.47]octane-6-carboxylate citrate monohydrate*

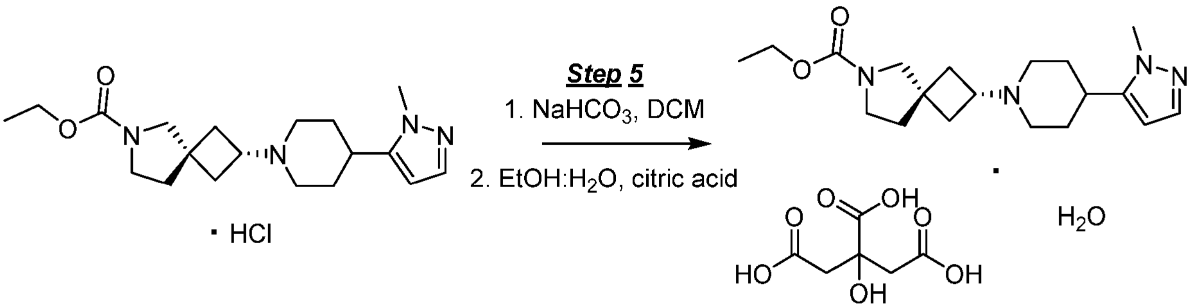


A suspension of ethyl cis-2-[4-(1-methyl-1H-pyrazol-5-yl)piperidin-1-yl]-6-azaspiro[3.4]octane-6-carboxylate hydrochloride is formed in dichloromethane. The mixture is stirred, and aqueous sodium bicarbonate is added. The mixture is stirred until all solids are dissolved. The organic layer (containing product) is washed sequentially with water and a solution of sodium chloride. The product is displaced into ethanol before citric acid and water are charged. The resulting suspension is heated until dissolution and then cooled to initiate crystallization. The solids are stirred and then isolated *via* filtration, washed with MTBE, and then dried to yield ethyl cis-2-[4-(1-methyl-1H-pyrazol-5-yl)piperidin-1-yl]-6-azaspiro[3.4]octane-6-carboxylate citrate monohydrate.

Synthetic Example 3. Further alternative synthesis of ethyl cis-2-[4-(1-methyl-1H-pyrazol-5-yl)piperidin-1-yl]-6-azaspiro[3.4]octane-6-carboxylate citrate monohydrate

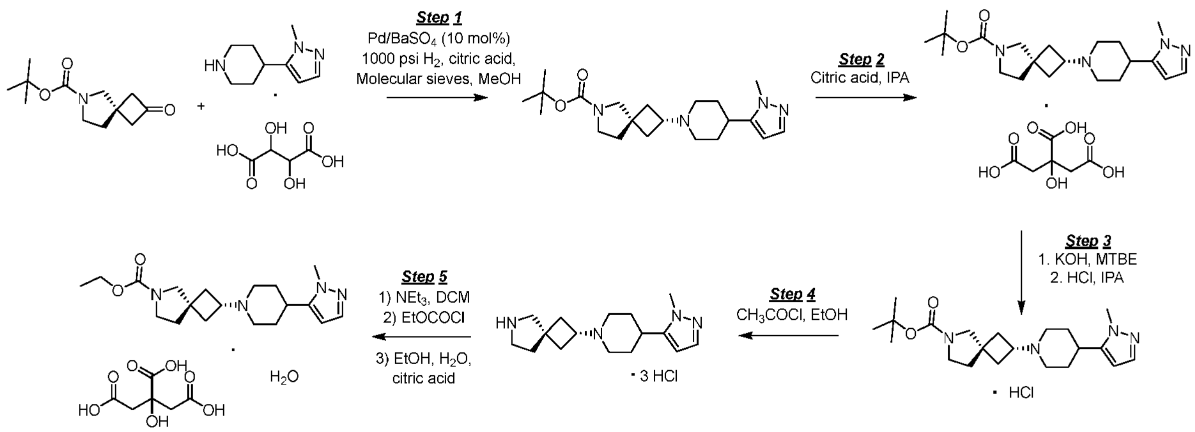

*Step 1: Tert-butyl cis-2-(4-(1-methyl-1H-pyrazol-5-yl)piperidin-1-yl)-6-azaspiro[3.4]octane-6-carboxylate*

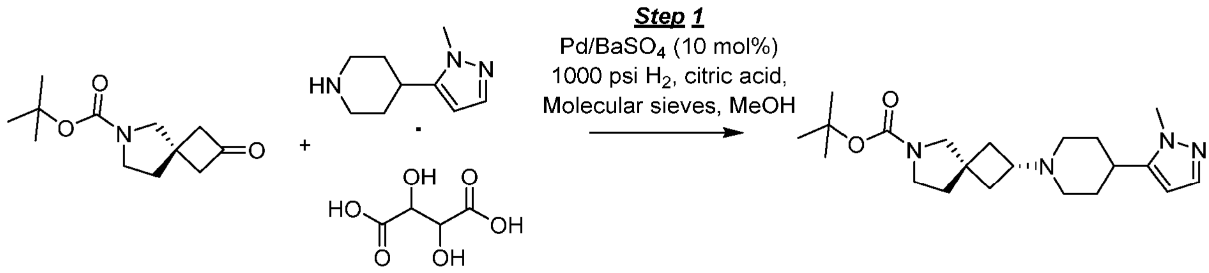


Tert-butyl 2-oxo-6-azaspiro[3.4]octane-6-carboxylate (75.0 g, 328 mmol), 4-(1-methyl-1H-pyrazol-5-yl)piperidine tartrate (132.1 g, 377.0 mmol), molecular sieves (124.0 g, 1041 mmol) (3 Angstrom), citric acid (130.0 g, 676.6 mmol), and methanol (686.5 mL) were charged sequentially to a 1500 mL bomb. 5.0 wt.% Pd on $BaSO_4$ (5:95, Palladium:Barium Sulfate, 69.74 g, 32.77 mmol) (Aldrich, Reduced) was added, and hydrogen was applied to 994 psi. The reaction mixture was stirred at 23 °C for 24 h and then the temperature raised to 35 °C for a further 22 h. Then, hydrogen pressure was vented, and catalyst removed by filtration on a 1500 mL "M" frit. The filter cake was washed with MeOH (811.4 g).

The clear yellow filtrate was concentrated by rotary evaporation to a solution (538.9 g). Water (251.1 g) was added and the pH adjusted to 13.64 with potassium hydroxide (256.8 g, 2060 mmol) while maintaining the temperature below 35 °C with an ice bath. The resultant milky mixture was concentrated on a rotary evaporator (813 g) and 2-methoxy-2-methylpropane (600 g) was added. The phases were separated, and the organics washed with water (250 g). The aqueous phases were serial back extracted with 2-Methoxy-2-methylpropane (223.0 g).

The organic phases were combined and concentrated to an oil on a rotary evaporator (188.6 g). Isopropyl alcohol (473.7 g) was added and concentrated to a yellow oil from which the title compound (tert-butyl cis-2-(4-(1-methyl-1H-pyrazol-5-yl)piperidin-1-yl)-6-azaspiro[3.4]octane-6-carboxylate) free base precipitated after about 30 min at room temperature in a yield of 65.59 g. HPLC showed 16 area% non-desired trans-isomer, giving 81.4% yield for desired cis-isomer. The title compound was used in the next step without further purification.

During process development a variety of reduction conditions were found to provide modest to reasonable selectivity for the desired cis-isomer and modest to excellent conversion of the starting materials, see, e.g., selected conditions in Table A below.

Table A

| *Solvent* | *Acid* | *Reductant* | *Cis* | *Trans* | *Highest conversion* | *Pressure* |
|---|---|---|---|---|---|---|
| Toluene | None | STAB | 56.7 | 43.3 | 100% | N/A |
| Toluene | $Ti(OiPr)_4$ | STAB | 59.3 | 40.7 | 100% | N/A |
| DCM | Succinic acid | STAB | 54 | 46 | 73% | N/! |
| MeOH | L-tartaric acid | $H_2$ / Pd Black / Mol. Sieve | 85 | 15 | 86.3% | 90-15 psi |
| MeOH | L-tartaric acid | $H_2$ / Pd Black / Mol. Sieve | 83.1 | 16.9 | 96.6 | 500 psi |
| Ethylene glycol | L-tartaric acid | $H_2$ / Pd/C / mol. Sieve | 76.7 | 23.3 | 100% | 90 psi |
| MeOH | L-tartaric acid | $H_2$ / Pd/C / mol. Sieve | 79.7 | 20.3 | 76.4 | 60 psi |
| MeOH | 2,2-dimethyl succinic acid | $H_2$ / Pd/C / mol. Sieve | 72.7 | 27.3 | 93.5 | 50 psi |
| MeOH | acetic acid | $H_2$ / Pd/C / mol. Sieve | 72.7 | 27.3 | 96.0 | 50 psi |
| MeOH | pivalic acid | $H_2$ / Pd/C / mol. Sieve | 71.1 | 28.9 | 94.7 | 50 psi |
| MeOH | L-tartaric acid | $H_2$ / Pd on barium sulfate / mol. Sieve | 83.5 | 16.5 | 56.4 | 60 psi |
| MeOH | L-tartaric acid | $H_2$ / Pd on calcium carbonate (unreduced) / mol. Sieve | 76.4 | 23.6 | 95.3 | 60-30 psi |

(continued)

| Solvent | Acid | Reductant | Cis | Trans | Highest conversion | Pressure |
|---|---|---|---|---|---|---|
| MeOH | L-tartaric acid | $H_2$ / Pearlman's catalyst / mol. Sieve | 75.4 | 24.6 | 82.8 | 90-15 psi |

*Step 2: Tert-butyl cis-2-(4-(1-methyl-1H-pyrazol-5-yl)piperidin-1-yl)-6-azaspiro[3.4]octane-6-carboxylate citrate*

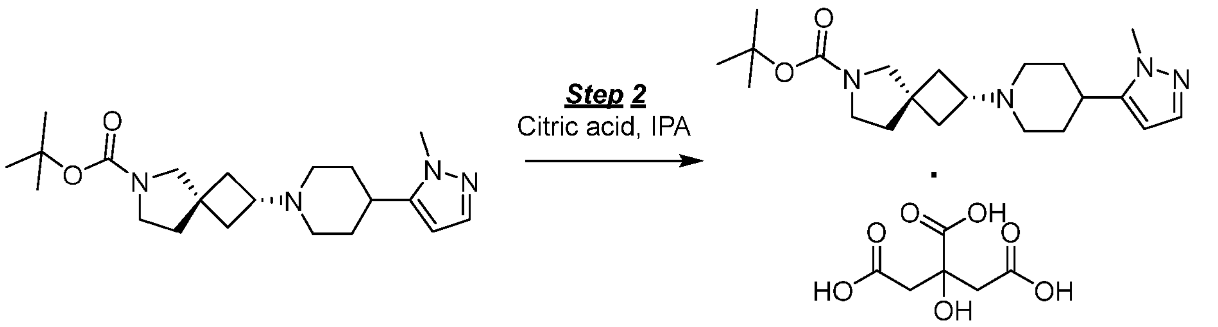


Isopropyl alcohol (1300.2 g, 21636 mmol) was added to the product of step 1 to form a solution having a pH of 9.46 at 22 °C. Freshly prepared 33.3% citric acid (77.14 g, 133.7 mmol) in ethanol was added to give a yellow solution having pH 6.68 at 24.2 °C. After 10 min, the solution was seeded with tert-butyl cis-2-(4-(1-methyl-1H-pyrazol-5-yl)piperidin-1-yl)-6-azaspiro[3.4]octane-6-carboxylate citrate crystals (0.517 g). After a further 1 h, the pH was 6.79 and 33.3% citric acid (51.26 g, 88.84 mmol) in ethanol was added dropwise. After a further 1 h, the pH was 6.45 and 33.3% citric acid (48.0 g, 83.3 mmol) in ethanol was added dropwise. After a further 3 h 15 min, the pH was 6.1 and the crystals were isolated by vacuum filtration on a 3 L "C" frit. The isolated crystals were washed with isopropyl alcohol (229.5 g) and dried overnight under nitrogen pressure to give the title compound (tert-butyl cis-2-(4-(1-methyl-1H-pyrazol-5-yl)piperidin-1-yl)-6-azaspiro[3.4]octane-6-carboxylate citrate) in a yield of 162.55 g (162.03 g corrected for seeds). HPLC analysis showed 7.5 area% trans isomer; NMR showed 4.11 wt% IPA and 1.00 equivalent citric acid.

HPLC of the filtrate showed 68.2: 31.8 trans: cis ratio (4.0% filtrate loss).

LCMS (ES+): found 375.4 [$M+H^+$] (tert-butyl cis-2-(4-(1-methyl-1H-pyrazol-5-yl)piperidin-1-yl)-6-azaspiro[3.4]octane-6-carboxylate); (ES-): found 191.1 [$M-H^-$] (citric acid)

*Step 3: Tert-butyl cis-2-(4-(1-methyl-1H-pyrazol-5-yl)piperidin-1-yl)-6-azaspiro[3.4]octane-6-carboxylate hydrochloride*

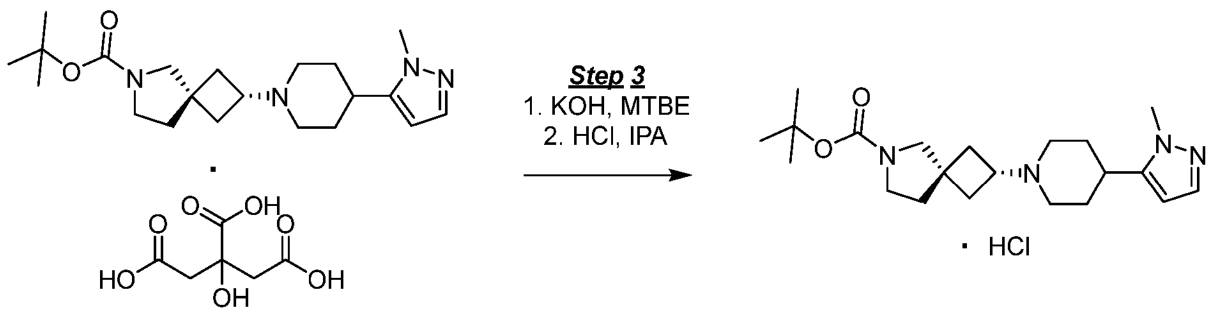


To a 2000 mL round-bottom flask was charged tert-butyl cis-2-[4-(1-methyl-1H-pyrazol-5-yl)piperidin-1-yl]-6-azaspiro[3.4]octane-6-carboxylate citrate (161.34 g, 244.21 mmol; 7.4% trans-isomer), 2-methoxy-2-methylpropane (556.8 g, 6317 mmol), and water (399.4 g, 22170 mmol) to give a biphasic solution. The pH of the resultant solution was adjusted from 3.44 to 13.49 with potassium hydroxide (106.8 g, 856.6 mmol). The phases were separated and the organics washed with water (103.9 g). The aqueous phases were serial back extracted with 2-Methoxy-2-methylpropane (196.0 g). A small amount of black interphasal solids during each phase separation was sent to aqueous. The combined organics

were concentrated to an oil (146.83 g) and isopropyl alcohol (840.5 g) was added. 22.31 wt% Hydrogen chloride (15.20 g, 93.01 mmol; 5.53 M in isopropyl alcohol) was added to the solution and the pH dropped from 10.41 to 6.32. The resultant solution was seeded with tert-butyl cis-2-(4-(1-methyl-1H-pyrazol-5-yl)piperidin-1-yl)-6-azaspiro[3.4]octane-6-carboxylate hydrochloride (0.21 g) and a slurry immediately began to form. After 10 min the pH was 6.45 and 22.31wt% hydrogen chloride (14.6 g, 89.3 mmol) in isopropyl alcohol was added dropwise over 10 minutes. After a further 15 min, the pH was 6.2 and 22.31wt% hydrogen chloride (15.0 g, 91.8 mmol) in isopropyl alcohol was added dropwise over 10 minutes with addition halted in the pH range 1.0-4.0. The mixture was placed in an ice bath for 1 h 15 min to cool to 3.0 °C. Then, the slurry was poured on to a 3000 mL "C" frit and allowed to settle before applying vacuum filtration. The filter cake was washed with isopropyl alcohol (300 mL) and then dried under nitrogen pressure for 2.5 days to provide the title compound (tert-butyl cis-2-(4-(1-methyl-1H-pyrazol-5-yl)piperidin-1-yl)-6-azaspiro[3.4]octane-6-carboxylate hydrochloride) in a yield of 95.55 g (95.34 g corrected for seeds). HPLC analysis showed 97.10 wt% as HCl salt with 0.20 area% as the undesired trans-isomer; NMR showed 0.35 wt% isopropyl alcohol.

HPLC of the filtrate showed 43.4: 56.6 cis: trans (5.52% filtrate loss).

LCMS (ES+): found 375.4 [M+H$^+$]

*Step 4: cis-2-(4-(1-Methyl-1H-pyrazol-5-yl)piperidin-1-yl)-6-azaspiro[3.4]octane trihydrochloride*

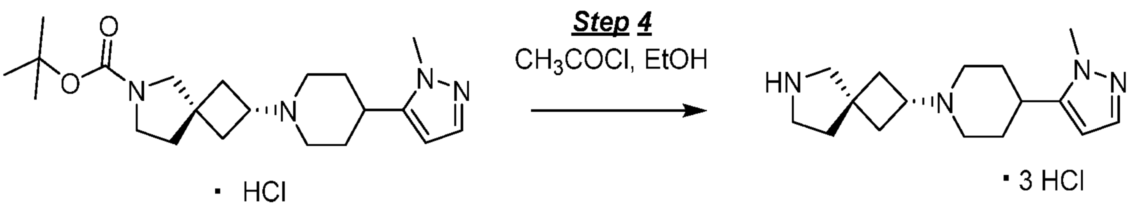


A 2000 mL round-bottom flask was charged with ethanol (340.90 g) and placed in an ice-water bath. Acetyl chloride (92.53 g, 1179 mmol) was added over 15 min, keeping the temperature less than 30 °C (vigorous exotherm during acetyl chloride addition). The ice-water bath was removed, and after 45 min, at a reaction temperature of 20 °C, tert-butyl cis-2-[4-(1-methyl-1H-pyrazol-5-yl)piperidin-1-yl]-6-azaspiro[3.4]octane-6-carboxylate hydrochloride (93.43 g, 220.7 mmol; 0.20 area% trans) was added, and rinsed in with ethanol (100.54 g). The mixture was stirred for 22 h at room temperature (22.9 °C). The resultant slurry of white crystals showed complete conversion of the starting material (<0.1% starting material by HPLC). 2-Methoxy-2-methylpropane (812 mL) was added in portions over 35 min. The resulting mixture was stirred for 15 min. The slurry was poured onto a 600 mL "C" frit and filtered to provide large white crystals. The filter cake was washed with 2-methoxy-2-methylpropane (84.46 g) then dried under nitrogen pressure to give the title compound (cis-2-(4-(1-methyl-1H-pyrazol-5-yl)piperidin-1-yl)-6-azaspiro[3.4]octane trihydrochloride) in a yield of 90.17 g (assigned purity 93.96% assuming 100% yield). HPLC showed 99.74 area% purity; NMR showed 0.84 wt% ethanol as only residual solvent).

LCMS (ES+): found 275.4 [M+H$^+$]

*Step 5. Ethyl cis-2-{4-(1-methyl-1H-pyrazol-5-yl)piperidin-1-yl]-6-azaspiro[3.4]octane-6-carboxylate citrate monohydrate*

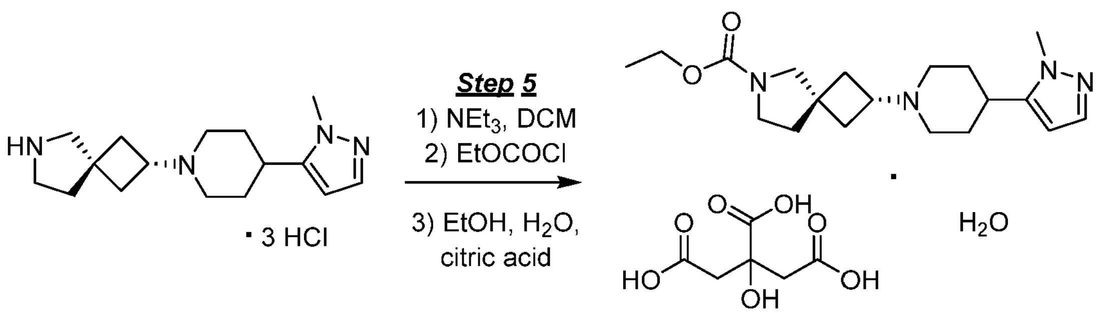


A 2000 mL round-bottom flask was charged with cis-2-[4-(1-methyl-1H-pyrazol-5-yl)piperidin-1-yl]-6-azaspiro[3.4]octane trihydrochloride (88.3 g, 216 mmol) and methylene chloride (858 g). The resultant slurry was cooled to 11 °C

with an ice bath. Triethylamine (169 mL, 1210 mmol) was added over 6 minutes while keeping the temperature less than 21 °C. With the reaction mixture at 16 °C, ethyl chloroformate (28.80 g, 265.4 mmol) was added over 7 minutes while keeping the temperature less than 28 °C. After stirring for 10 min, HPLC analysis showed that reaction was complete. Water (150.0 g) was added, and the mixture stirred at 17 °C for 15 minutes, then the phases were separated. To the organic phase was added water (150 g) and the pH adjusted from 8.33 to 12.2 with sodium hydroxide (17.7 g). The phases were separated and the organics washed with water (150 g). The three aqueous fractions were serial back extracted with methylene chloride (250 mL); note, sodium hydroxide (4.50 g, 56.2 mmol) was added to the sodium hydroxide wash fraction to adjust pH from 9.6 to 12.81. The combined organic fractions were concentrated to an oil (113.3 g) and ethanol (157.5 g) was added before concentrating again to an oil (98.3 g). Ethanol (884.3 g) was added again, followed by water (30.67 g, 1702 mmol) and citric acid (44.90 g, 233.7 mmol). The resultant solution was seeded with crystals of ethyl cis-2-[4-(1-methyl-1H-pyrazol-5-yl) piperidin-1-yl]-6-azaspiro[3.4]octane-6-carboxylate citrate monohydrate (0.35 g) and stirred overnight at room temperature to five a slurry or large crystals. The slurry was placed in an ice-water bath to cool the mixture to 1-2 °C for 2 h 15 min. The crystals were isolated by vacuum filtration on a 600 mL "C" frit then washed with a mixture of ethanol (123.5 g, 2681 mmol) and water (8.2 g, 460 mmol) at 0 °C, followed by 2-methoxy-2-methylpropane (167 g, 1890 mmol) at 0°C. The product was dried overnight under nitrogen pressure to provide the title compound (ethyl cis-2-[4-(1-methyl-1H-pyrazol-5-yl)piperidin-1-yl]-6-azaspiro[3.4]octane-6-carboxylate citrate monohydrate) in a yield of 110.24 g (109.89 g corrected for seeds). HPLC showed 99.7 area% of target (210 nm); Less than 0.05% trans isomer.

HPLC vs. ethyl cis-2-[4-(1-methyl-1H-pyrazol-5-yl)piperidin-1-yl]-6-azaspiro[3.4]octane-6-carboxylate citrate monohydrate standard shows 101.3wt%. Karl Fischer titration shows 3.31 wt% water (theory = 3.24 wt% for monohydrate). NMR shows 0.10 wt% ethanol as only residual solvent.

HPLC shows 8.5% product in filtrate and washes.

NMR spectrum of ethyl cis-2-[4-(1-methyl-1H-pyrazol-5-yl)piperidin-1-yl]-6-azaspiro[3.4]octane-6-carboxylate citrate monohydrate produced by this method is shown in Figures 30 & 31.

LCMS (ES+): found 347.3 [M+H$^+$] (ethyl cis-2-[4-(1-methyl-1H-pyrazol-5-yl)piperidin-1-yl]-6-azaspiro[3.4]octane-6-carboxylate); (ES-): found 191.1 [M-H$^-$] (citric acid)

Reference Synthetic Example 7: ethyl cis-2-[4-(1-methyl-1H-pyrazol-5-yl)piperidin-1-yl]-6-azaspiro[3.4]octane-6-carboxylate hydrochloride (HCl salt)

Acetyl chloride (0.44 mL, 5.98 mmol) was added to EtOH (10.25 mL) at 0 °C and after 10 minutes at this temperature this solution was added to ethyl cis-2-[4-(1-methyl-1H-pyrazol-5-yl)piperidin-1-yl]-6-azaspiro[3.4]octane-6-carboxylate (2.05 g, 5.92 mmol) in EtOH (10.25 mL) at 0 °C. The reaction mixture was stirred at 0 °C overnight before being concentrated to dryness. 2-BuOH (6.15 mL) was added to the residues and the mixture heated until complete dissolution was achieved (110 °C). The reaction mixture was held at this temperature for 10 min, before being allowed to cool to 20-25°C over 3 h. Diethyl ether (8 mL) was added to the thick suspension to aid stirring. After a further 10 min at 20-25°C the suspension was filtered, and pulled dry on the sinter funnel for 2h. This solid was then transferred to a vacuum oven where it was dried at 40°C overnight to yield the HCl salt (1.497 g, 3.91 mmol, 66% yield) as an off white solid.

Reference Synthetic Example 8: ethyl cis-2-[4-(1-methyl-1H-pyrazol-5-yl)piperidin-1-yl]-6-azaspiro[3.4]octane-6-carboxylate fumarate (fumarate salt)

Ethyl cis-2-[4-(1-methyl-1H-pyrazol-5-yl)piperidin-1-yl]-6-azaspiro[3.4]octane-6-carboxylate fumarate (fumarate salt) was prepared by precipitation from ethanol (EtOH). Ethyl cis-2-[4-(1-methyl-1H-pyrazol-5-yl)piperidin-1-yl]-6-azaspiro[3.4]octane-6-carboxylate (240.0 mg) and fumaric acid (80.1 mg, ~1 mol. eq.) were charged to a 4 mL vial. EtOH (2.5 vols) was added and the mixture was stirred and heated to 45 °C for ~2 hours. After which time the temperature was reduced by 5 °C (every ~10 minutes) until ambient temperature was reached. The resulting solids were isolated by vacuum filtration. The solids were washed twice with EtOH (~5 vols) each time. The solids were air dried on the filter for ~1.5 hours before being transferred to a pre weighed vial. The solids were further dried in the vacuum oven at 40 °C until of constant weight. The fumarate salt was generated in ~34 % yield.

## EXAMPLE A

## SALT SCREEN

Physical properties of a number of salt forms of ethyl cis-2-[4-(1-methyl-1H-pyrazol-5-yl)piperidin-1-yl]-6-azaspiro[3.4]octane-6-carboxylate were evaluated for suitability for further development. The objective was to find salts with desirable properties (e.g., reduced hygroscopicity, solubility, crystallinity, physical stability etc.) to support later phase clinical formulations. The following acids were investigated: 2,5-dihydroxybenzoic (gentisic), 2-furoic, acetic, butandioic

(succinic), citric, ethansulfonic (ESA), fumaric, gluconic (D), glucuronic (D), hydroxyacetic (glycolic), hydrochloric (HCl), maleic, malic (L), malonic, N-acetylglycine (aceturic), nicotinic, orthophosphoric (phosphoric), oxoglutaric (ketoglutaric), p-toluenesulfonic (p-TSA), pyroglutamic (L), sulphuric and tartaric (L).

Experiments were carried out at a scale of 20-30 mg with 1:1, 1:2, 2:1 and 4:1 stoichiometries (API: acid). Solutions of the free base in the chosen solvent were added to either solutions of the salt former or neat salt former. ethyl cis-2-[4-(1-methyl-1H-pyrazol-5-yl)piperidin-1-yl]-6-azaspiro[3.4]octane-6-carboxylate (free base) was added to a 5 mL (ACN, EtOH, THF, ACN/water EtOH/water or THF/water) or 20mL (MeOH) volumetric flask and made to volume to form a 1.0 M or 0.1M solution. This was sonicated in the water bath to ensure complete dissolution.

The salts were subjected to a number of tests and analytical techniques, including: NMR and XRPD analysis, humidity stress testing, dynamic vapour sorption (DVS) testing. Crystallisation, crystallinity, aqueous solubility and ease of manufacture were also assessed. The fumarate, citrate and HCl salts exhibited the best properties and so were selected for further study.

**Ethyl cis-2-[4-(1-methyl-1H-pyrazol-5-yl)piperidin-1-yl]-6-azaspiro[3.4]octane-6-carboxylate citrate (citrate salt) Characterisation**

The XRPD pattern obtained for the citrate salt (Synthetic Example 6), Pattern B is shown in Figure 1. The XRPD pattern is indicative of a crystalline material.

Thermogravimetric/Differential Thermal Analysis (TG/DTA) was performed to determine the thermal profile and associated % weight changes of the citrate salt (Figure 2). TG/DTA showed a weight loss of ~33% between ~140 °C and ~220 °C, most likely due to the loss of citric acid. A second weight loss at temperatures greater than 220 °C corresponded to the initiation of decomposition of the material. A broad endotherm was observed at onset temperature ~174 °C. The analysis indicated the citrate salt was anhydrous.

Polarised light microscopy (Figure 3) showed that the material was composed of crystalline solids with an irregular morphology.

The 1H NMR spectrum (Figure 4) was concordant with the molecular structure and minimal solvent was detected (<0.1 molar eq. of 2-BuOH). The ratio of API:acid was 1:1.

The hygroscopicity and the sorption properties of the citrate salt were determined using Dynamic Vapour Sorption (DVS). The isotherm obtained is shown in Figure 5. The sample was dried at 0 %RH prior to performing sorption and desorption. The isotherm showed the material exhibits gradual uptake of moisture, generating an increase in weight from 0 %RH to 80 %RH followed by a sharper weight gain between 80 to 90 %RH. The isotherm showed the total weight gain observed between ambient (i.e. 40 %RH) and 80 %RH to be ~0.8% which indicated that the sample is slightly hygroscopic, based on the European Pharmacopoeia classification, Table 1. Very little hysteresis was observed between the sorption and desorption curves and all moisture gained was lost by 0% RH. XRPD analysis performed post DVS (Figure 6) showed that the citrate salt had remained as Pattern B.

**Ethyl cis-2-[4-(1-methyl-1H-pyrazol-5-yl)piperidin-1-yl]-6-azaspiro[3.4]octane-6-carboxylate citrate monohydrate Characterisation**

Ethyl cis-2-[4-(1-methyl-1H-pyrazol-5-yl)piperidin-1-yl]-6-azaspiro[3.4]octane-6-carboxylate citrate monohydrate was obtained by the processes of Synthetic Examples 1, 2 and 3. A representative powder X-Ray Power Diffraction (XRPD) pattern is shown in Figure 25 and Table B shows the corresponding peaks and intensities. The XRPD diffractogram in Figure 25 suggested the sample was crystalline and exhibited a pattern consistent with that of ethyl cis-2-[4-(1-methyl-1H-pyrazol-5-yl)piperidin-1-yl]-6-azaspiro[3.4]octane-6-carboxylate citrate monohydrate.

Table B

| 2θ (degrees) | Height, counts | 2θ (degrees) | Height, counts |
|---|---|---|---|
| 6.513 | 284 | 25.862 | 518 |
| 9.133 | 70 | 26.172 | 203 |
| 10.184 | 515 | 26.572 | 338 |
| 11.266 | 544 | 27.884 | 312 |
| 12.536 | 189 | 28.480 | 154 |
| 12.790 | 214 | 28.927 | 498 |
| 13.777 | 268 | 29.803 | 281 |

(continued)

| 2θ (degrees) | Height, counts | 2θ (degrees) | Height, counts |
|---|---|---|---|
| 14.622 | 25.4 | 30.012 | 471 |
| 15.043 | 691 | 30.374 | 164 |
| 15.837 | 537 | 31.53 | 87 |
| 16.473 | 197 | 31.840 | 349 |
| 17.126 | 400 | 32.124 | 110 |
| 17.637 | 1846 | 32.533 | 328 |
| 18.4169 | 3781 | 33.79 | 38.4 |
| 18.688 | 85 | 34.09 | 235 |
| 19.175 | 792 | 36.133 | 96 |
| 19.993 | 261 | 36.797 | 76 |
| 20.530 | 2772 | 37.140 | 145 |
| 21.693 | 435 | 37.742 | 110 |
| 22.05 | 296 | 38.37 | 65 |
| 22.289 | 1162 | 38.579 | 138 |
| 22.740 | 710 | 39.332 | 292 |
| 23.416 | 141 | 40.49 | 109 |
| 24.121 | 904 | 42 | 0.089 |
| 24.24 | 130 | 43.08 | 123 |
| 25.458 | 111 | 44.18 | 68 |

Thermogravimetric/Differential Thermal Analysis (TG/DTA) was performed to determine the thermal profile and associated % weight changes of the citrate monohydrate salt (Figure 26). TG/DTA showed a weight loss step of ~0.155% from RT to ~65 °C, followed by a weight loss step of 3.188% from ~65 to 110 °C which appears to be associated with broad endothermic event with an onset of ~88.15 °C. This is likely due to the dehydration/loss of a bound hydrate (theoretical stoichiometric monohydrate - 3.14%) along with loss/change of crystal structure. This event is followed by a second weight step of 27.399% from ~170 to 210 °C which appears to be associated with an overlapping endothermic event including a sharp endotherm with onset of ~175.48 °C. This is likely due to the melt of the dehydrated form along with the loss of the acid (theoretical stoichiometric citrate - 25.7%), followed by immediate degradation.

Four samples of similar weight were prepared and analyzed via DSC with different ramping rates (5.0 °C/min, 10.0 °C/min, 20.0 °C/min, and 50.0 °C/min) to investigate the material's thermal behavior. The onset of dehydration/melt appears to be dependent of the heating rate, ranging from ~79 °C to 103 °C. The second sharper endothermic event appears to be somewhat dependent of the heating rate and was observed to range from about 174.1 °C to 178.5 °C, having an average onset melt of 176.4 °C ± 2 °C.

The hygroscopicity and the sorption properties of the citrate monohydrate salt were determined using Dynamic Vapour Sorption (DVS). The isotherm obtained is shown in Figure 27. The sorption isotherm above suggests citrate monohydrate salt is slightly hygroscopic, based on the European Pharmacopoeia classification, Table 1, with adsorption of ~0.770% water at 25 °C/80% RH. Though the moisture content is obtained at low humidity (likely recovering the loss from the drying step) and remains relatively constant. Diffractograms taken pre- and post-DVS suggest there is no change in form due to humidity change.

The primary particles for the citrate monohydrate were observed as prismatic/bladed particles generally less than 50 μm in size, which form aggregates generally greater than 100 μm. Representative images of aggregates by Polarized Light Microscopy (PLM ) and Scanning Electron Microscopy (SEM) and a single particle by SEM are shown in Figures 28 & 29.

## Reference ethyl cis-2-[4-(1-methyl-1H-pyrazol-5-yl)piperidin-1-yl]-6-azaspiro[3.4]octane-6-carboxylate hydrochloride (HCl salt) Characterisation

The XRPD pattern obtained for ethyl cis-2-[4-(1-methyl-1H-pyrazol-5-yl)piperidin-1-yl]-6-azaspiro[3.4]octane-6-carboxylate hydrochloride shown in Figure 7 was indicative of a crystalline material.

Thermogravimetric/Differential Thermal Analysis (TG/DTA) was performed to determine the thermal profile and associated % weight changes of ethyl cis-2-[4-(1-methyl-1H-pyrazol-5-yl)piperidin-1-yl]-6-azaspiro[3.4]octane-6-carboxylate hydrochloride (Figure 8). Weight loss of 3.6 % was noted from ~25 °C to 200 °C which corresponded to the loss of ~0.75 moles of water indicating that the HCl salt contained moisture. A second weight loss at temperatures greater than 210 °C corresponded to the initiation of decomposition of the material. An endotherm was observed at onset temperature ~234 °C.

The DSC thermogram obtained for ethyl cis-2-[4-(1-methyl-1H-pyrazol-5-yl)piperidin-1-yl]-6-azaspiro[3.4]octane-6-carboxylate hydrochloride is shown in Figure 9. The melting point could not be determined as decomposition initiated prior to the melt.

Polarised light microscopy (Figure 10) showed that the material was composed of crystalline solids with an irregular morphology.

The 1H NMR spectrum (Figure 11) was concordant with the molecular structure and minimal solvent was detected.

The hygroscopicity and the sorption properties of ethyl cis-2-[4-(1-methyl-1H-pyrazol-5-yl)piperidin-1-yl]-6-azaspiro[3.4]octane-6-carboxylate hydrochloride were determined using Dynamic Vapour Sorption (DVS). The isotherm obtained is shown in Figure 12. The sample was dried at 0 %RH prior to performing sorption and desorption. The isotherm showed the material exhibited a gradual uptake of moisture from 0 to 60 %RH followed by an increase in weight at a higher rate, between 60 %RH and 70 %RH. The rate of weight gain reduces between 70 %RH and 80 %RH and increases again between 80 and 90 %RH. The isotherm showed the total weight gain observed between ambient (i.e. 40 %RH) and 80 % RH to be ~7.5 %w/w which indicated that the sample was hygroscopic, based on the European Pharmacopoeia classification, Table 1. The rate of desorption was comparable with that of sorption between 90 and 70 %RH. However hysteresis was observed between the sorption and desorption plots between 70 and 0 %RH and ~3 % water remained at 0 %RH. XRPD analysis performed post DVS (Figure 13) showed that ethyl cis-2-[4-(1-methyl-1H-pyrazol-5-yl)piperidin-1-yl]-6-azaspiro[3.4]octane-6-carboxylate hydrochloride had converted to a known alternative HCl salt form. These results suggested that conversion to the alternative HCl salt material was occurring between 60 and 70 %RH.

## Reference ethyl cis-2-[4-(1-methyl-1H-pyrazol-5-yl)piperidin-1-yl]-6-azaspiro[3.4]octane-6-carboxylate fumarate (fumarate salt) Characterisation

The XRPD pattern obtained for ethyl cis-2-[4-(1-methyl-1H-pyrazol-5-yl)piperidin-1-yl]-6-azaspiro[3.4]octane-6-carboxylate fumarate, Pattern A, is shown in Figure 14. The XRPD pattern is indicative of a crystalline material.

Thermogravimetric/Differential Thermal Analysis (TG/DTA) was performed to determine the thermal profile and associated % weight changes of ethyl cis-2-[4-(1-methyl-1H-pyrazol-5-yl)piperidin-1-yl]-6-azaspiro[3.4]octane-6-carboxylate fumarate (Figure 15). No weight loss was observed between ~25°C and 180°C which indicated ethyl cis-2-[4-(1-methyl-1H-pyrazol-5-yl)piperidin-1-yl]-6-azaspiro[3.4]octane-6-carboxylate fumarate was anhydrous. A second weight loss at temperatures greater than 180 °C corresponded to the initiation of decomposition of the material. An endotherm most likely due to the melt was observed at onset temperature ~175 °C.

Polarised light microscopy (Figure 16) showed the material was crystalline and consisted of fine particles that tended to form aggregates/agglomerates.

The 1H NMR spectrum (Figure 17) was concordant with the molecular structure and no solvent was detected. The API:acid ratio was determined as 1:1.35.

The hygroscopicity and sorption properties of ethyl cis-2-[4-(1-methyl-1H-pyrazol-5-yl)piperidin-1-yl]-6-azaspiro[3.4]octane-6-carboxylate fumarate were determined using Dynamic Vapour Sorption (DVS). The isotherm obtained is shown in Figure 18. The sample was dried at 0 %RH prior to performing sorption and desorption. The isotherm showed the material exhibited a gradual uptake of moisture between 0 %RH to 60 %RH followed by a more pronounced uptake of water between 60 %RH to 80 %RH and a sharper uptake between 80 and 90 %RH. The isotherm showed the total weight gain observed between ambient (i.e. 40 %RH) and 80 %RH to be 0.3% which indicated that the sample was slightly hygroscopic, based on the European Pharmacopoeia classification, Table 1. There was little hysteresis between the sorption and desorption curves and all moisture gained was lost by 0% RH. XRPD analysis was performed post DVS (Figure 19). The diffractogram showed that ethyl cis-2-[4-(1-methyl-1H-pyrazol-5-yl)piperidin-1-yl]-6-azaspiro[3.4]octane-6-carboxylate fumarate had remained as Pattern A.

## Humidity stressing of salts

Samples of reference compound ethyl cis-2-[4-(1-methyl-1H-pyrazol-5-yl)piperidin-1-yl]-6-azaspiro[3.4]octane-6-carboxylate fumarate (fumarate salt), ethyl cis-2-[4-(1-methyl-1H-pyrazol-5-yl)piperidin-1-yl]-6-azaspiro[3.4]octane-6-carboxylate citrate (citrate salt) and reference compound ethyl cis-2-[4-(1-methyl-1H-pyrazol-5-yl)piperidin-1-yl]-6-azaspiro[3.4]octane-6-carboxylate hydrochloride (HCl salt) were placed in a relative humidity chamber at 40 °C/75 % RH for 7 days. XRPD analysis of samples after stressing did not detect a change in form for the fumarate and citrate salts (Figure 20, Figure 21), however the HCl salt showed conversion to an alternative HCl salt form (Figure 22).

## Aqueous solubility of salts

The aqueous solubility of the reference compound ethyl cis-2-[4-(1-methyl-1H-pyrazol-5-yl)piperidin-1-yl]-6-azaspiro[3.4]octane-6-carboxylate fumarate (fumarate salt) and ethyl cis-2-[4-(1-methyl-1H-pyrazol-5-yl)piperidin-1-yl]-6-azaspiro[3.4]octane-6-carboxylate citrate (citrate salt) was assessed by aliquot addition. The fumarate salt had almost all dissolved at ~320 mg/mL and the solution pH was ~4-5. However, on addition of more water, solids were precipitating. These solids were analysed by XRPD and determined to be fumaric acid (Figure 23). This would suggest that the fumarate salt was converting to free base and fumaric acid in water. The citrate salt showed aqueous solubility ~850-1700 mg/mL and the solution pH was ~3-4. The aqueous solubility of the HCl salt was ~340-390 mg/mL.

**Table 1: Hygroscopicity Classification (adopted from Ph.Eur. and Sihorkar et al (*Pharmaceutical Dev. & Technol.* (2013), 18(2), 348-358)**

| Classification | Weight increase from 40-80% RH (25 °C) |
|---|---|
| Non hygroscopic | <0.2% |
| Slightly hygroscopic | ≥0.2% and <2% |
| Hygroscopic | ≥2% and <15% |
| Very hygroscopic | ≥15% |
| Deliquescent | Sufficient water is absorbed to form a liquid |

## BIOLOGICAL DATA

Biological Example 1

Phospho-ERK1/2 assays

Functional assays were performed using the Alphascreen Surefire phospho-ERK1/2 assay (Crouch & Osmond, Comb. Chem. High Throughput Screen, 2008). ERK1/2 phosphorylation is a downstream consequence of both Gq/11 and Gi/o protein coupled receptor activation, making it highly suitable for the assessment of $M_1$, $M_3$ (Gq/11 coupled) and $M_2$, $M_4$ receptors (Gi/o coupled), rather than using different assay formats for different receptor subtypes. CHO cells stably expressing the human muscarinic $M_1$, $M_2$, $M_3$ or $M_4$ receptor were plated (25K / well) onto 96-well tissue culture plates in MEM-alpha + 10% dialysed FBS. Once adhered, cells were serum-starved overnight. Agonist stimulation was performed by the addition of 5 μL agonist to the cells for 5 min (37 °C). Media was removed and 50 μL of lysis buffer added. After 15 min, a 4 μL sample was transferred to 384-well plate and 7 μL of detection mixture added. Plates were incubated for 2 h with gentle agitation in the dark and then read on a PHERAstar plate reader. $pEC_{50}$ and $E_{max}$ values were calculated from the resulting data for each receptor subtype.

Activity data for ethyl cis-2-[4-(1-methyl-1H-pyrazol-5-yl)piperidin-1-yl]-6-azaspiro[3.4]octane-6-carboxylate is shown in Table D below.

Table D

| Ex.No. | $pEC_{50}$ $M_1$ (% Emax cf. ACh) | $pEC_{50}$ $M_2$ (% Emax cf. ACh) | $pEC_{50}$ $M_3$ (% Emax cf. ACh) | $pEC_{50}$ $M_4$ (% Emax cf. ACh) |
|---|---|---|---|---|
| ACh | 8.3 (102) | 7.8 (105) | 8.1 (115) | 8.1 (110) |

(continued)

| Ex.No. | $pEC_{50}$ $M_1$ (% Emax cf. ACh) | $pEC_{50}$ $M_2$ (% Emax cf. ACh) | $pEC_{50}$ $M_3$ (% Emax cf. ACh) | $pEC_{50}$ $M_4$ (% Emax cf. ACh) |
|---|---|---|---|---|
| 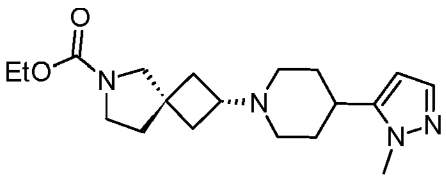 | <4.7 (11) | <4.7 (8) | <4.7 (0) | 7.4 (79) |

## Equivalents

The foregoing examples are presented for the purpose of illustrating the invention and should not be construed as imposing any limitation on the scope of the invention.

## Claims

1. A citrate salt of a compound according to formula (1):

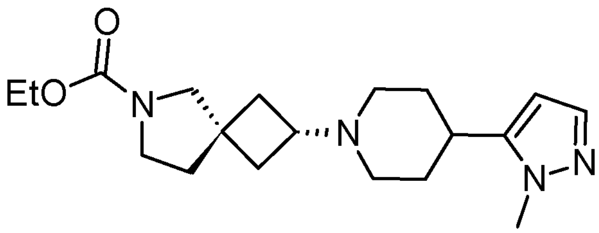

(1).

2. The citrate salt according to claim 1, wherein the citrate salt is a citrate monohydrate salt of formula (4b):

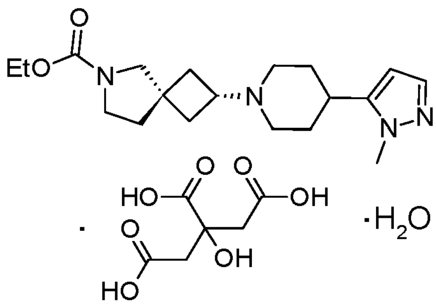

(4b).

3. The citrate salt according to claim 1, wherein the citrate salt is a compound of formula (3b):

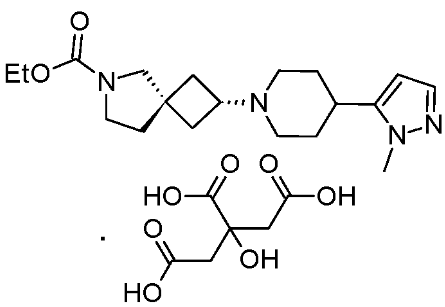

(3b).

4. A pharmaceutical composition comprising a citrate salt as defined in any one of claims 1 to 3, and a pharmaceutically acceptable excipient.

5. The citrate salt according to any one of claims 1-3; or

the composition according to claim 4;
for use in medicine.

6. The citrate salt according to any one of claims 1-3; or

the composition according to claim 4;
for use in the treatment of a cognitive disorder or psychotic disorder or for the treatment or lessening the severity of acute, chronic, neuropathic, or inflammatory pain.

7. A citrate salt or composition for use according to claim 6 wherein the disorder is Alzheimer's disease, dementia with Lewy bodies or schizophrenia.

8. A crystalline form of a salt of ethyl cis-2-[4-(1-methyl-1*H*-pyrazol-5-yl)piperidin-1-yl]-6-azaspiro[3.4]octane-6-carboxylate, wherein:
said salt of ethyl cis-2-[4-(1-methyl-1*H*-pyrazol-5-yl)piperidin-1-yl]-6-azaspiro[3.4]octane-6-carboxylate is a citrate salt of ethyl cis-2-[4-(1-methyl-1*H*-pyrazol-5-yl)piperidin-1-yl]-6-azaspiro[3.4]octane-6-carboxylate.

9. The crystalline form of claim 8, wherein said citrate salt of ethyl cis-2-[4-(1-methyl-1*H*-pyrazol-5-yl)piperidin-1-yl]-6-azaspiro[3.4]octane-6-carboxylate is ethyl cis-2-[4-(1-methyl-1*H*-pyrazol-5-yl)piperidin-1-yl]-6-azaspiro[3.4]octane-6-carboxylate citrate hydrate.

10. The crystalline form of claim 8 or 9, wherein said citrate salt of ethyl cis-2-[4-(1-methyl-1*H*-pyrazol-5-yl)piperidin-1-yl]-6-azaspiro[3.4]octane-6-carboxylate is ethyl cis-2-[4-(1-methyl-1*H*-pyrazol-5-yl)piperidin-1-yl]-6-azaspiro[3.4]octane-6-carboxylate citrate monohydrate.

11. The crystalline form according to any one of claims 8-10, **characterised by** three XRPD peaks, in terms of 2-theta, at:

11.3°± 0.2°, 19.2°± 0.2°, and 20.5°± 0.2°.

12. The crystalline form according to any one of claims 8-10, **characterised by** three XRPD peaks, in terms of 2-theta, at:

11.3°± 0.2°, 18.4°± 0.2°, and 20.5°± 0.2°.

13. The crystalline form according to any one of claims 8-10, **characterised by** three XRPD peaks, in terms of 2-theta, at:

11.3°± 0.2°, 18.4°± 0.2°, and 19.2°± 0.2°.

14. The crystalline form according to any one of claims 8-10, **characterised by** three XRPD peaks, in terms of 2-theta, at:

18.4°± 0.2°, 19.2°± 0.2°, and 20.5°± 0.2°.

15. The crystalline form according to any one of claims 8-10, **characterised by** four XRPD peaks, in terms of 2-theta, at 11.3°± 0.2°, 18.4°± 0.2°, 19.2°± 0.2°, and 20.5°± 0.2°.

16. The crystalline form according to any one of claims 8-10, **characterised by** an XRPD spectrum as shown in Figure 25.

17. The crystalline form according to any one of claims 10-16, **characterised by** a DSC thermogram having an endothermic peak with onset at 176 °C.

18. The crystalline form according to any one of claims 10-16, **characterised by** a DSC thermogram as shown in Figure 26.

**19.** The crystalline form according to any one of claims 10-18, **characterised by** a TGA thermogram with a weight loss of 3.3% loss from room temperature to 110 °C.

**20.** The crystalline form according to any one of claims 10-18, **characterised by** a TGA thermogram as shown in Figure 26.

**21.** The crystalline form of claim 8, wherein said citrate salt of ethyl cis-2-[4-(1-methyl-1H-pyrazol-5-yl)piperidin-1-yl]-6-azaspiro[3.4]octane-6-carboxylate is ethyl cis-2-[4-(1-methyl-1*H*-pyrazol-5-yl)piperidin-1-yl]-6-azaspiro[3.4]octane-6-carboxylate citrate.

**22.** The crystalline form according to claim 8 or 21, having:
at least one characterisation selected from the group consisting of:

(1) an XRPD spectrum as shown in Figure 1;
(2) a DTA thermogram having an endothermic peak with onset at 174 °C; and
(3) a TGA thermogram with a weight loss of 33% loss from 140 °C to 220 °C.

**23.** The crystalline form according to claim 8 or 21, having:
at least one characterisation selected from the group consisting of:

(1) an XRPD spectrum as shown in Figure 1;
(2) a DTA thermogram as shown in Figure 2; and
(3) a TGA thermogram as shown in Figure 2.

**24.** The crystalline form according to claim 22 or 23, wherein the crystalline form has characterisation (1).

**25.** The crystalline form according to claim 22 or 23, wherein the crystalline form has characterisation (2).

**26.** The crystalline form according to claim 22 or 23, wherein the crystalline form has characterisation (3).

**27.** The crystalline form according to claim 22 or 23, wherein the crystalline form has characterisations (1) and (2).

**28.** The crystalline form according to claim 22 or 23, wherein the crystalline form has characterisations (1) and (3).

**29.** The crystalline form according to claim 22 or 23, wherein the crystalline form has characterisations (2) and (3).

**30.** The crystalline form according to claim 22 or 23, wherein the crystalline form has characterisations (1), (2) and (3).

**31.** A pharmaceutical composition comprising a crystalline form according to any one of claims 8-30, and a pharmaceutically acceptable excipient.

**32.** A citrate salt according to any one of claims 1-3, or

a crystalline form according to any one of claims 8-30, or
a pharmaceutical composition according to claim 4 or 31,
for use in the treatment of a cognitive disorder or psychotic disorder.

**33.** The citrate salt, crystalline form, or pharmaceutical composition for use according to claim 32, wherein:
the cognitive disorder or psychotic disorder is, comprises, arises from or is associated with a condition selected from: cognitive impairment, Mild Cognitive Impairment (MCI), frontotemporal dementia, vascular dementia, dementia with Lewy bodies, presenile dementia, senile dementia, Friedreich's ataxia, Down's syndrome, Huntington's chorea, hyperkinesia, mania, Tourette's syndrome, Alzheimer's disease , progressive supranuclear palsy, impairment of cognitive functions including attention, orientation, learning disorders, memory and language function; cognitive impairment as a result of stroke, Huntington's disease, Pick disease, AIDS-related dementia or other dementia states such as multi-infarct dementia, alcoholic dementia, hypothyroidism-related dementia, and dementia associated to other degenerative disorders such as cerebellar atrophy and amyotrophic lateral sclerosis; other acute or sub-acute conditions that may cause cognitive decline such as delirium or depression, trauma, head trauma, age related cognitive decline, stroke, neurodegeneration, drug-induced states, neurotoxic agents, age related cognitive impairment, autism related cognitive impairment, Down's syndrome, cognitive deficit related to psychosis, and post-electroconvulsive treatment related cognitive disorders; cognitive disorders due to drug abuse or drug withdrawal including nicotine, cannabis, amphetamine, cocaine, Attention Deficit Hyperactivity Disorder (ADHD) and dyskinetic disorders such as Parkinson's disease, neuroleptic-induced parkinsonism, and tardive dyskinesias, schizophrenia, schizophreniform diseases, psychotic depression, mania, acute mania, paranoid, hallucinogenic and delusional disorders, personality disorders, obsessive compulsive disorders, schizotypal disorders, delusional disorders, psychosis due to malignancy, metabolic disorder, endocrine disease or narcolepsy, psychosis due to drug abuse or drug withdrawal, bipolar disorders, schizo-affective disorder, and Alzheimer's psychosis.

**34.** The citrate salt, crystalline form, or pharmaceutical composition for use according to claim 32, wherein the treatment is treatment of Schizophrenia.

**35.** The citrate salt, crystalline form, or pharmaceutical composition for use according to claim 32, wherein the treatment is treatment of bipolar disorders.

**36.** The citrate salt, crystalline form, or pharmaceutical composition for use according to claim 32, wherein the treatment is treatment of Alzheimer's psychosis.

## Patentansprüche

**1.** Citrat-Salz einer Verbindung gemäß Formel (1):

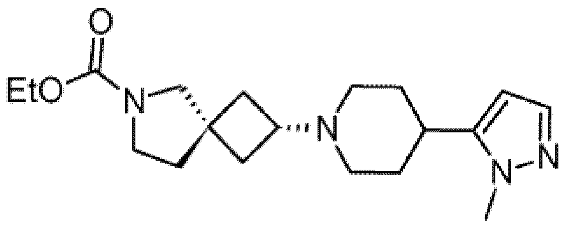

(1).

**2.** Citrat-Salz nach Anspruch 1, wobei das Citrat-Salz ein Citrat-monohydrat-Salz der Formel (4b) ist:

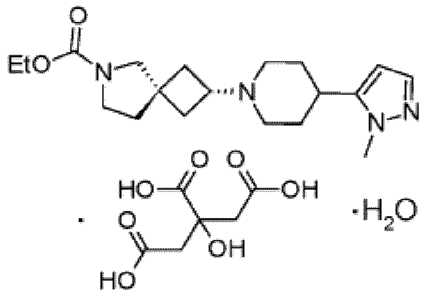

(4b).

**3.** Citrat-Salz nach Anspruch 1, wobei das Citrat-Salz eine Verbindung der Formel (3b) ist:

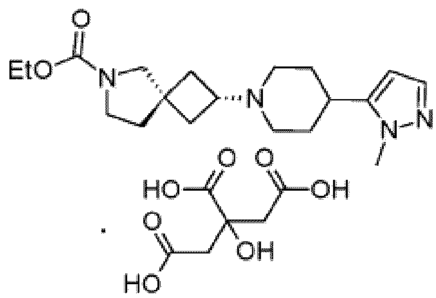

(3b).

4. Pharmazeutische Zusammensetzung, die ein Citrat-Salz wie in einem der Ansprüche 1 bis 3 definiert und einen pharmazeutisch annehmbaren Hilfsstoff umfasst.

5. Citrat-Salz nach einem der Ansprüche 1 bis 3 oder Zusammensetzung nach Anspruch 4
zur Verwendung in der Medizin.

6. Citrat-Salz nach einem der Ansprüche 1 bis 3; oder Zusammensetzung nach Anspruch 4
zur Verwendung bei der Behandlung einer kognitiven Störung oder psychotischen Störung oder zur Behandlung oder Linderung des Schweregrads von akuten, chronischen, neuropathischen oder entzündungsbedingten Schmerzen.

7. Citrat-Salz oder Zusammensetzung zur Verwendung nach Anspruch 6, wobei die Störung Morbus Alzheimer, Demenz mit Lewy-Körperchen oder Schizophrenie ist.

8. Kristalline Form eines Salzes von Ethyl-cis-2-[4-(1-methyl-1H-pyrazol-5-yl)piperidin-1-yl]-6-azaspiro[3.4]octan-6-carboxylat, wobei:
das Salz von Ethyl-cis-2-[4-(1-methyl-1H-pyrazol-5-yl)piperidin-1-yl]-6-azaspiro[3.4]-octan-6-carboxylat ein Citrat-Salz von Ethyl-cis-2-[4-(1-methyl-1H-pyrazol-5-yl)piperidin-1-yl]-6-azaspiro[3.4]octan-6-carboxylat ist.

9. Kristalline Form nach Anspruch 8, wobei das Citrat-Salz von Ethyl-cis-2-[4-(1-methyl-1H-pyrazol-5-yl)piperidin-1-yl]-6-azaspiro[3.4]octan-6-carboxylat Ethyl-cis-2-[4-(1-methyl-1H-pyrazol-5-yl)piperidin-1-yl]-6-azaspiro[3.4]octan-6-carboxylat-citrat-hydrat ist.

10. Kristalline Form nach Anspruch 8 oder 9, wobei das Citrat-Salz von Ethyl-cis-2-[4-(1-methyl-1H-pyrazol-5-yl)piperidin-1-yl]-6-azaspiro[3.4]octan-6-carboxylat Ethyl-cis-2-[4-(1-methyl-1H-pyrazol-5-yl)piperidin-1-yl]-6-azaspiro[3.4]octan-6-carboxylat-citrat-monohydrat ist.

11. Kristalline Form nach einem der Ansprüche 8 bis 10, **gekennzeichnet durch** drei XRPD-Reflexe, ausgedrückt als 2θ, bei:

$$11,3° \pm 0,2°,\ 19,2° \pm 0,2° \text{ und } 20,5° \pm 0,2°.$$

12. Kristalline Form nach einem der Ansprüche 8 bis 10, **gekennzeichnet durch** drei XRPD-Reflexe, ausgedrückt als 2θ, bei:

$$11,3° \pm 0,2°,\ 18,4° \pm 0,2° \text{ und } 20,5° \pm 0,2°.$$

13. Kristalline Form nach einem der Ansprüche 8 bis 10, **gekennzeichnet durch** drei XRPD-Reflexe, ausgedrückt als 2θ, bei:

$$11,3° \pm 0,2°,\ 18,4° \pm 0,2° \text{ und } 19,2° \pm 0,2°.$$

14. Kristalline Form nach einem der Ansprüche 8 bis 10, **gekennzeichnet durch** drei XRPD-Reflexe, ausgedrückt als 2θ, bei:

$$18,4° \pm 0,2°,\ 19,2° \pm 0,2° \text{ und } 20,5° \pm 0,2°.$$

15. Kristalline Form nach einem der Ansprüche 8 bis 10, **gekennzeichnet durch** vier XRPD-Reflexe, ausgedrückt als 2θ, bei:

$$11,3° \pm 0,2°,\ 18,4° \pm 0,2°,\ 19,2° \pm 0,2° \text{ und } 20,5° \pm 0,2°.$$

16. Kristalline Form nach einem der Ansprüche 8 bis 10, **gekennzeichnet durch** ein XRPD-Spektrum wie in Figur 25 dargestellt.

17. Kristalline Form nach einem der Ansprüche 10 bis 16, **gekennzeichnet durch** ein DSC-Thermogramm mit einem

endothermen Peak, beginnend bei 176 °C.

**18.** Kristalline Form nach einem der Ansprüche 10 bis 16, **gekennzeichnet durch** ein DSC-Thermogramm wie in Figur 26 dargestellt.

**19.** Kristalline Form nach einem der Ansprüche 10 bis 18, **gekennzeichnet durch** ein TGA-Thermogramm mit einem Gewichtsverlust von 3,3 % Verlust von Raumtemperatur bis 110 °C.

**20.** Kristalline Form nach einem der Ansprüche 10 bis 18, **gekennzeichnet durch** ein TGA-Thermogramm wie in Figur 26 dargestellt.

**21.** Kristalline Form nach Anspruch 8, wobei das Citrat-Salz von Ethyl-cis-2-[4-(1-methyl-1H-pyrazol-5-yl)piperidin-1-yl]-6-azaspiro[3.4]octan-6-carboxylat Ethyl-cis-2-[4-(1-methyl-1H-pyrazol-5-yl)piperidin-1-yl]-6-azaspiro[3.4]octan-6-carboxylat-citrat ist.

**22.** Kristalline Form nach Anspruch 8 oder 21, die Folgendes aufweist:
zumindest eine Charakterisierung, die aus der aus den folgenden bestehenden Gruppe ausgewählt ist:

(1) einem XRPD-Spektrum wie in Fig. 1 dargestellt;
(2) einem DTA-Thermogramm mit einem endothermen Peak, beginnend bei 174 °C; und
(3) einem TGA-Thermogramm mit einem Gewichtsverlust von 33 % Verlust von 140 °C bis 220 °C.

**23.** Kristalline Form nach Anspruch 8 oder 21, die Folgendes aufweist:
zumindest eine Charakterisierung, die aus der aus den folgenden bestehenden Gruppe ausgewählt ist:

(1) einem XRPD-Spektrum wie in Figur 1 dargestellt;
(2) einem DTA-Thermogramm wie in Figur 2 dargestellt; und
(3) einem TGA-Thermogramm wie in Figur 2 dargestellt.

**24.** Kristalline Form nach Anspruch 22 oder 23, wobei die kristalline Form die Charakterisierung (1) aufweist.

**25.** Kristalline Form nach Anspruch 22 oder 23, wobei die kristalline Form die Charakterisierung (2) aufweist.

**26.** Kristalline Form nach Anspruch 22 oder 23, wobei die kristalline Form die Charakterisierung (3) aufweist.

**27.** Kristalline Form nach Anspruch 22 oder 23, wobei die kristalline Form die Charakterisierungen (1) und (2) aufweist.

**28.** Kristalline Form nach Anspruch 22 oder 23, wobei die kristalline Form die Charakterisierungen (1) und (3) aufweist.

**29.** Kristalline Form nach Anspruch 22 oder 23, wobei die kristalline Form die Charakterisierungen (2) und (3) aufweist.

**30.** Kristalline Form nach Anspruch 22 oder 23, wobei die kristalline Form die Charakterisierungen (1), (2) und (3) aufweist.

**31.** Pharmazeutische Zusammensetzung, die eine kristalline Form nach einem der Ansprüche 8 bis 30 und einen pharmazeutisch annehmbaren Hilfsstoff umfasst.

**32.** Citrat-Salz nach einem der Ansprüche 1 bis 3 oder

kristalline Form nach einem der Ansprüche 8 bis 30 oder
pharmazeutische Zusammensetzung nach Anspruch 4 oder 31
zur Verwendung bei der Behandlung einer kognitiven Störung oder psychotischen Störung.

**33.** Citrat-Salz, kristalline Form oder pharmazeutische Zusammensetzung zur Verwendung nach Anspruch 32, wobei:
die kognitive Störung oder psychotische Störung ein Leiden umfasst, daraus entsteht oder damit im Zusammenhang steht, das aus den folgenden ausgewählt ist:
kognitiver Beeinträchtigung, milder kognitiver Beeinträchtigung (MCI), frontotemporaler Demenz, vaskulärer Demenz, Demenz mit Lewy-Körpern, präseniler Demenz, seniler Demenz, Friedreich-Ataxie, Down-Syndrom, Chorea

Huntington, Hyperkinesie, Manie, Tourette-Syndrom, Alzheimer-Krankheit, progressiver supranukleärer Lähmung, Beeinträchtigung kognitiver Funktionen einschließlich Aufmerksamkeit, Orientierung, Lernstörungen, Gedächtnis und Sprachfunktion; kognitiver Beeinträchtigung infolge eines Schlaganfalls, der Huntington-Krankheit, der Pick-Krankheit, einer AIDS-bedingten Demenz oder anderer Demenzzustände wie Multi-Infarkt-Demenz, alkoholbedingte Demenz, Demenz im Zusammenhang mit einer Schilddrüsenunterfunktion und Demenz im Zusammenhang mit anderen degenerativen Erkrankungen wie Kleinhirnatrophie und amyotropher Lateralsklerose; anderen akuten oder subakuten Erkrankungen, die einen kognitiven Verfall verursachen können, wie Delirium oder Depression, Trauma, Schädel-Hirn-Trauma, altersbedingter kognitiver Verfall, Schlaganfall, Neurodegeneration, drogeninduzierte Zustände, neurotoxische Wirkstoffe, altersbedingte kognitive Beeinträchtigungen, autismusbedingte kognitive Beeinträchtigungen, Down-Syndrom, kognitive Defizite im Zusammenhang mit Psychosen und kognitive Störungen nach Elektrokrampfbehandlungen; kognitiven Störungen aufgrund von Drogenmissbrauch oder Drogenentzug, einschließlich Nikotin, Cannabis, Amphetamin, Kokain, Aufmerksamkeitsdefizit-Hyperaktivitätsstörung (ADHS) und dyskinetischen Störungen wie Parkinson-Krankheit, Neuroleptika-induzierter Parkinsonismus und tardive Dyskinesien, Schizophrenie, schizophreniforme Erkrankungen, psychotische Depression, Manie, akute Manie, paranoide, halluzinogene und wahnhafte Störungen, Persönlichkeitsstörungen, Zwangsstörungen, schizotypische Störungen, wahnhafte Störungen, Psychosen aufgrund von bösartigen Erkrankungen, Stoffwechselstörungen, endokrinen Erkrankungen oder Narkolepsie, Psychosen aufgrund von Drogenmissbrauch oder Drogenentzug, bipolaren Störungen, schizo-affektiven Störungen und Alzheimer-Psychosen.

**34.** Citrat-Salz, kristalline Form oder pharmazeutische Zusammensetzung zur Verwendung nach Anspruch 32, wobei die Behandlung die Behandlung von Schizophrenie ist.

**35.** Citrat-Salz, kristalline Form oder pharmazeutische Zusammensetzung zur Verwendung nach Anspruch 32, wobei die Behandlung die Behandlung von bipolaren Störungen ist.

**36.** Citrat-Salz, kristalline Form oder pharmazeutische Zusammensetzung zur Verwendung nach Anspruch 32, wobei die Behandlung die Behandlung von Alzheimer-Psychose ist.

## Revendications

**1.** Sel de citrate d'un composé de formule (1) :

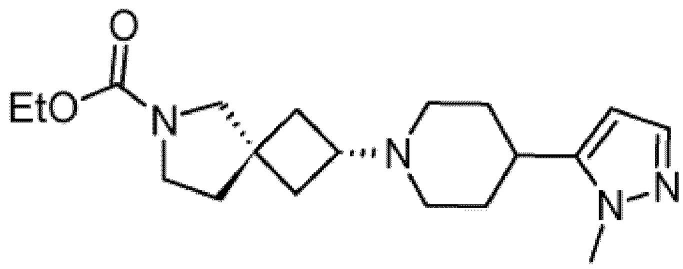

(1).

**2.** Sel de citrate selon la revendication 1, dans lequel le sel de citrate est un sel de citrate monohydraté de formule (4b) :

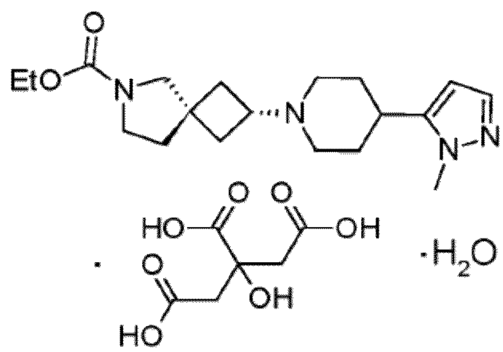

(4b).

**3.** Sel de citrate selon la revendication 1, dans lequel le sel de citrate est un sel de citrate monohydraté de formule (3b) :

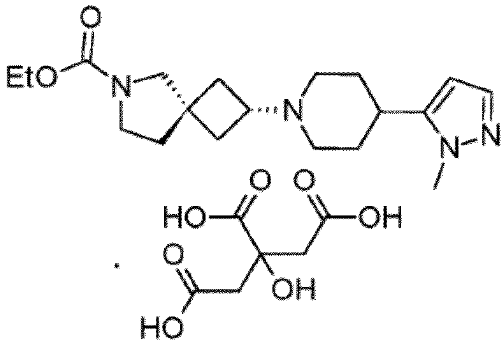

(3b).

**4.** Composition pharmaceutique comprenant un sel de citrate tel que défini dans l'une quelconque des revendications 1 à 3 et un excipient pharmaceutiquement acceptable.

**5.** Sel de citrate selon l'une quelconque des revendications 1 à 3 ; ou

composition selon la revendication 4 ;
pour une utilisation en médecine.

**6.** Sel de citrate selon l'une quelconque des revendications 1 à 3 ; ou

composition selon la revendication 4 ;
pour une utilisation dans le traitement d'un trouble cognitif ou psychotique ou pour le traitement ou l'atténuation de la sévérité d'une douleur aiguë, chronique, neuropathique ou inflammatoire.

**7.** Sel de citrate ou composition pour une utilisation selon la revendication 6 dans lesquels le trouble est la maladie d'Alzheimer, la démence à corps de Lewy ou la schizophrénie.

**8.** Forme cristalline d'un sel de cis-2-[4-(1-méthyl-1H-pyrazol-5-yl)pipéridin-1-yl]-6-azaspiro[3.4]octane-6-carboxylate d'éthyle, dans laquelle :
ledit sel de cis-2-[4-(1-méthyl-1H-pyrazol-5-yl)pipéridin-1-yl]-6-azaspiro[3.4]octane-6-carboxylate d'éthyle est un sel citrate de cis-2-[4-(1-méthyl-1H-pyrazol-5-yl)pipéridin-1-yl]-6-azaspiro[3.4]octane-6-carboxylate d'éthyle est un sel de citrate de cis-2-[4-(1-méthyl-1H-pyrazol-5-yl)pipéridin-1-yl]-6-azaspiro[3.4]octane-6-carboxylate d'éthyle est un sel citrate de cis-2-[4-(1-méthyl-1H-pyrazol-5-yl)pipéridin-1-yl]-6-azaspiro[3.4]octane-6-carboxylate d'éthyle.

**9.** Forme cristalline selon la revendication 8, dans laquelle ledit sel citrate de cis-2-[4-(1-méthyl-1H-pyrazol-5-yl) pipéridin-1-yl]-6-azaspiro[3.4]octane-6-carboxylate d'éthyle est le citrate de cis-2-[4-(1-méthyl-1H-pyrazol-5-yl)pipéridin-1-yl]-6-azaspiro[3.4]octane-6-carboxylate d'éthyle hydraté.

**10.** Forme cristalline selon la revendication 8 ou 9, dans laquelle ledit sel citrate de cis-2-[4-(1-méthyl-1H-pyrazol-5-yl) pipéridin-1-yl]-6-azaspiro[3.4]octane-6-carboxylate d'éthyle est le citrate de cis-2-[4-(1-méthyl-1H-pyrazol-5-yl)pipéridin-1-yl]-6-azaspiro[3.4]octane-6-carboxylate d'éthyle monohydraté.

**11.** Forme cristalline selon l'une quelconque des revendications 8 à 10, **caractérisée par** trois pics de XRPD, en termes de 2-thêta, à :

11,3°± 0,2°, 19,2°± 0,2° et 20,5°± 0,2°.

**12.** Forme cristalline selon l'une quelconque des revendications 8 à 10, **caractérisée par** trois pics de XRPD, en termes de 2-thêta, à :

11,3°± 0,2°, 18,4°± 0,2° et 20,5°± 0,2°.

**13.** Forme cristalline selon l'une quelconque des revendications 8 à 10, **caractérisée par** trois pics de XRPD, en termes de 2-thêta, à :

11,3°± 0,2°, 18,4°± 0,2° et 19,2°± 0,2°.

**14.** Forme cristalline selon l'une quelconque des revendications 8 à 10, **caractérisée par** trois pics de XRPD, en termes de 2-thêta, à :

18,4°± 0,2°, 19,2°± 0,2° et 20,5°± 0,2°.

**15.** Forme cristalline selon l'une quelconque des revendications 8 à 10, **caractérisée par** quatre pics de XRPD, en termes de 2-thêta, à 11,3°± 0,2°, 18,4°± 0,2°, 19,2°± 0,2° et 20,5°± 0,2°.

**16.** Forme cristalline selon l'une quelconque des revendications 8 à 10, **caractérisée par** un spectre XRPD tel qu'illustré sur la figure 25.

**17.** Forme cristalline selon l'une quelconque des revendications 10 à 16, **caractérisée par** un thermogramme DSC présentant un pic endothermique apparaissant à 176 °C.

**18.** Forme cristalline selon l'une quelconque des revendications 10 à 16, **caractérisée par** un thermogramme DSC tel qu'illustré sur la figure 26.

**19.** Forme cristalline selon l'une quelconque des revendications 10 à 18, **caractérisée par** un thermogramme TGA présentant une perte de masse d'environ 3,3 % de la température ambiante à environ 110 °C.

**20.** Forme cristalline selon l'une quelconque des revendications 10 à 18, **caractérisée par** un thermogramme TGA tel qu'illustré sur la figure 26.

**21.** Forme cristalline selon la revendication 8, dans laquelle ledit sel citrate de cis-2-[4-(1-méthyl-1H-pyrazol-5-yl) pipéridin-1-yl]-6-azaspiro[3.4]octane-6-carboxylate d'éthyle est le citrate de cis-2-[4-(1-méthyl-1H-pyrazol-5-yl)pipéridin-1-yl]-6-azaspiro[3.4]octane-6-carboxylate d'éthyle.

**22.** Forme cristalline selon la revendication 8 ou 21, ayant :
au moins une caractéristique choisie parmi :

(1) un spectre XRPD tel qu'illustré sur la figure 1 ;
(2) un thermogramme DTA ayant un pic endothermique apparaissant à 174 °C ; et
(3) un thermogramme TGA présentant une perte de masse d'environ 33 % de 140 °C à 220 °C.

**23.** Forme cristalline selon la revendication 8 ou 21, ayant :
au moins une caractéristique choisie parmi :

(1) un spectre XRPD tel qu'illustré sur la figure 1 ;
(2) un thermogramme DTA tel qu'illustré sur la figure 2 ; et
(3) un thermogramme TGA tel qu'illustré sur la figure 2.

**24.** Forme cristalline selon la revendication 22 ou 23, dans laquelle la forme cristalline a la caractéristique (1).

**25.** Forme cristalline selon la revendication 22 ou 23, dans laquelle la forme cristalline a la caractéristique (2).

**26.** Forme cristalline selon la revendication 22 ou 23, dans laquelle la forme cristalline a la caractéristique (3).

**27.** Forme cristalline selon la revendication 22 ou 23, dans laquelle la forme cristalline a les caractéristiques (1) et (2).

**28.** Forme cristalline selon la revendication 22 ou 23, dans laquelle la forme cristalline a les caractéristiques (1) et (3).

**29.** Forme cristalline selon la revendication 22 ou 23, dans laquelle la forme cristalline a les caractéristiques (2) et (3).

**30.** Forme cristalline selon la revendication 22 ou 23, dans laquelle la forme cristalline a les caractéristiques (1), (2) et (3).

**31.** Composition pharmaceutique comprenant la forme cristalline selon l'une quelconque des revendications 8 à 30 et un excipient pharmaceutiquement acceptable.

**32.** Sel de citrate selon l'une quelconque des revendications 1 à 3, ou

forme cristalline selon l'une quelconque des revendications 8 à 30, ou
composition pharmaceutique selon la revendication 4 ou 31,
pour une utilisation dans le traitement d'un trouble cognitif ou psychotique.

**33.** Sel de citrate, forme cristalline ou composition pharmaceutique pour une utilisation selon la revendication 32, dans lesquels :
le trouble cognitif ou le trouble psychotique est, comprend, provient de ou est associé à une affection choisie parmi : la déficience cognitive, la déficience cognitive légère (DCL), la démence frontotemporale, la démence vasculaire, la démence à corps de Lewy, la démence présénile, la démence sénile, l'ataxie de Friedreich, le syndrome de Down, la chorée de Huntington, l'hyperkinésie, la manie, le syndrome de Gilles de la Tourette, la maladie d'Alzheimer, la paralysie supranucléaire progressive, la déficience des fonctions cognitives, y compris l'attention, l'orientation, les troubles de l'apprentissage, la mémoire et la fonction du langage ; la déficience cognitive résultant d'un accident vasculaire cérébral, la maladie de Huntington, la maladie de Pick, la démence liée au sida ou d'autres états démentiels tels que la démence par infarctus multiples, la démence alcoolique, la démence liée à l'hypothyroïdie et la démence associée à d'autres troubles dégénératifs tels que l'atrophie cérébelleuse et la sclérose latérale amyotrophique ; d'autres affections aiguës ou subaiguës pouvant entraîner un déclin cognitif telles que le délire ou la dépression, un traumatisme, un traumatisme crânien, un déclin cognitif lié à l'âge, un accident vasculaire cérébral, une neurodégénérescence, des états induits par des médicaments, des agents neurotoxiques, une déficience cognitive liée à l'âge, une déficience cognitive liée à l'autisme, le syndrome de Down, un déficit cognitif lié à la psychose et des troubles cognitifs liés à un traitement post-électroconvulsif ; des troubles cognitifs dus à l'abus de drogues ou au sevrage de drogues, y compris la nicotine, le cannabis, les amphétamines, la cocaïne, le trouble déficitaire de l'attention avec hyperactivité (TDAH) et les troubles dyskinétiques tels que la maladie de Parkinson, le parkinsonisme induit par les neuroleptiques et les dyskinésies tardives, la schizophrénie, les maladies schizophréniformes, la dépression psychotique, la manie, la manie aiguë, les troubles paranoïaques, hallucinogènes et délirants, les troubles de la personnalité, les troubles obsessionnels compulsifs, les troubles schizotypiques, les troubles délirants, la psychose due à une tumeur maligne, les troubles métaboliques, les maladies endocriniennes ou la narcolepsie, la psychose due à l'abus de drogues ou au sevrage de drogues, les troubles bipolaires, le trouble schizoaffectif et la psychose d'Alzheimer.

**34.** Sel de citrate, forme cristalline ou composition pharmaceutique pour une utilisation selon la revendication 32, dans lesquels le traitement est le traitement de la schizophrénie.

**35.** Sel de citrate, forme cristalline ou composition pharmaceutique pour une utilisation selon la revendication 32, dans lesquels le traitement est le traitement des troubles bipolaires.

**36.** Sel de citrate, forme cristalline ou composition pharmaceutique pour une utilisation selon la revendication 32, dans lesquels le traitement est le traitement de la psychose d'Alzheimer.

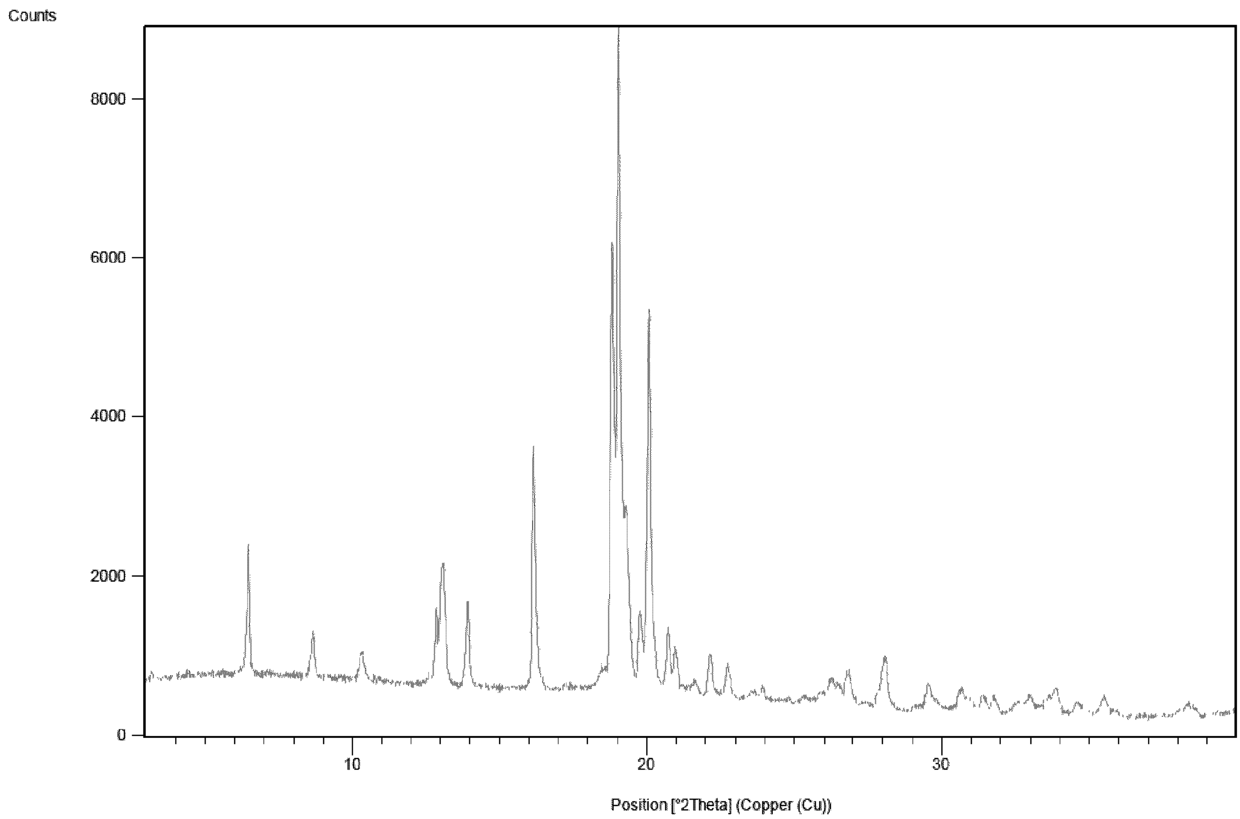
Counts
8000
6000
4000
2000
0
10
20
30
Position [°2Theta] (Copper (Cu))

Fig. 1

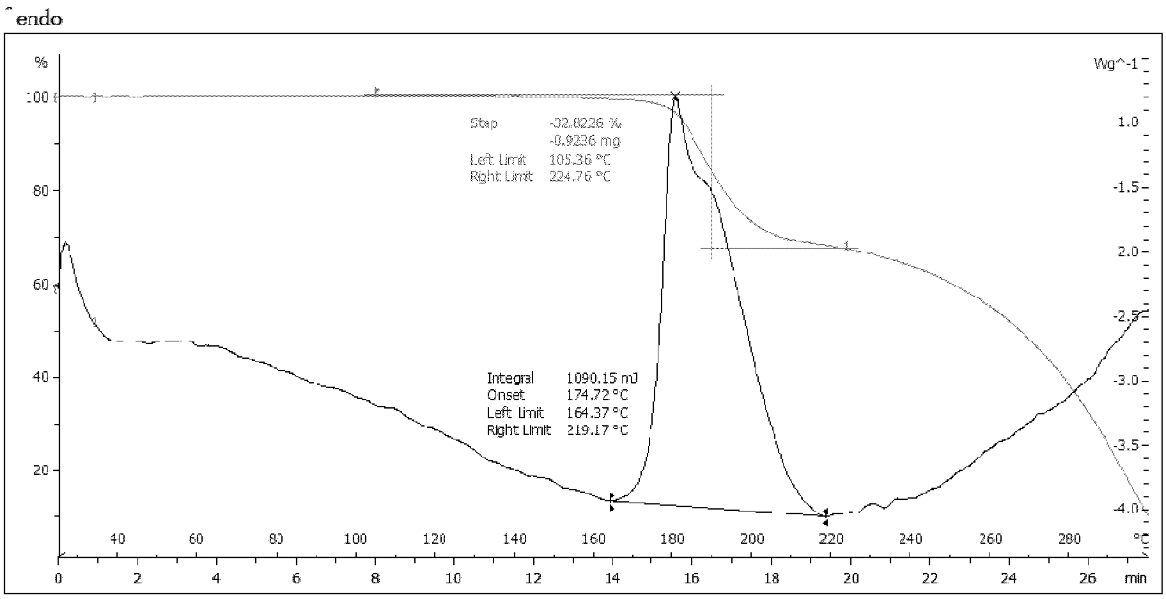
^ endo
%
100
80
60
40
20
Wg^-1
1.0
-1.5
2.0
-2.5
-3.0
-3.5
-4.0
Step -32.8226 %
-0.9236 mg
Left Limit 105.36 °C
Right Limit 224.76 °C
Integral 1090.15 mJ
Onset 174.72 °C
Left Limit 164.37 °C
Right Limit 219.17 °C
40 60 80 100 120 140 160 180 200 220 240 260 280 °C
0 2 4 6 8 10 12 14 16 18 20 22 24 26 min

**Fig. 2**

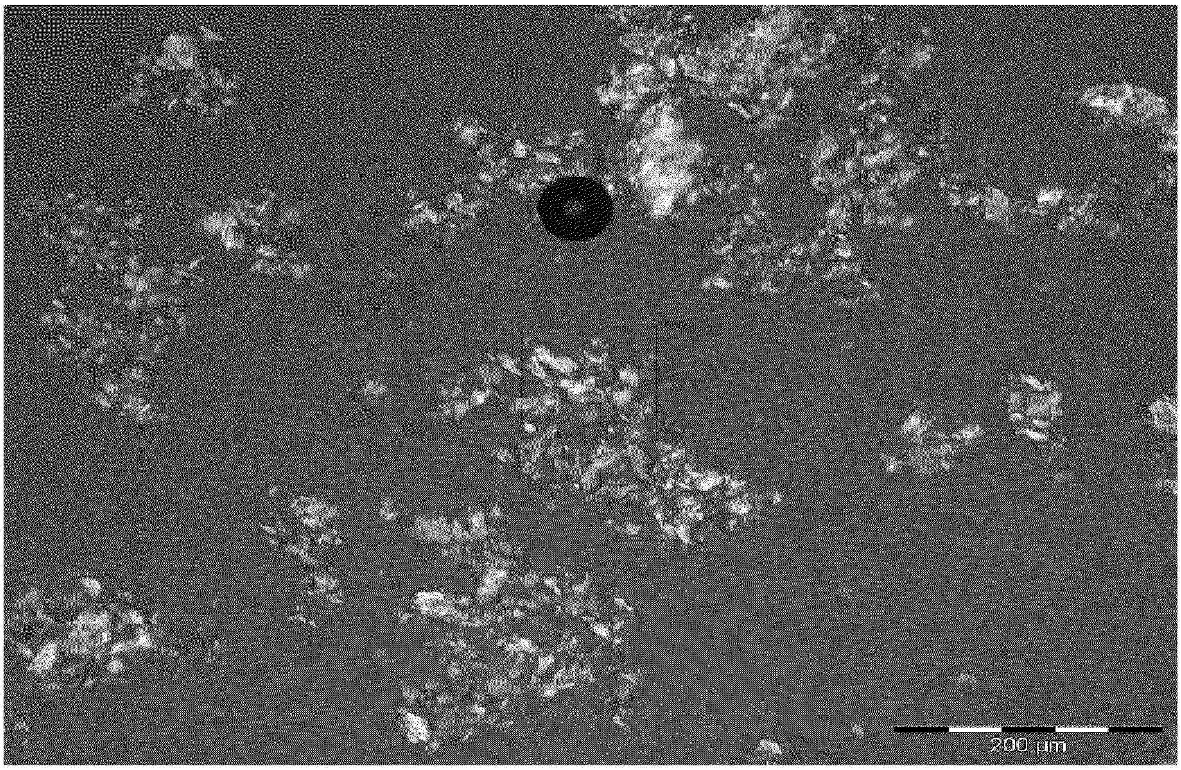


Fig. 3

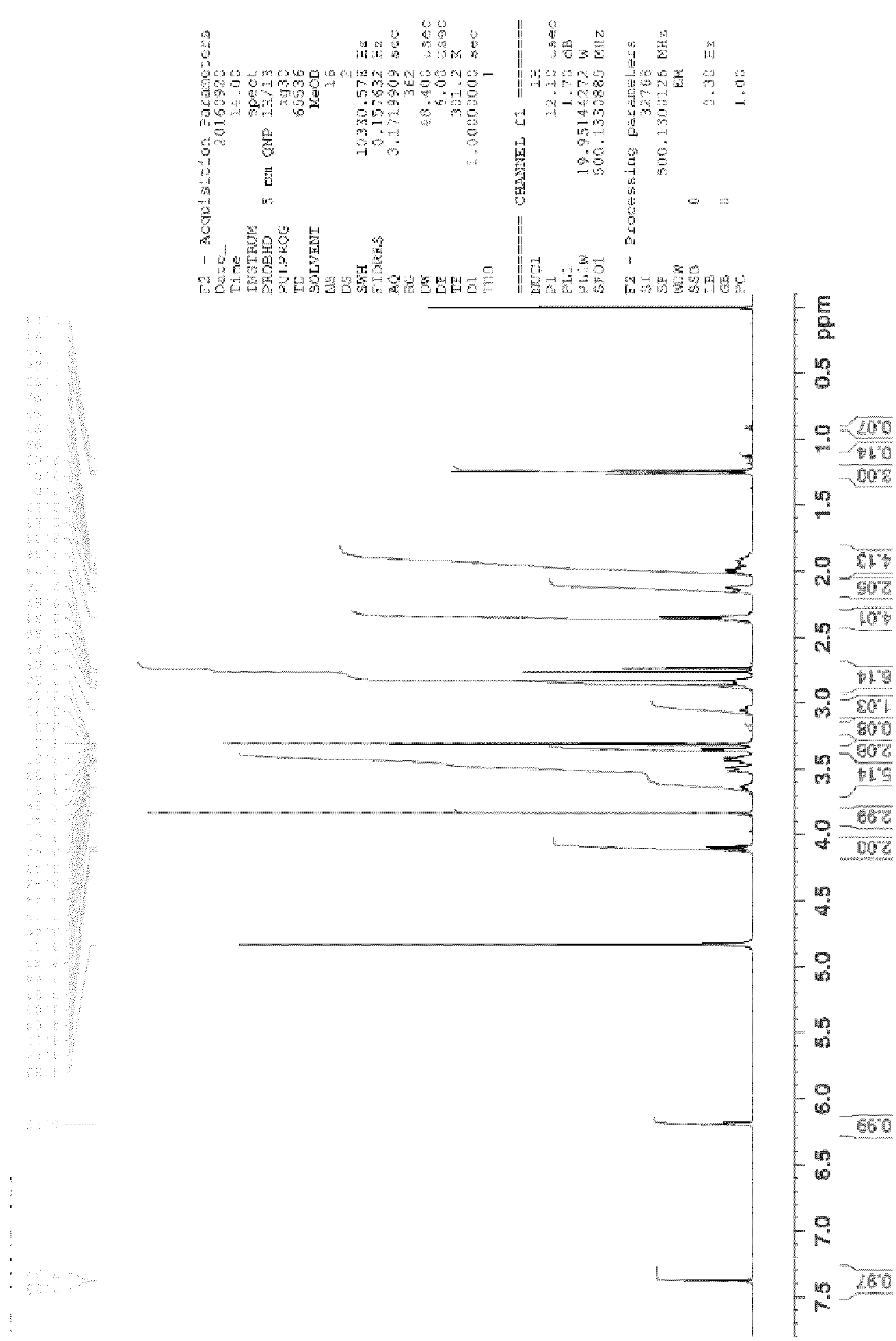

Fig. 4

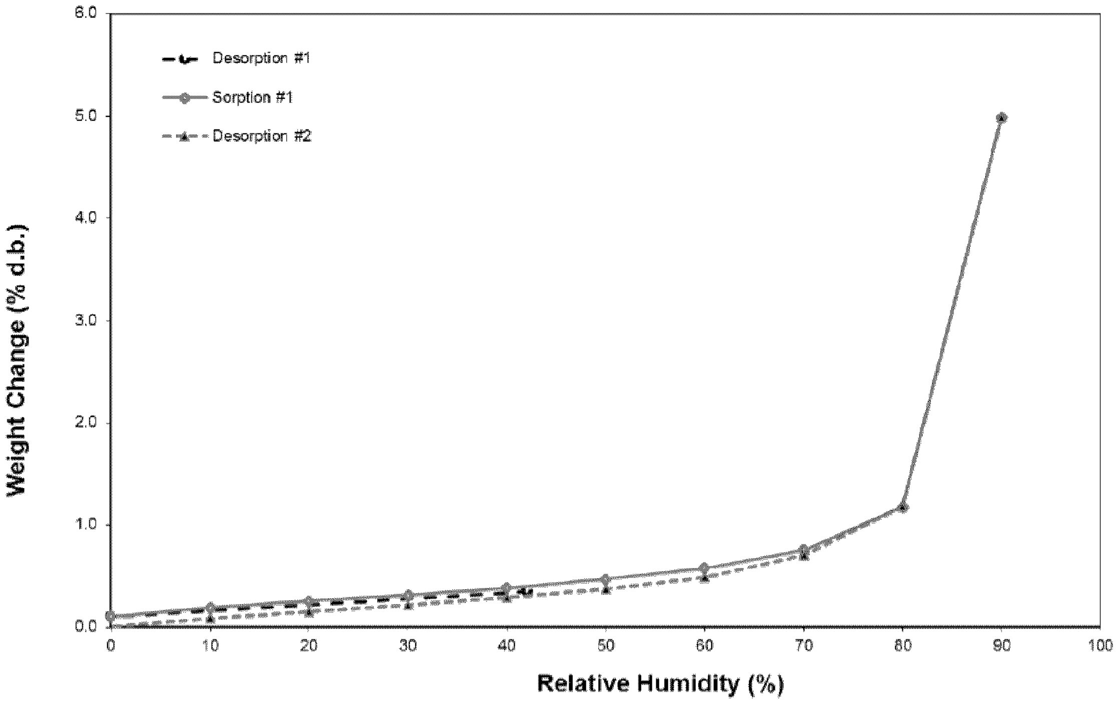
6.0
5.0
4.0
3.0
2.0
1.0
0.0
Desorption #1
Sorption #1
Desorption #2
Weight Change (% d.b.)
0
10
20
30
40
50
60
70
80
90
100
Relative Humidity (%)

**Fig. 5**

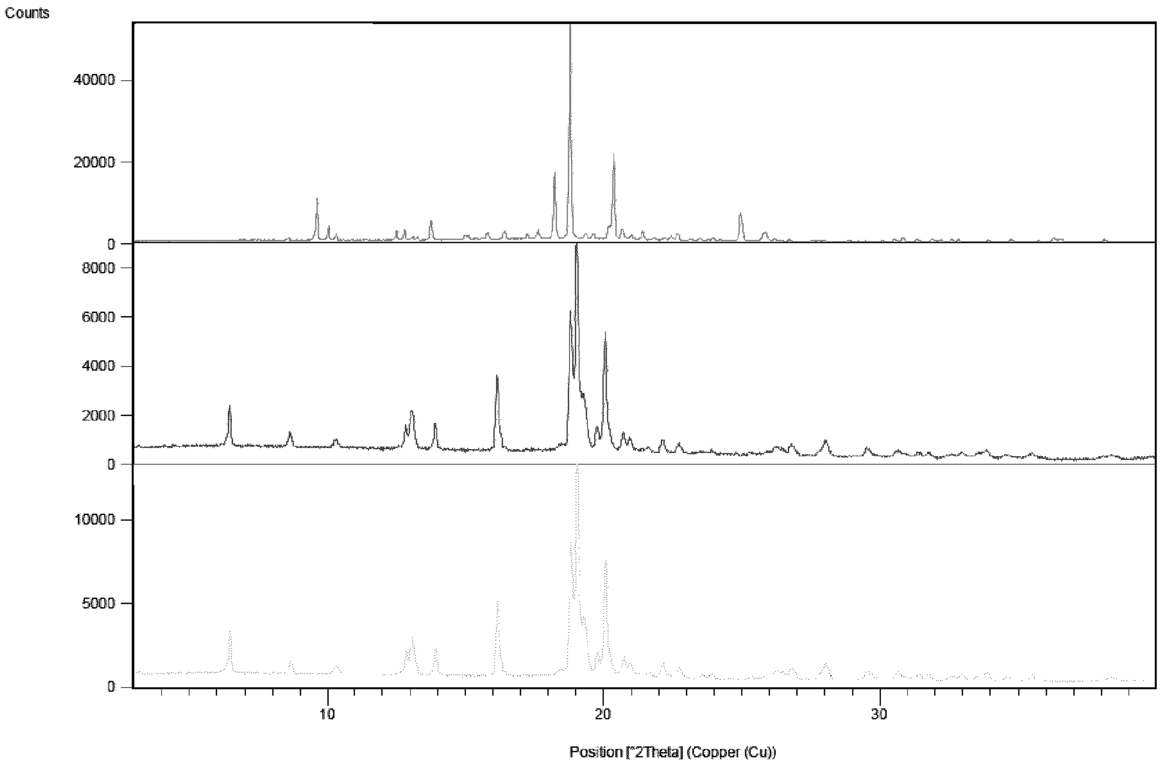
Counts
40000
20000
0
8000
6000
4000
2000
0
10000
5000
0
10
20
30
Position [°2Theta] (Copper (Cu))

**Fig. 6**

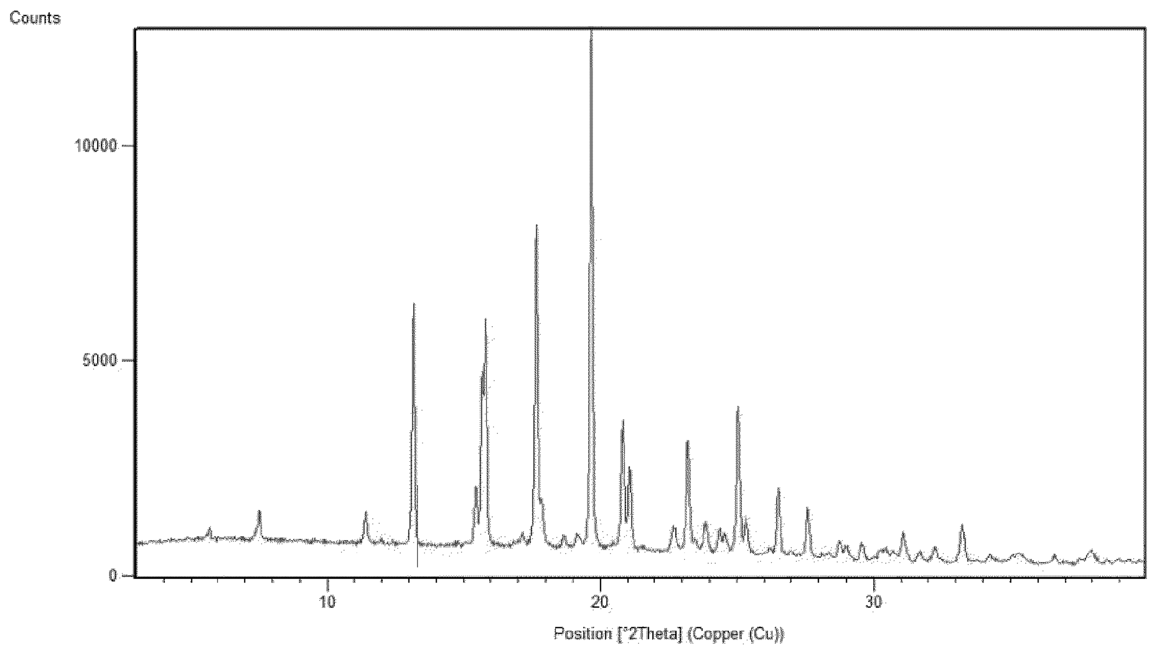


**Fig. 7**

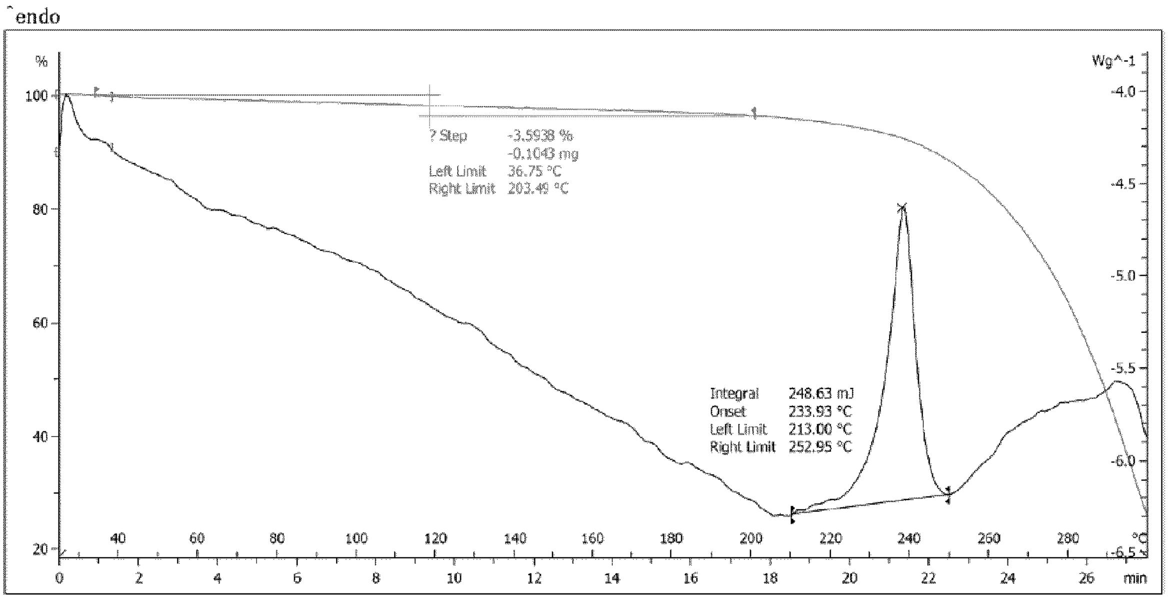
^endo
%
Wg^-1
? Step -3.5938 %
-0.1043 mg
Left Limit 36.75 °C
Right Limit 203.49 °C
Integral 248.63 mJ
Onset 233.93 °C
Left Limit 213.00 °C
Right Limit 252.95 °C
°C
min

**Fig. 8**

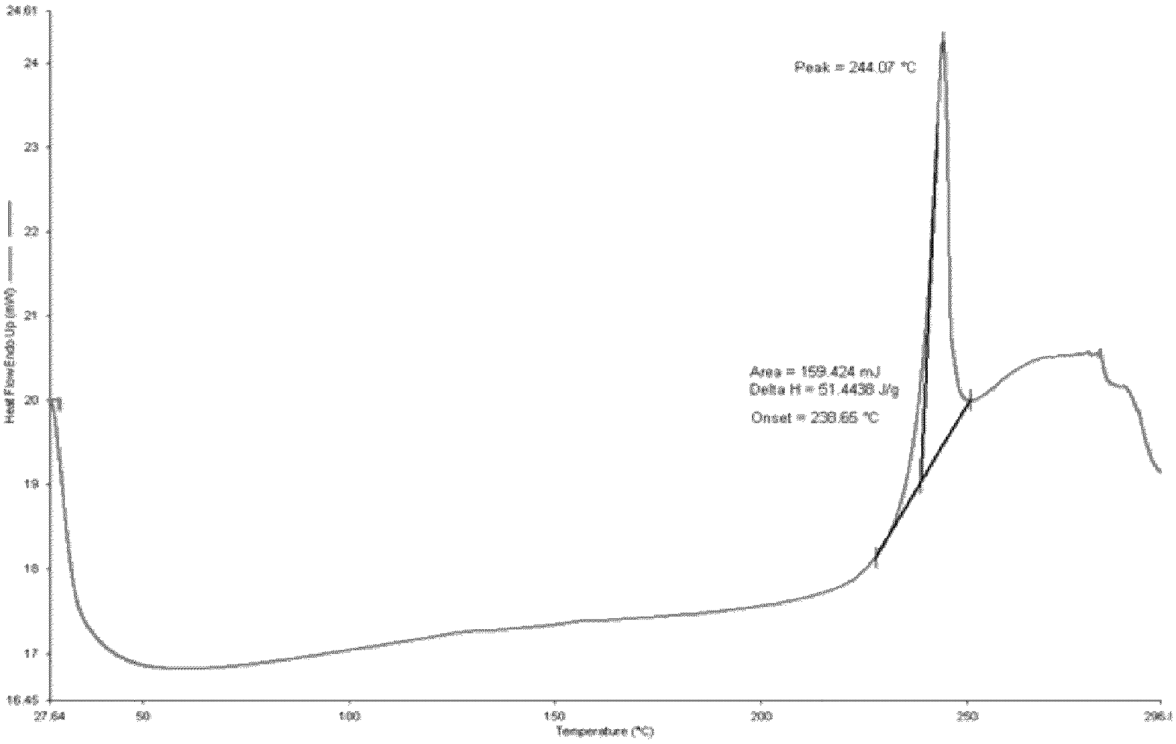
Peak = 244.07 °C
Area = 159.424 mJ
Delta H = 51.4438 J/g
Onset = 238.65 °C
Heat Flow Endo Up (mW)
24.61
24
23
22
21
20
19
18
17
16.45
27.64
50
100
150
200
250
296.1
Temperature (°C)

Fig. 9

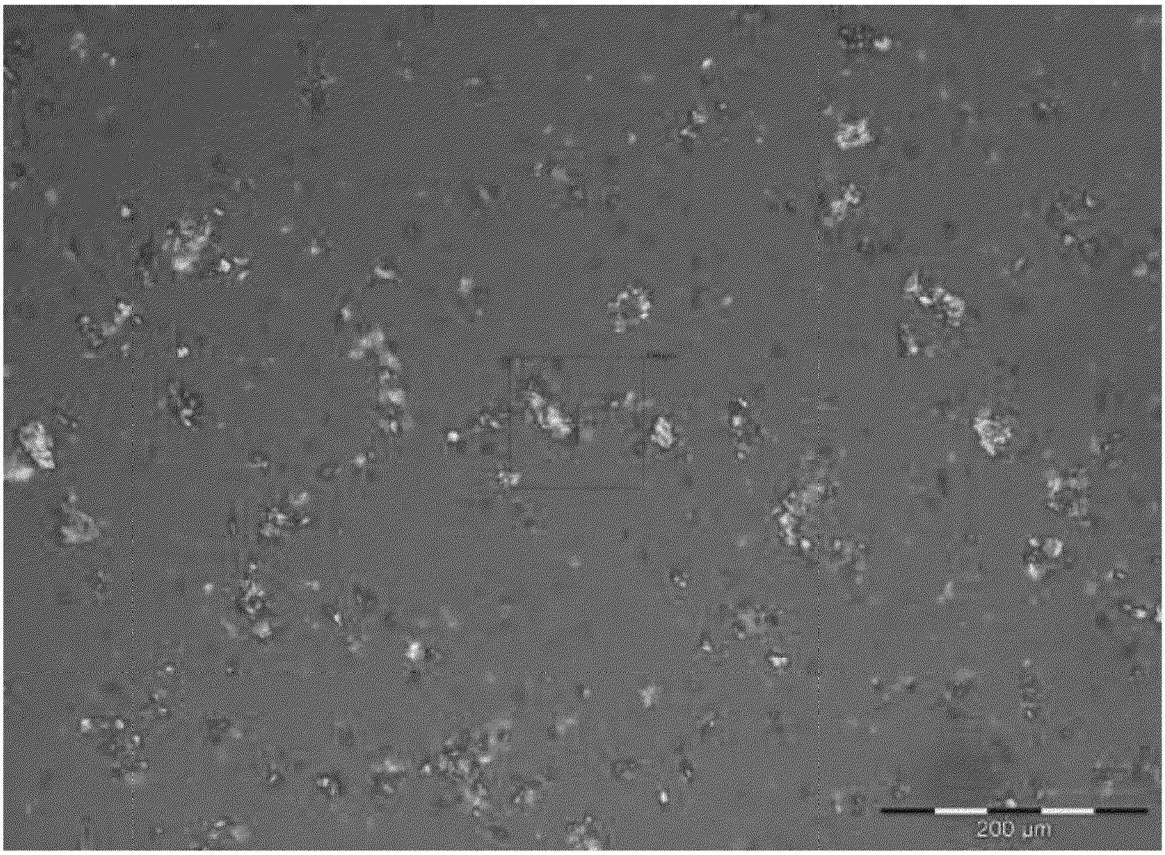


Fig. 10

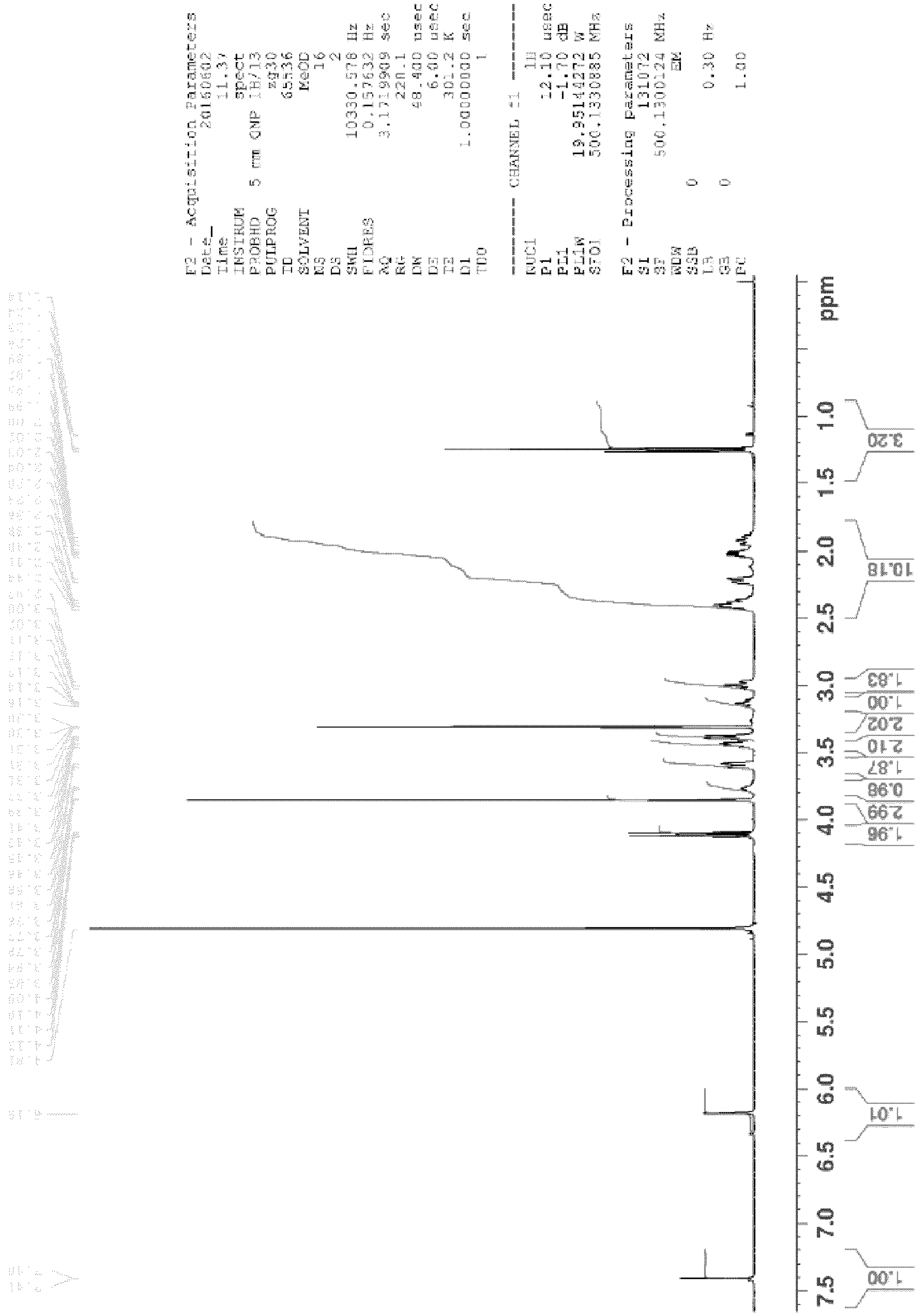
F2 - Acquisition Parameters
Date_ 20160602
Time 11.37
INSTRUM spect
PROBHD 5 mm QNP 1H/13
PULPROG zg30
TD 65536
SOLVENT MeOD
NS 16
DS 2
SWH 10330.578 Hz
FIDRES 0.157632 Hz
AQ 3.1719909 sec
RG 228.1
DW 48.400 usec
DE 6.00 usec
TE 301.2 K
D1 1.00000000 sec
TD0 1
======== CHANNEL f1 ========
NUC1 1H
P1 12.10 usec
PL1 -1.70 dB
PL1W 19.95144272 W
SFO1 500.1330885 MHz
F2 - Processing parameters
SI 131072
SF 500.1300124 MHz
WDW EM
SSB 0
LB 0.30 Hz
GB 0
PC 1.00
1.00
1.01
1.96
2.99
0.98
1.87
2.10
2.02
1.00
1.83
10.18
3.20
7.5
7.0
6.5
6.0
5.5
5.0
4.5
4.0
3.5
3.0
2.5
2.0
1.5
1.0
ppm

Fig. 11

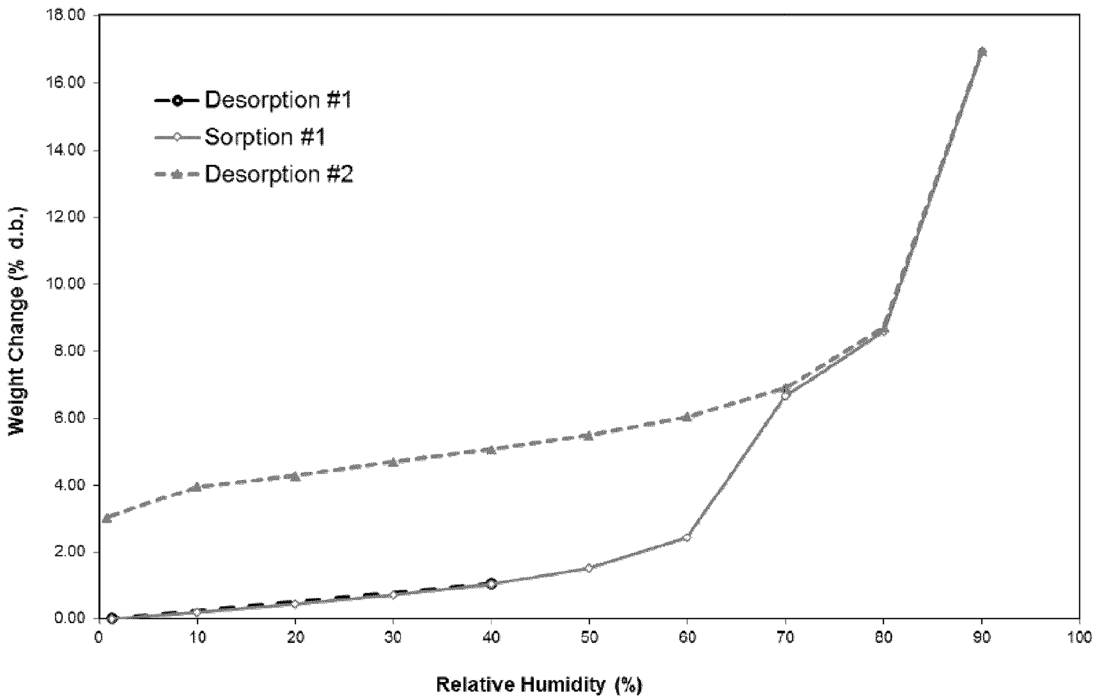
18.00
16.00
14.00
12.00
10.00
8.00
6.00
4.00
2.00
0.00
0
10
20
30
40
50
60
70
80
90
100
Desorption #1
Sorption #1
Desorption #2
Weight Change (% d.b.)
Relative Humidity (%)

Fig. 12

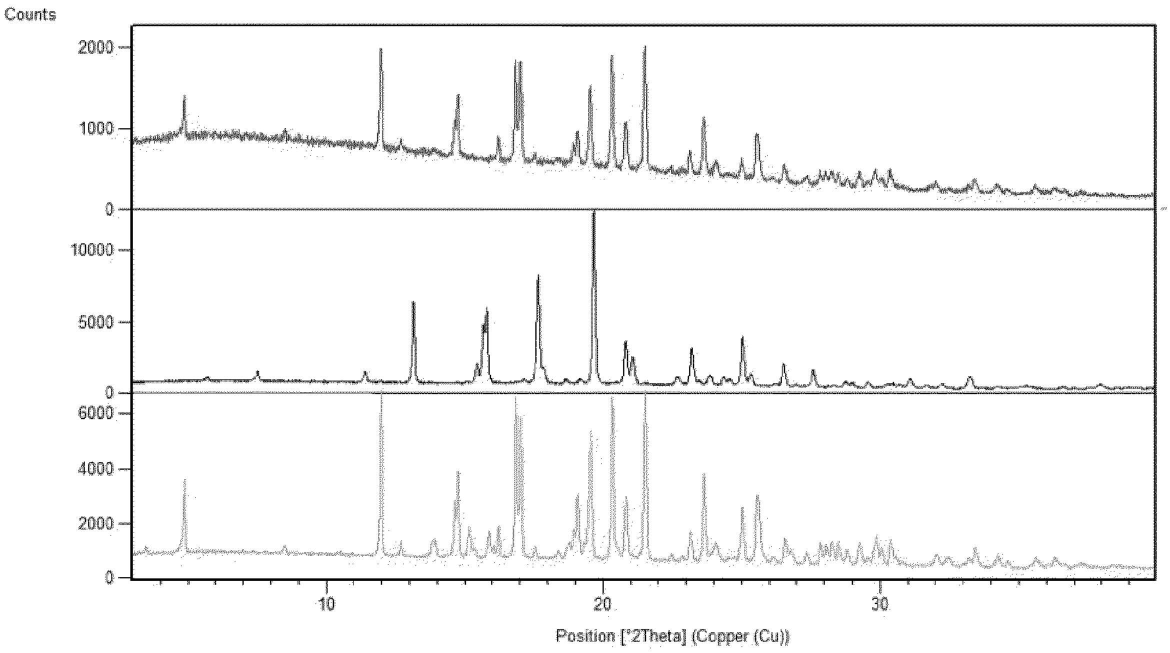
Counts
2000
1000
0
10000
5000
0
6000
4000
2000
0
10
20
30
Position [°2Theta] (Copper (Cu))

**Fig. 13**

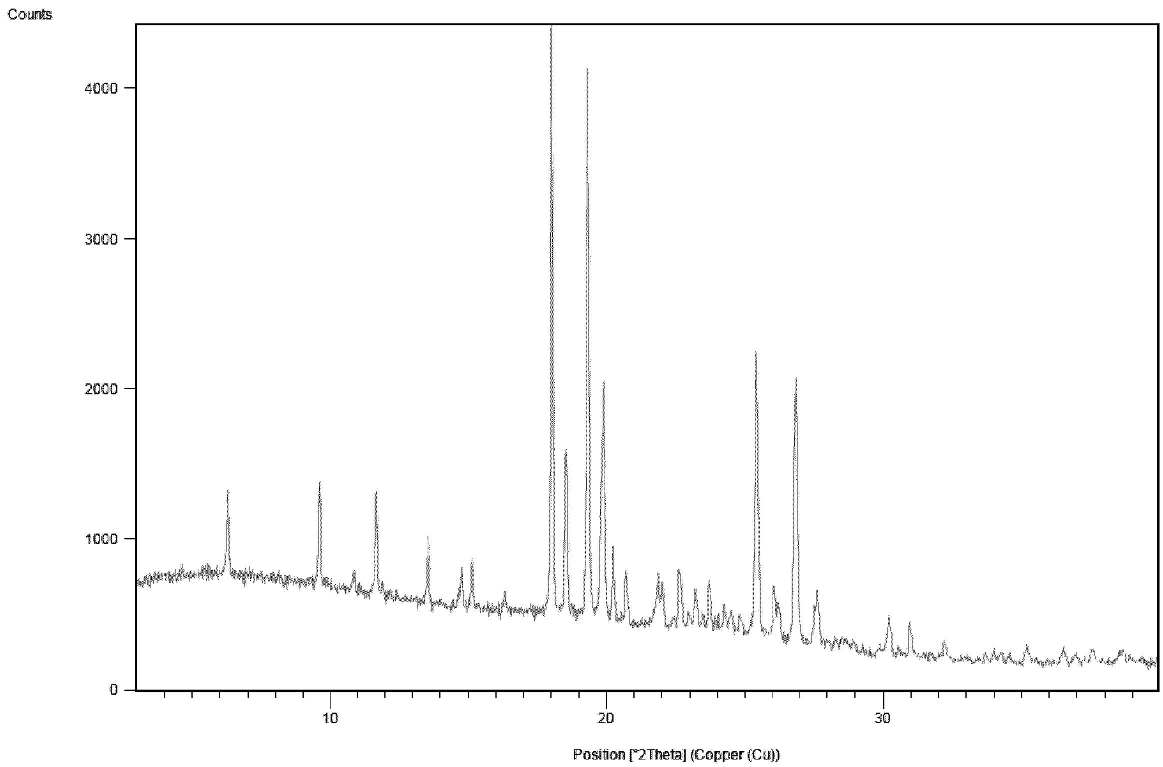
Counts
4000
3000
2000
1000
0
10
20
30
Position [°2Theta] (Copper (Cu))

Fig. 14

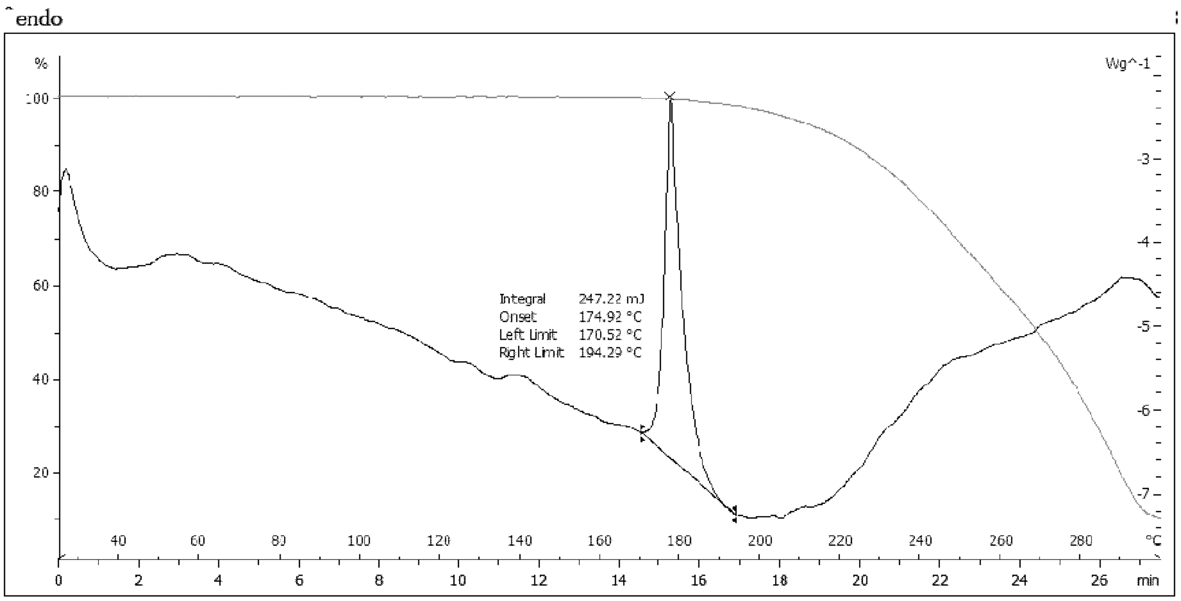
^endo
%
Wg^-1
100
80
60
40
20
-3
-4
-5
-6
-7
Integral 247.22 mJ
Onset 174.92 °C
Left Limit 170.52 °C
Right Limit 194.29 °C
40 60 80 100 120 140 160 180 200 220 240 260 280 °C
0 2 4 6 8 10 12 14 16 18 20 22 24 26 min

**Fig. 15**

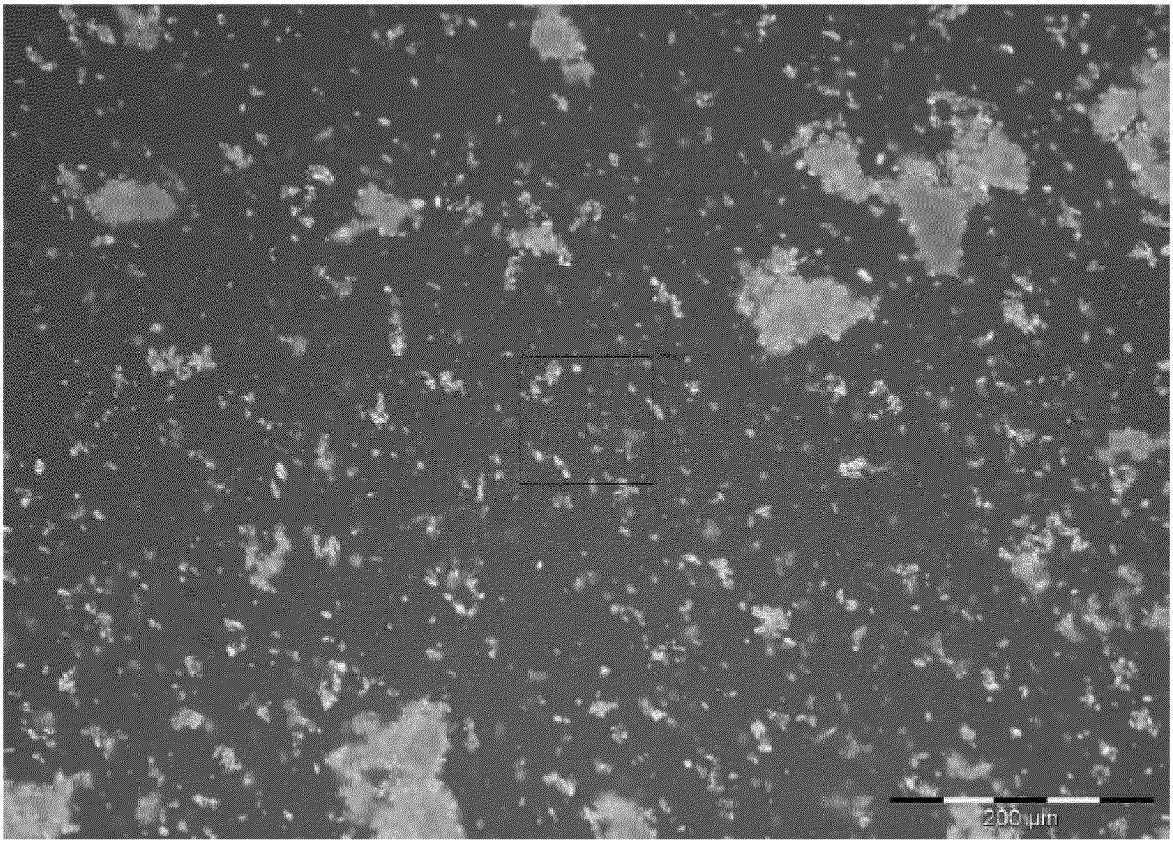


Fig. 16

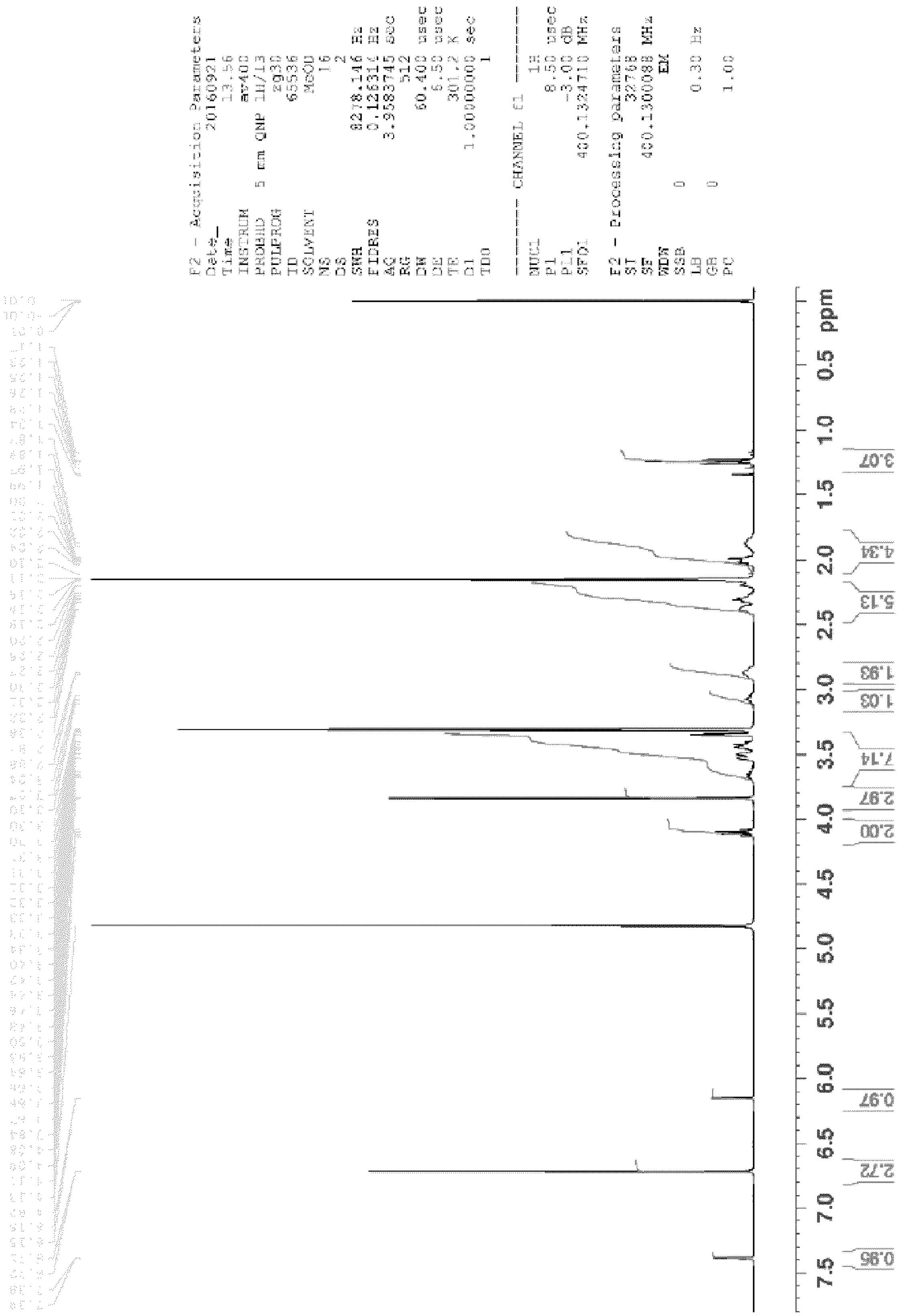
F2 - Acquisition Parameters
Date_ 20160921
Time 13.56
INSTRUM av400
PROBHD 5 mm QNP 1H/13
PULPROG zg30
TD 65536
SOLVENT MeOD
NS 16
DS 2
SWH 8278.146 Hz
FIDRES 0.126314 Hz
AQ 3.9583745 sec
RG 512
DW 60.400 usec
DE 6.50 usec
TE 301.2 K
D1 1.00000000 sec
TD0 1
======== CHANNEL f1 ========
NUC1 1H
P1 8.50 usec
PL1 -3.00 dB
SFO1 400.1324710 MHz
F2 - Processing parameters
SI 32768
SF 400.1300088 MHz
WDW EM
SSB 0
LB 0.30 Hz
GB 0
PC 1.00
ppm
0.5
1.0
1.5
2.0
2.5
3.0
3.5
4.0
4.5
5.0
5.5
6.0
6.5
7.0
7.5
3.07
4.34
5.13
1.93
1.03
7.14
2.97
2.00
0.97
2.72
0.95

Fig. 17

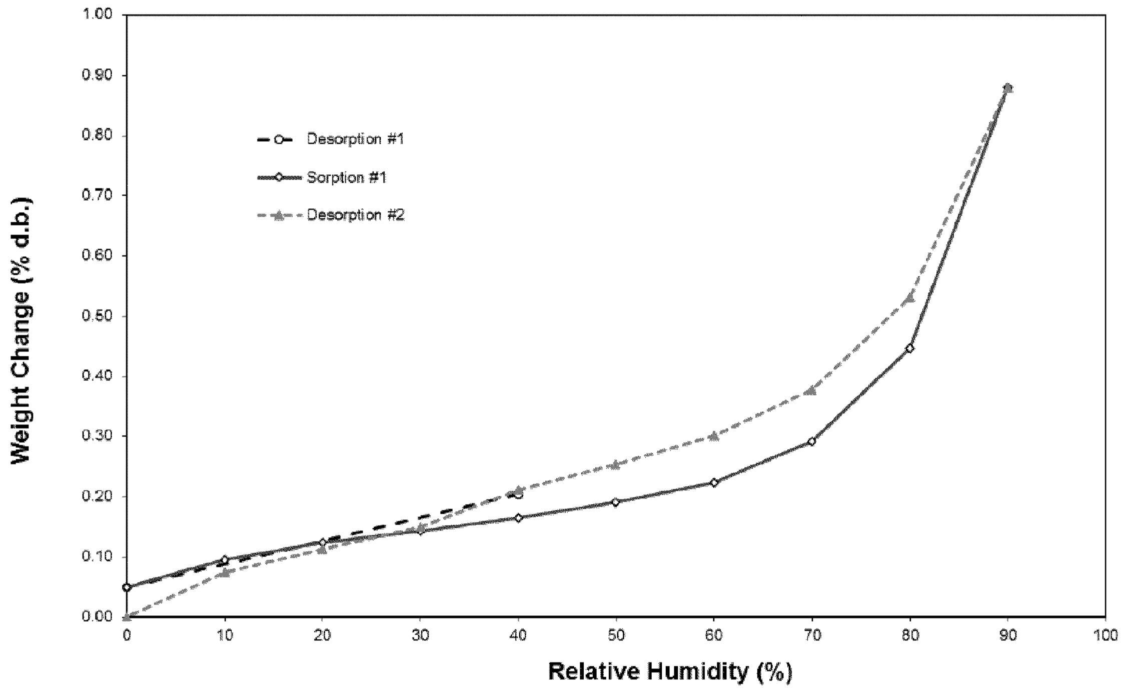

1.00
0.90
0.80
0.70
0.60
0.50
0.40
0.30
0.20
0.10
0.00
Weight Change (% d.b.)
Desorption #1
Sorption #1
Desorption #2
0
10
20
30
40
50
60
70
80
90
100
Relative Humidity (%)


Fig. 18

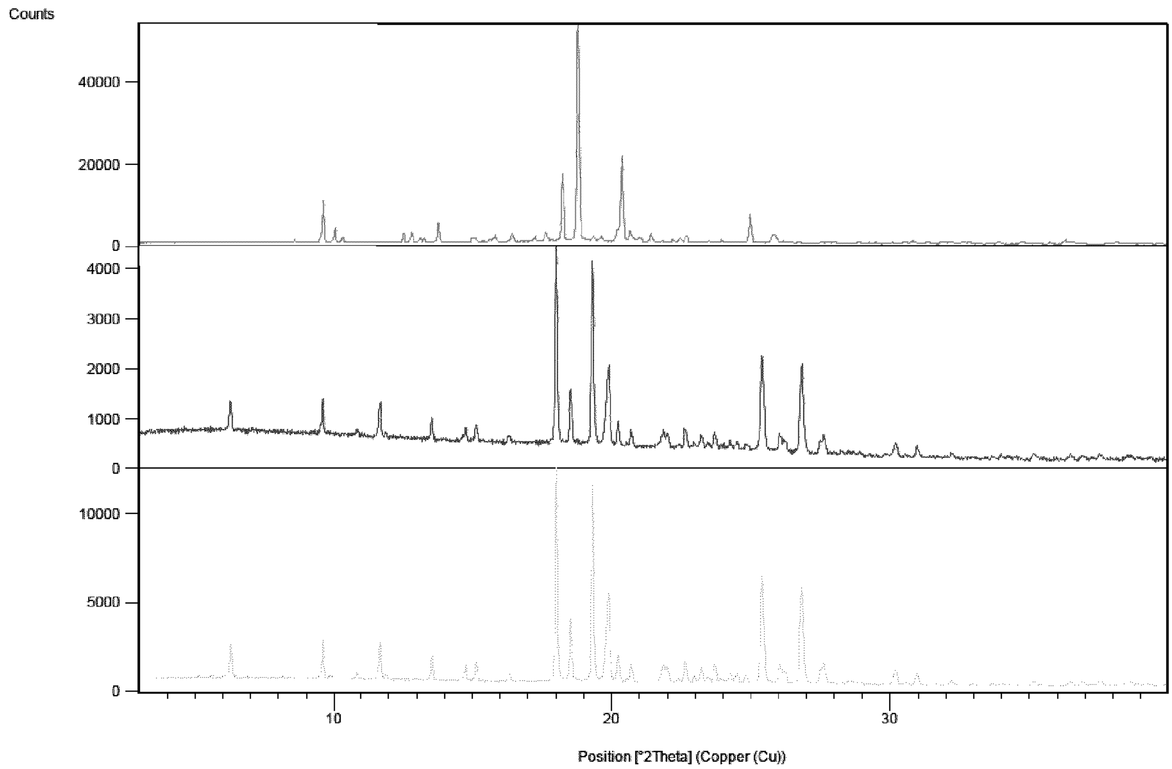
Counts
40000
20000
0
4000
3000
2000
1000
0
10000
5000
0
10
20
30
Position [°2Theta] (Copper (Cu))

**Fig. 19**

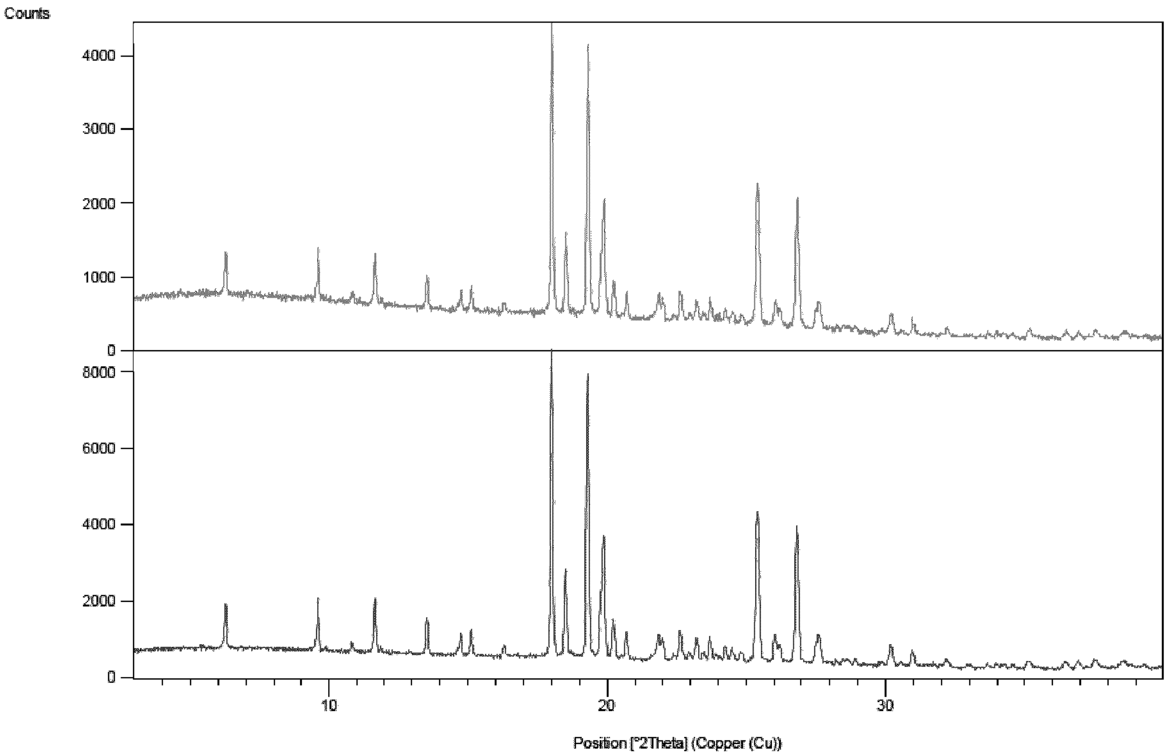
Counts
4000
3000
2000
1000
0
8000
6000
4000
2000
0
10
20
30
Position [°2Theta] (Copper (Cu))

**Fig. 20**

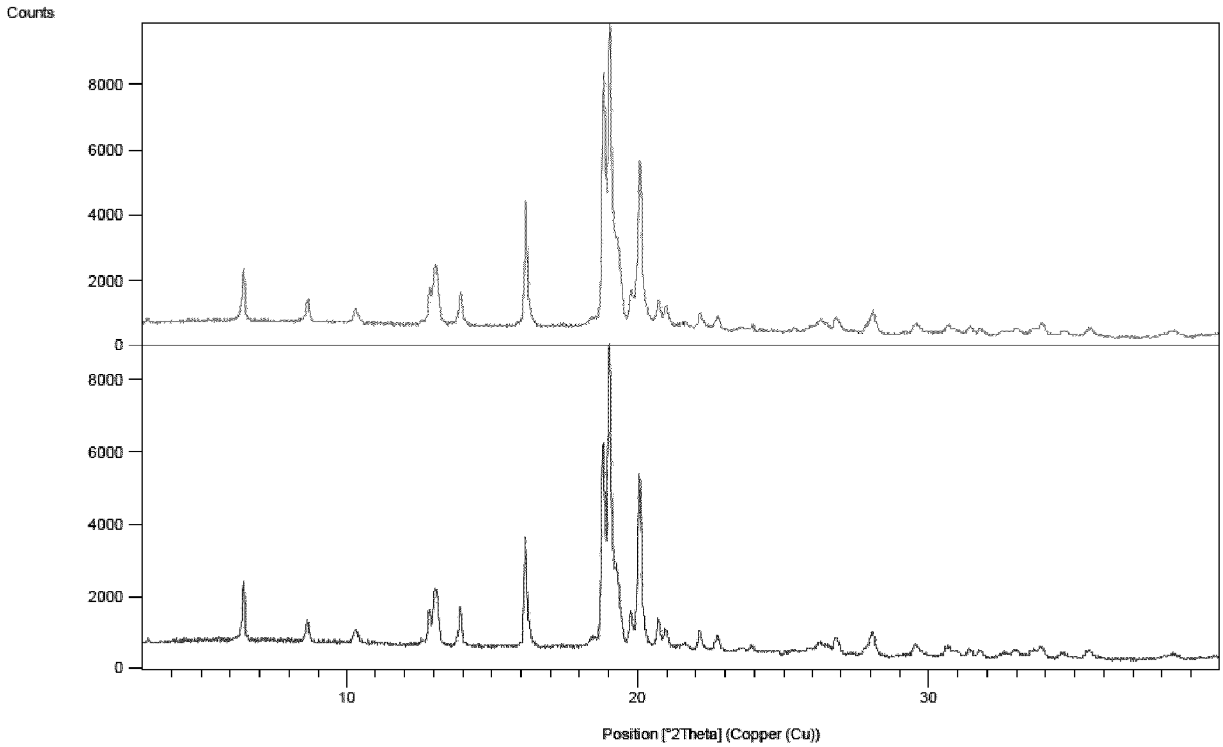
Counts
8000
6000
4000
2000
0
8000
6000
4000
2000
0
10
20
30
Position [°2Theta] (Copper (Cu))

Fig. 21

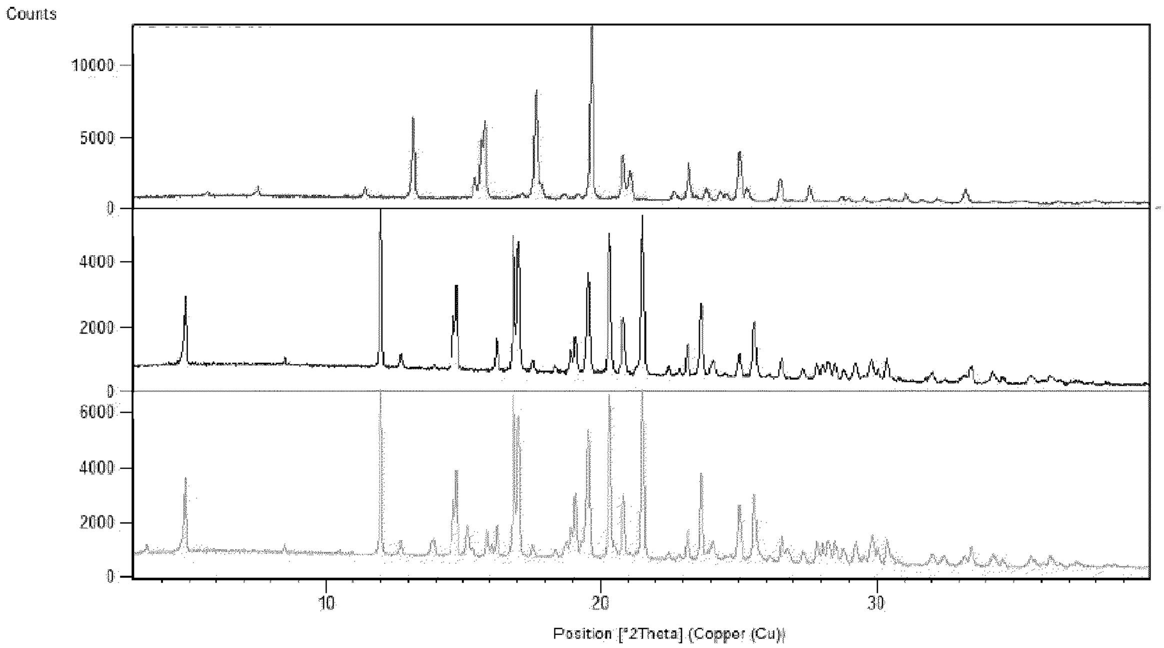
Counts
10000
5000
0
4000
2000
0
6000
4000
2000
0
10
20
30
Position [°2Theta] (Copper (Cu))

Fig. 22

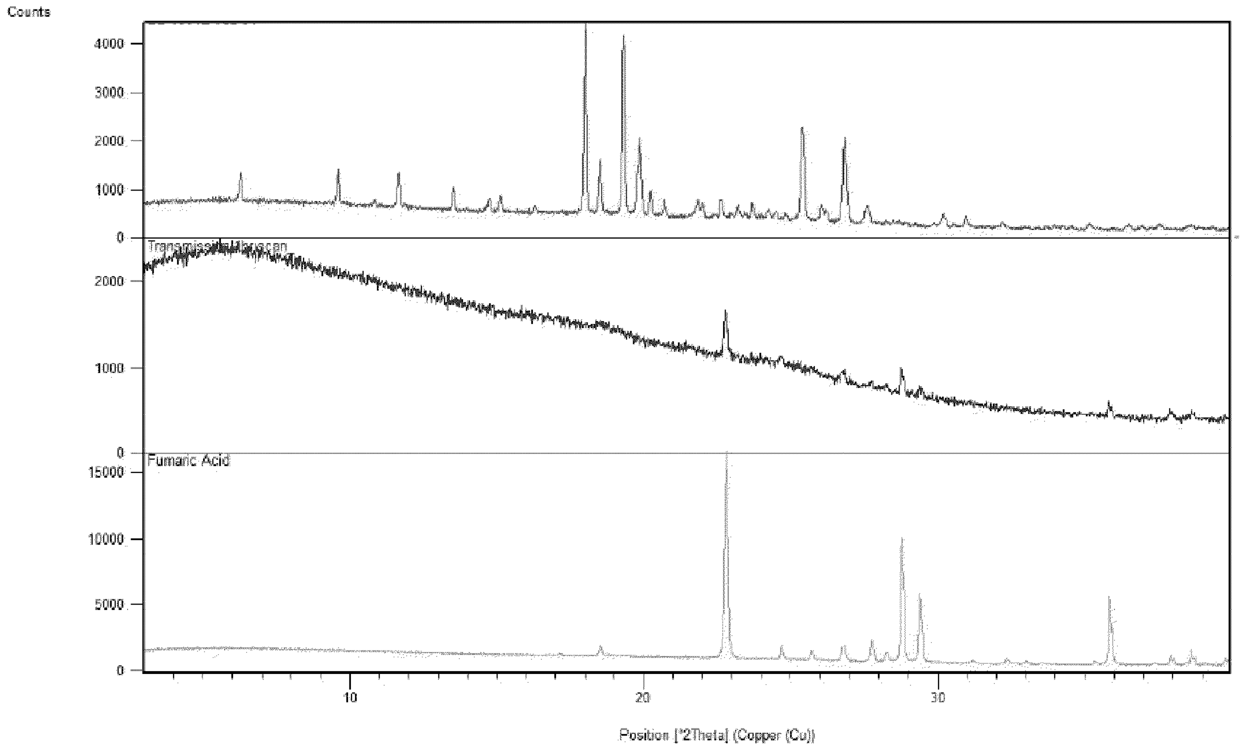
Counts
4000
3000
2000
1000
0
Transmission Hiroscan
2000
1000
0
Fumaric Acid
15000
10000
5000
0
10
20
30
Position [°2Theta] (Copper (Cu))

Fig. 23

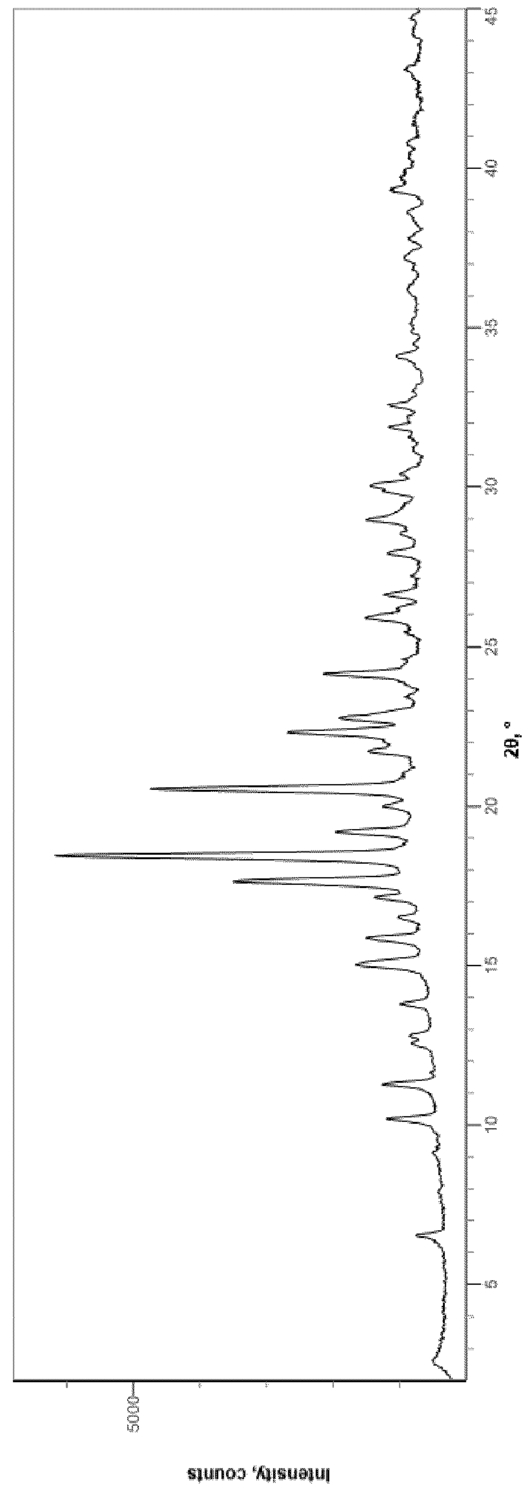


Fig. 25

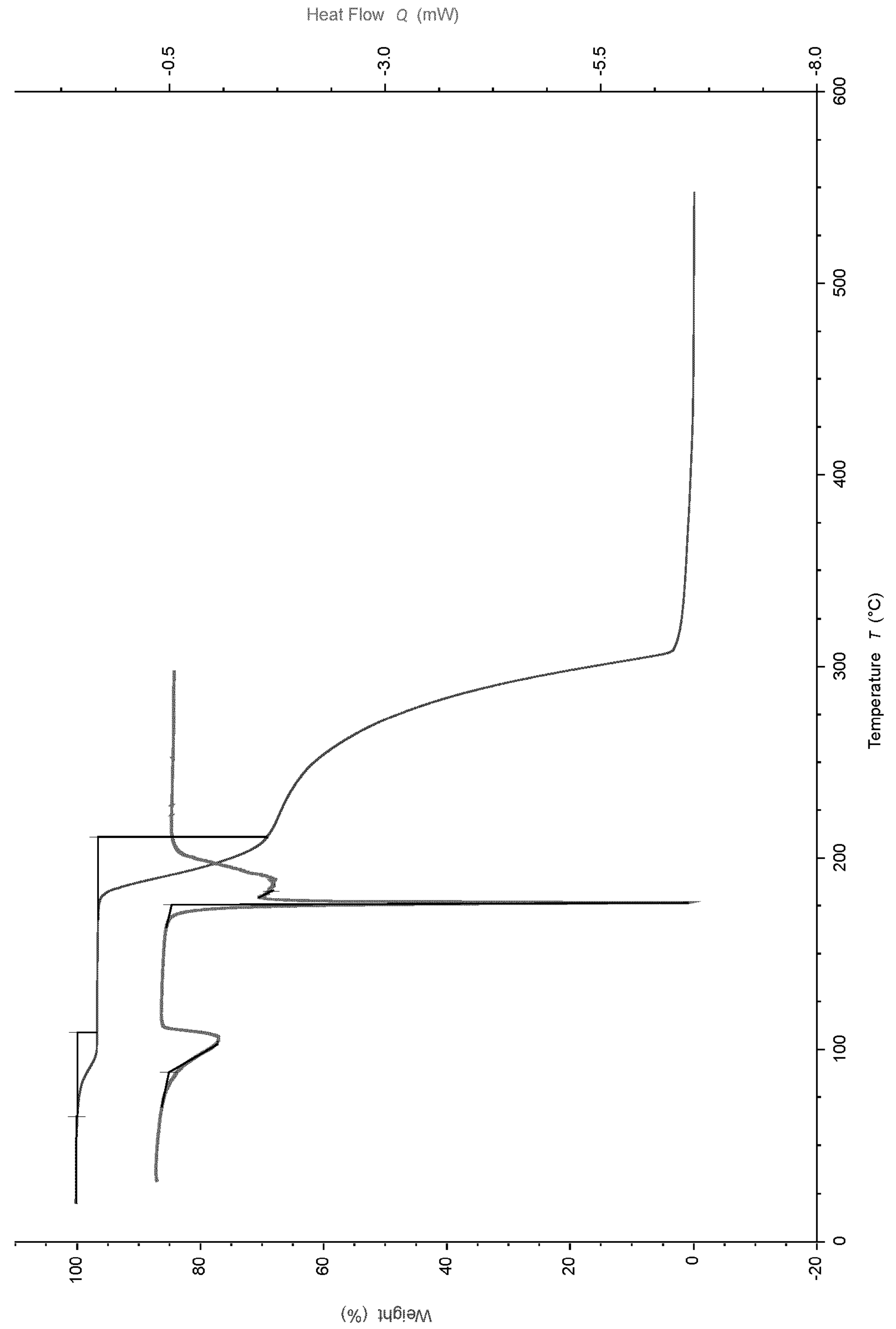
Heat Flow Q (mW)
-0.5
-3.0
-5.5
-8.0
Weight (%)
100
80
60
40
20
0
-20
Temperature T (°C)
0
100
200
300
400
500
600

**Fig. 26**

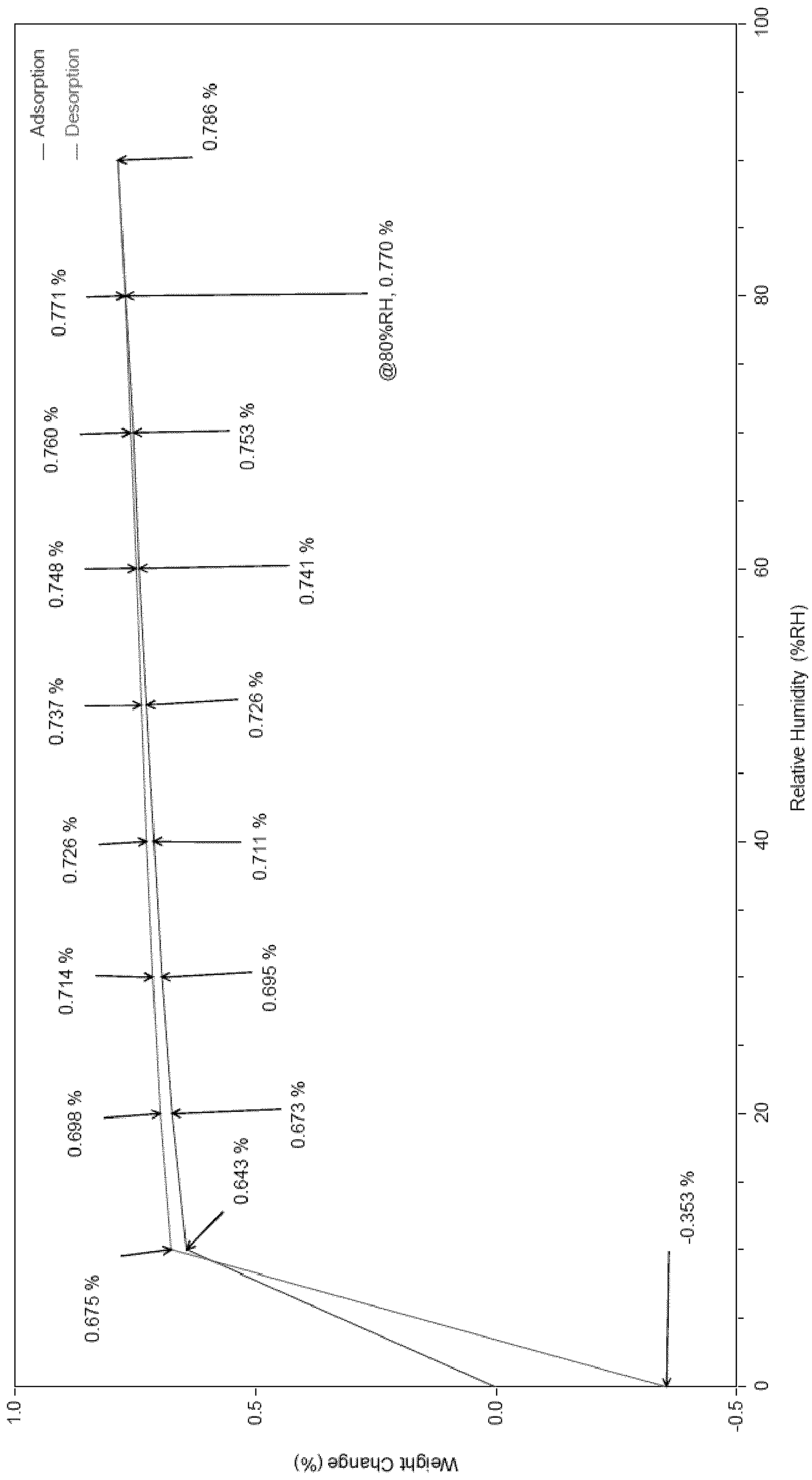
Adsorption
Desorption
0.786 %
@80%RH, 0.770 %
0.771 %
0.760 %
0.753 %
0.748 %
0.741 %
0.737 %
0.726 %
0.726 %
0.711 %
0.714 %
0.695 %
0.698 %
0.673 %
0.643 %
0.675 %
-0.353 %
Weight Change (%)
Relative Humidity (%RH)
1.0
0.5
0.0
-0.5
0
20
40
60
80
100

**Fig. 27**

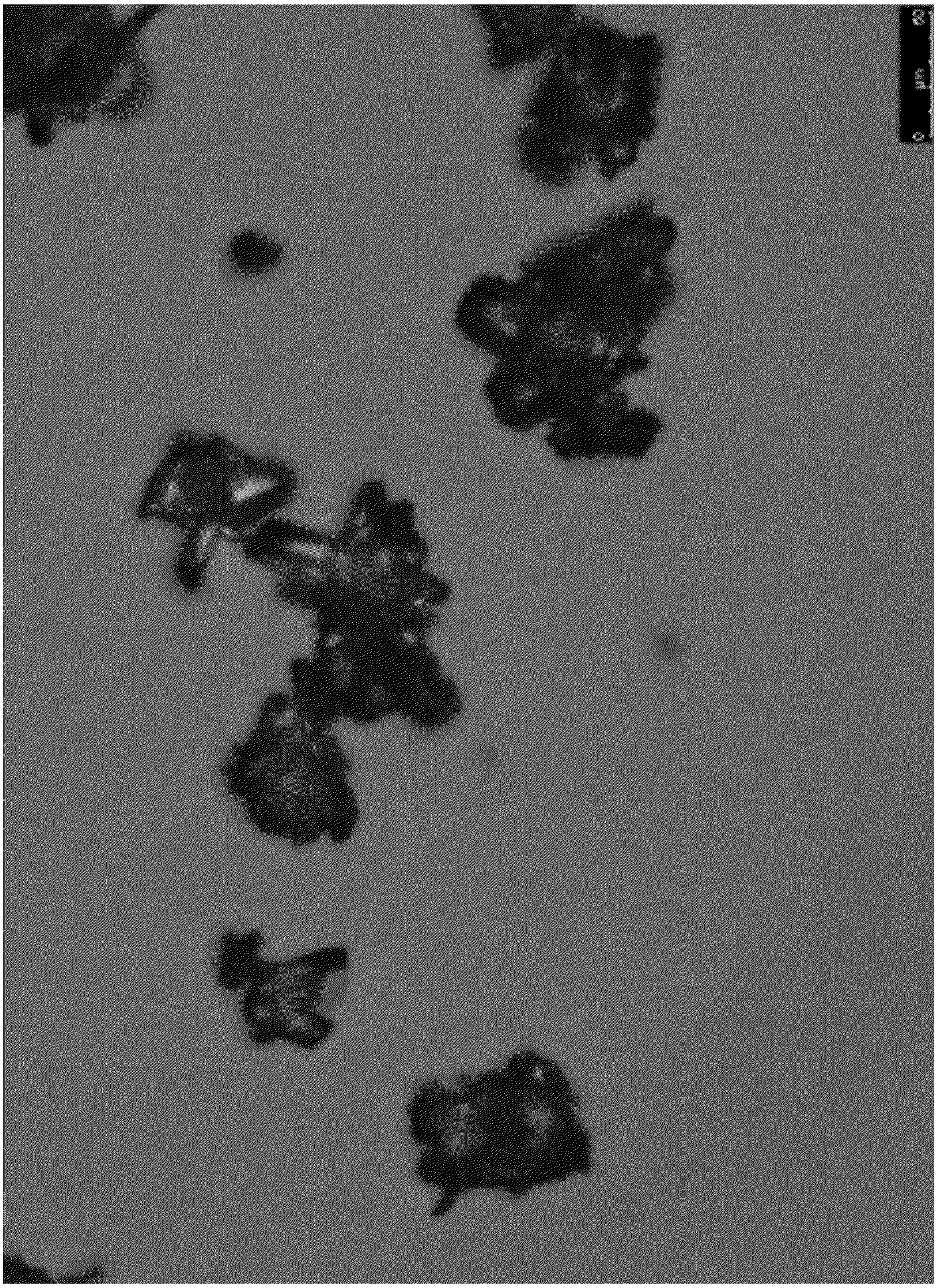

Fig. 28

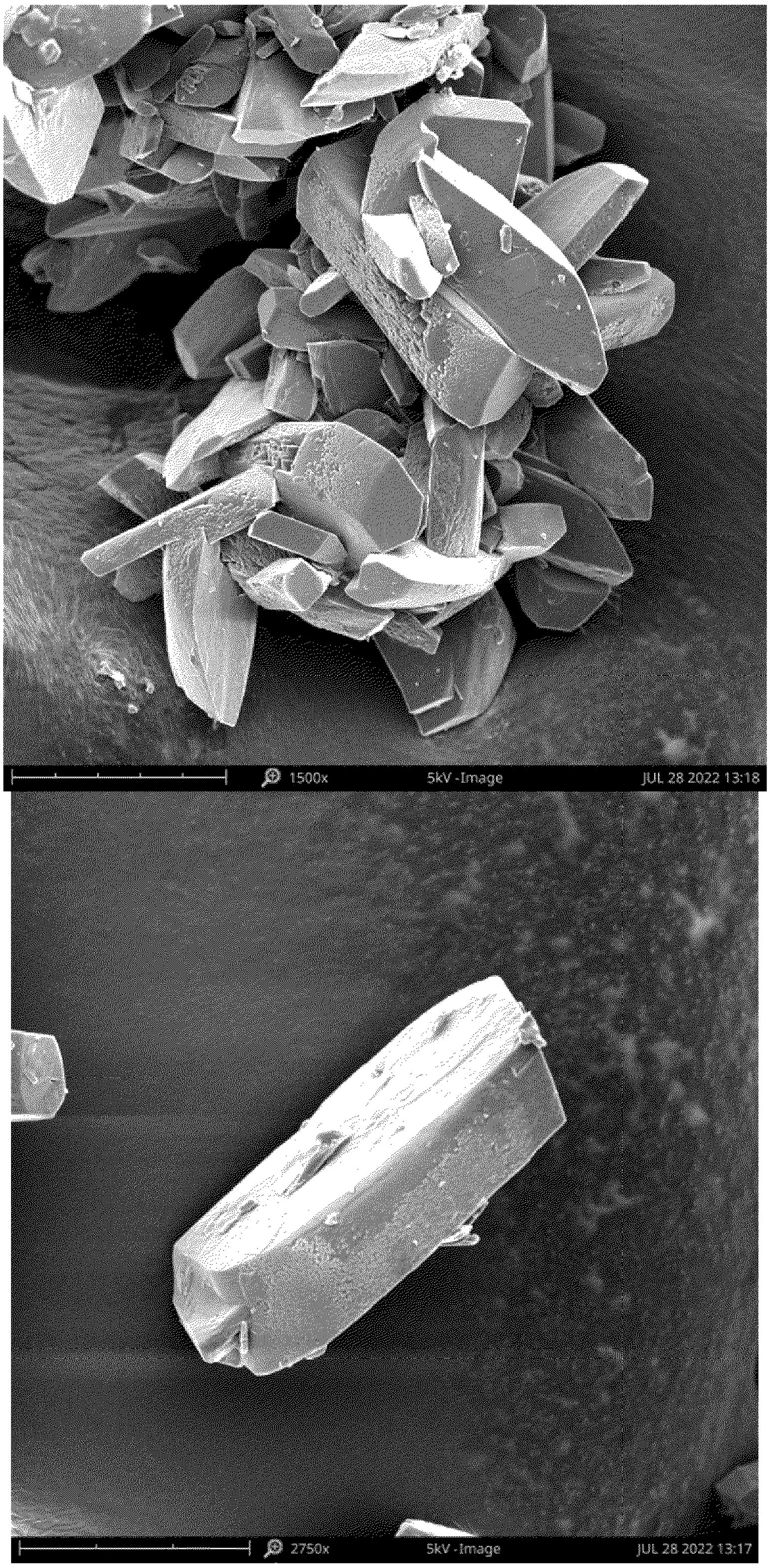
1500x
5kV -Image
JUL 28 2022 13:18
2750x
5kV -Image
JUL 28 2022 13:17

Fig. 29

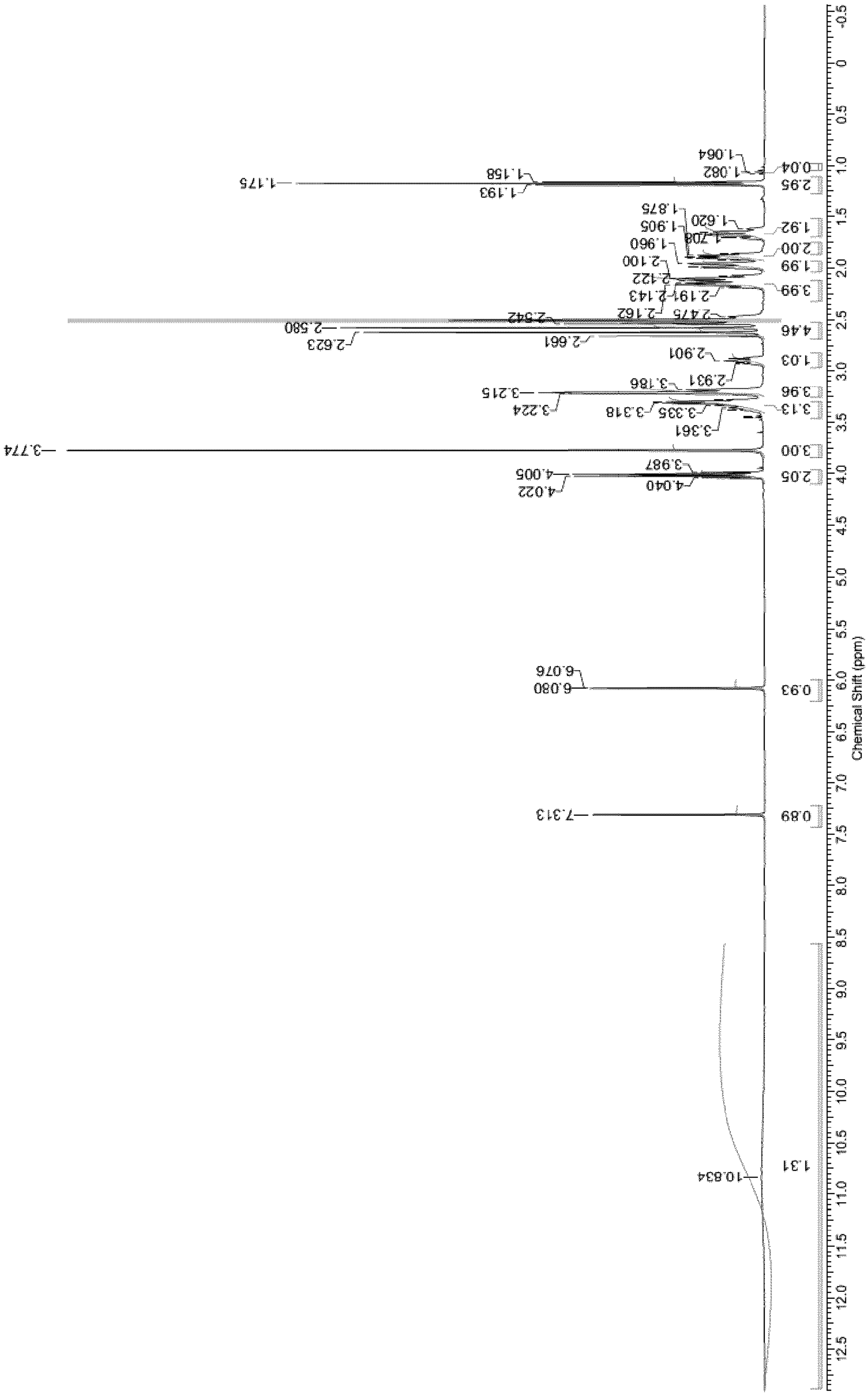
Chemical Shift (ppm)
-0.5 0 0.5 1.0 1.5 2.0 2.5 3.0 3.5 4.0 4.5 5.0 5.5 6.0 6.5 7.0 7.5 8.0 8.5 9.0 9.5 10.0 10.5 11.0 11.5 12.0 12.5
1.064
1.082
1.158
1.175
1.193
1.620
1.708
1.875
1.905
1.960
2.100
2.122
2.143
2.162
2.191
2.475
2.542
2.580
2.623
2.661
2.901
2.931
3.186
3.215
3.224
3.318
3.335
3.361
3.774
3.987
4.005
4.022
4.040
6.076
6.080
7.313
10.834
0.04
2.95
1.92
2.00
1.99
3.99
4.46
1.03
3.96
3.13
3.00
2.05
0.93
0.89
1.31

Fig. 30

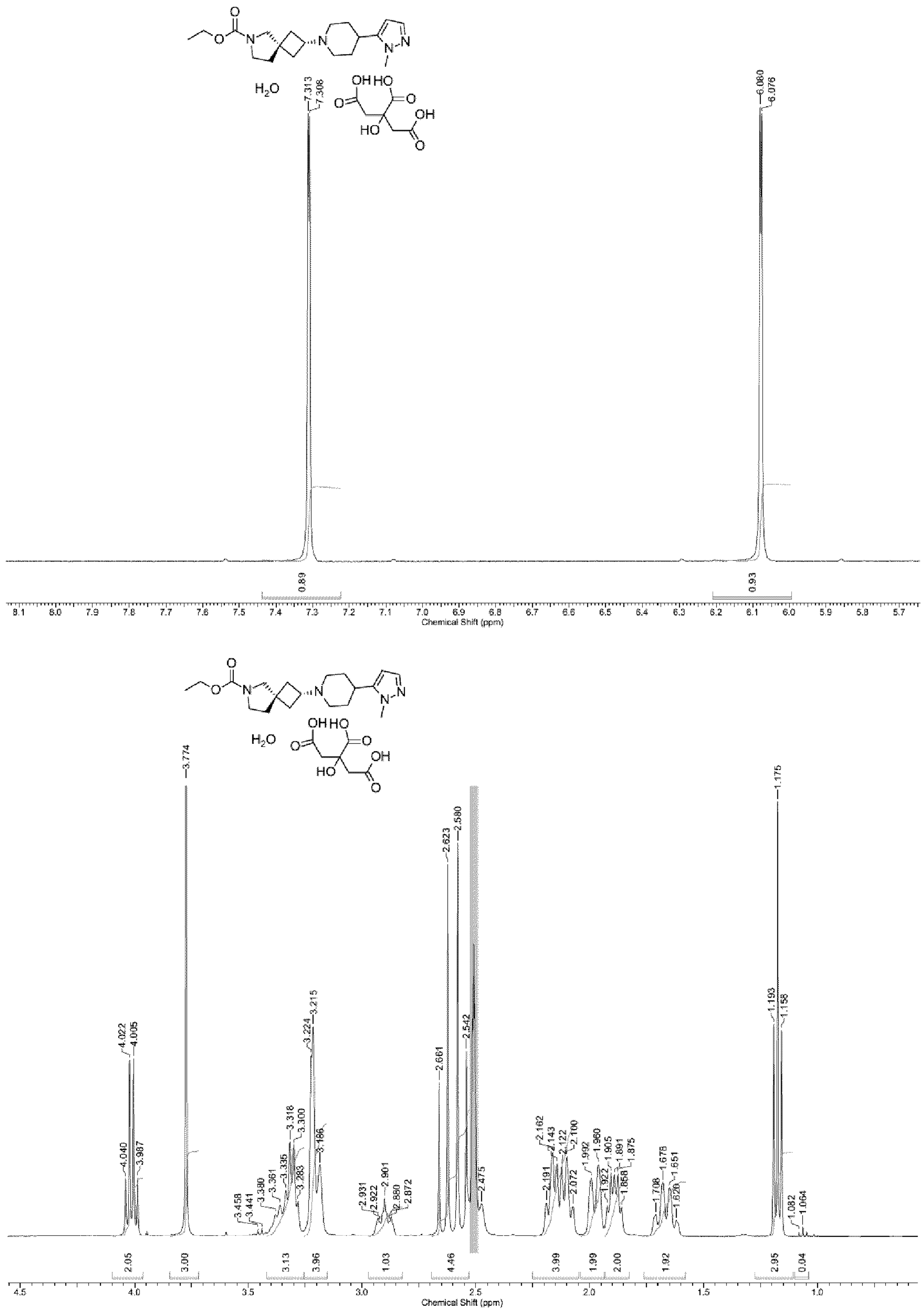


**Fig. 31**

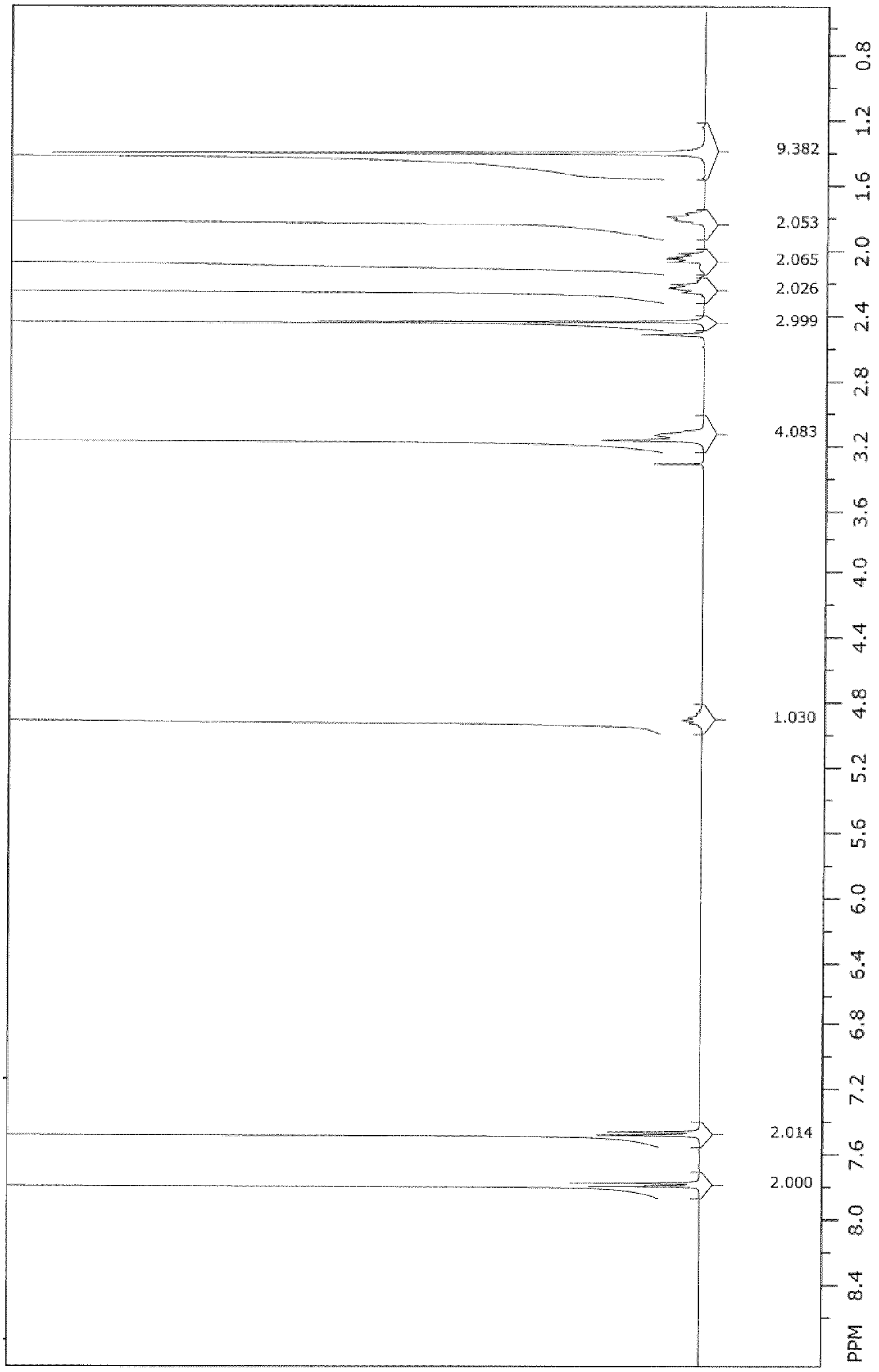
9.382
2.053
2.065
2.026
2.999
4.083
1.030
2.014
2.000
0.8
1.2
1.6
2.0
2.4
2.8
3.2
3.6
4.0
4.4
4.8
5.2
5.6
6.0
6.4
6.8
7.2
7.6
8.0
8.4
PPM

Fig. 34

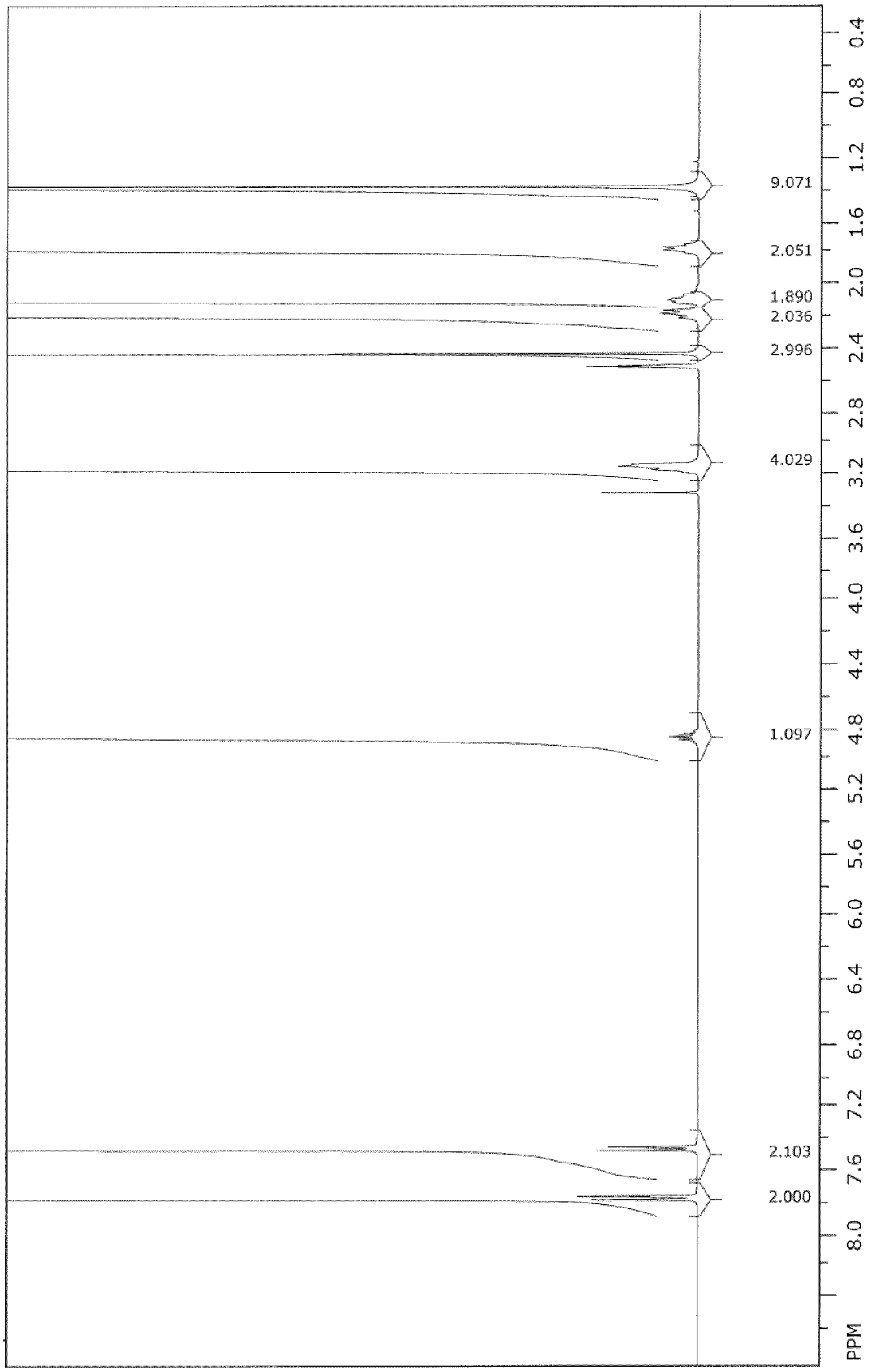
9.071
2.051
1.890
2.036
2.996
4.029
1.097
2.103
2.000
0.4
0.8
1.2
1.6
2.0
2.4
2.8
3.2
3.6
4.0
4.4
4.8
5.2
5.6
6.0
6.4
6.8
7.2
7.6
8.0
PPM

Fig. 35

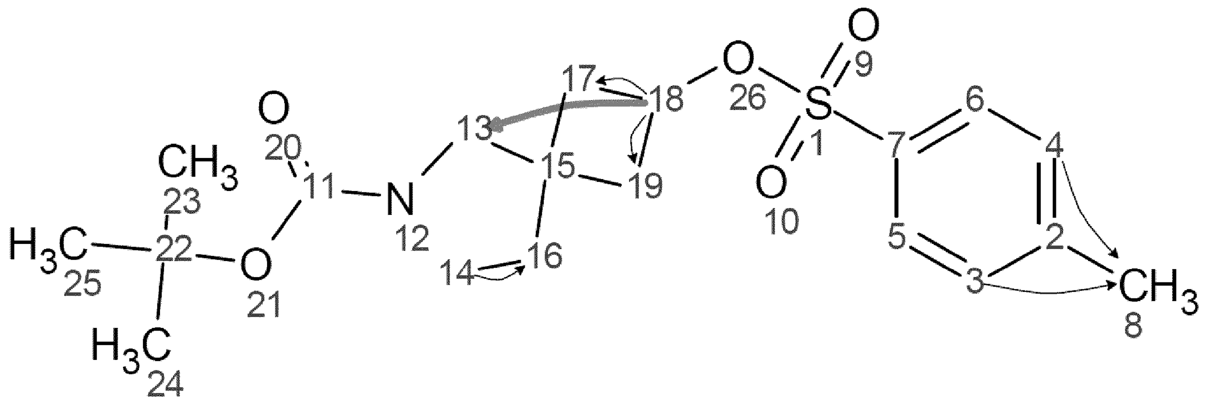

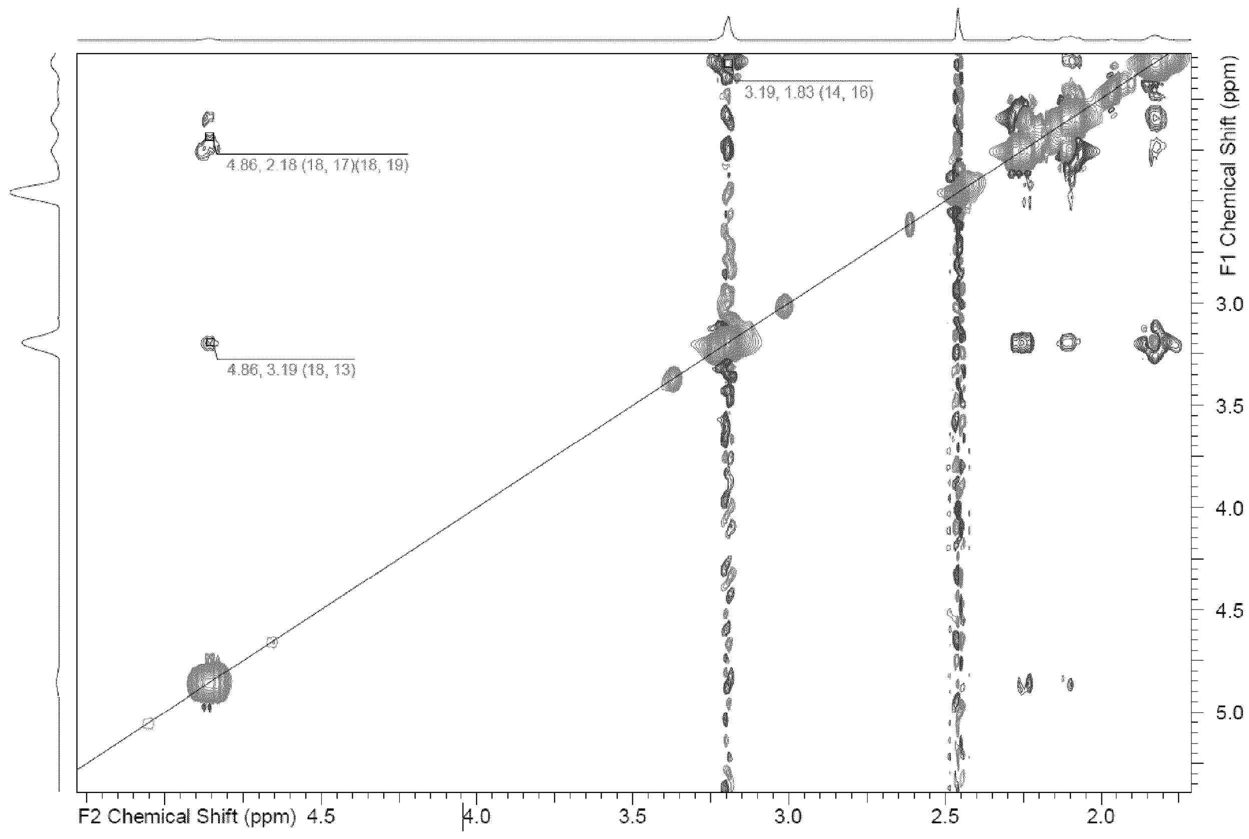
3.19, 1.83 (14, 16)
4.86, 2.18 (18, 17)(18, 19)
4.86, 3.19 (18, 13)
F1 Chemical Shift (ppm)
F2 Chemical Shift (ppm)
4.5
4.0
3.5
3.0
2.5
2.0
5.0

Fig. 36

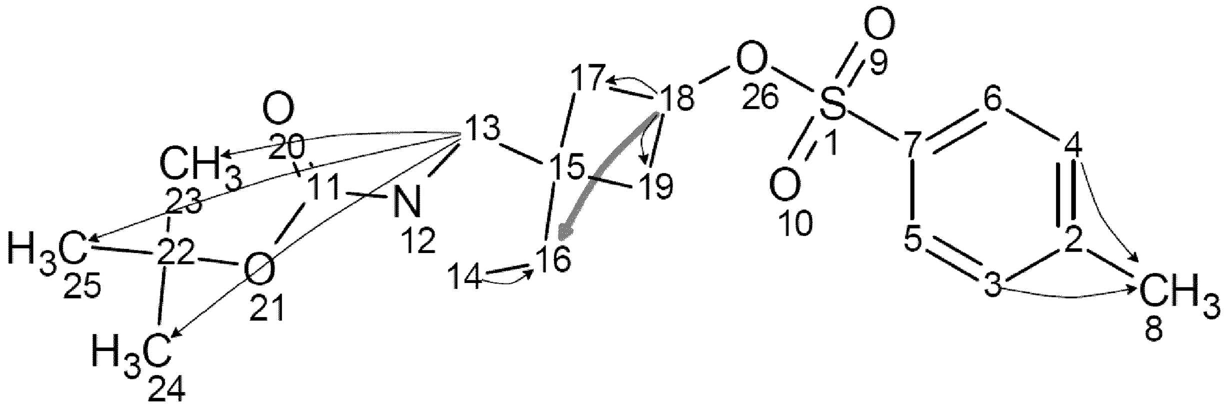

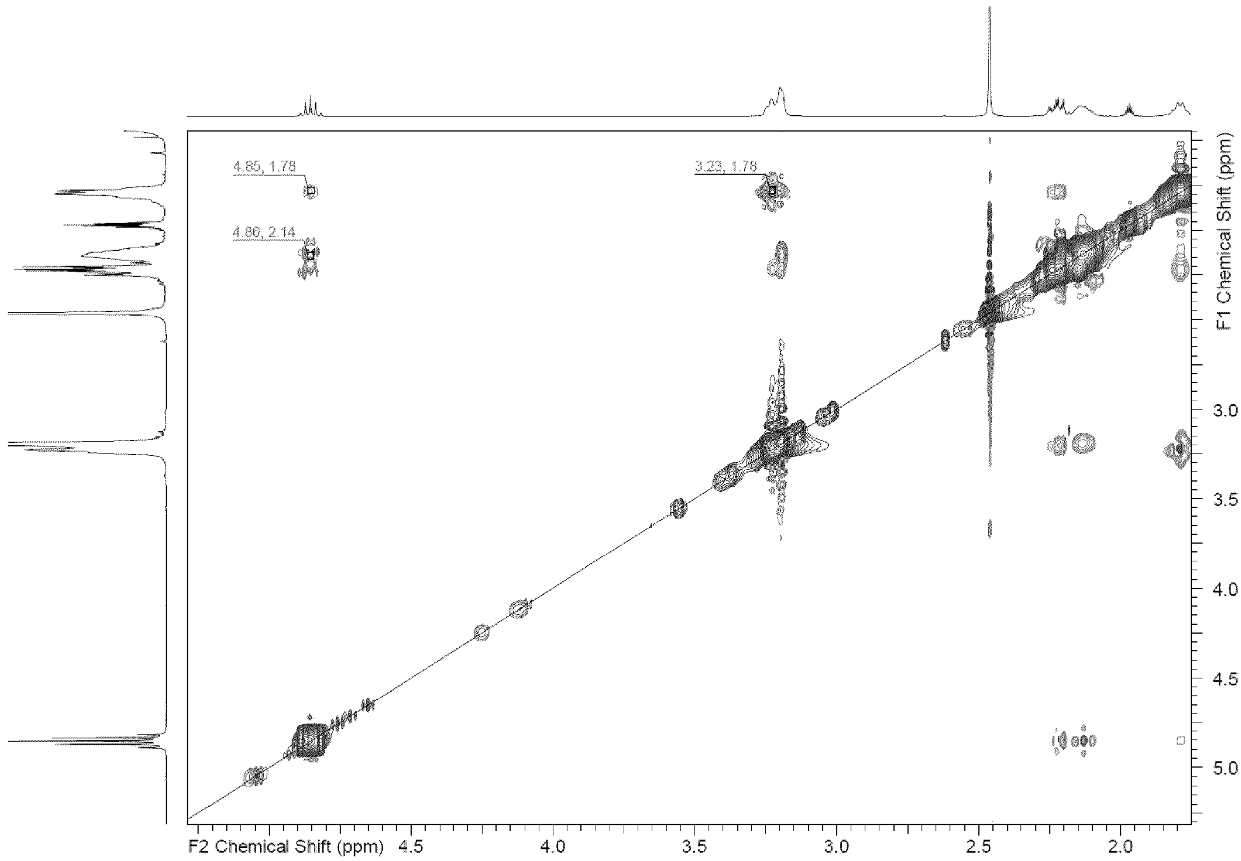
4.85, 1.78
4.86, 2.14
3.23, 1.78
F2 Chemical Shift (ppm)
4.5
4.0
3.5
3.0
2.5
2.0
F1 Chemical Shift (ppm)
3.0
3.5
4.0
4.5
5.0

**Fig. 37**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015118342 A
- WO 2015118342 A1


### Non-patent literature cited in the description


- **LANGMEAD et al.** *Br J Pharmacol*, 2008
- **WHITEHOUSE et al.** *Science*, 1982
- **DEAN et al.** *Mol Psychiatry*, 2002
- **CACCAMO et al.** *Neuron*, 2006
- **NITSCH et al.** *Neurol*, 2000
- **MIRZA et al.** *CNS Drug Rev*, 1999
- **SCHMIDT et al.** *Psychopharmacology*, August 2011, vol. 216 (3), 367-78
- **BODICK et al.** *Arch Neurol*, 1997
- **SHEKHAR et al.** *Am J Psych*, 2008
- **BRIDGES et al.** *Bioorg Med Chem Lett*, 2008
- **JOHNSON et al.** *Bioorg Med Chem Lett*, 2010
- **BUDZIK et al.** *ACS Med Chem Lett*, 2010
- *CHEMICAL ABSTRACTS*, 1803346-98-6
- *CHEMICAL ABSTRACTS*, 2920742-36-3
- Compendium of Chemical Terminology. **A. D. MCNAUGHT** ; **A. WILKINSON.** IUPAC. Blackwell Scientific Publications, 1997
- Pharmaceutical Salts: Properties, Selection, and Use. August 2002, 388
- **BERGE et al.** Pharmaceutically Acceptable Salts. *J. Pharm. Sci.*, 1977, vol. 66, 1-19
- **JERRY MARCH**. Advanced Organic Chemistry. John Wiley & Sons, 1992, 109-114
- **CAHN** ; **INGOLD** ; **PRELOG**. *Angew. Chem. Int. Ed. Engl.*, 1966, vol. 5, 385-415
- **BRYN et al.** Solid-State Chemistry of Drugs. SSCI, Inc of West Lafayette, 1999
- **C. GIACOVAZZO** ; **H. L. MONACO** ; **D. VITERBO** ; **F. SCORDARI** ; **G. GILLI** ; **G. ZANOTTI** ; **M. CATTI**. International Union of Crystallography. Oxford University Press
- **HANCOCK et al.** *J. Pharm. Sci*, 1997, vol. 86, 1
- Fiesers' Reagents for Organic Synthesis. John Wiley, vol. 1-17
- **T. GREENE** ; **P. WUTS**. Protective Groups in Organic Synthesis. John Wiley and Sons, 1999
- **SAMBROOK et al.** Molecular Cloning: A Laboratory Manual. Cold Spring Harbor Laboratory Press, 2001
- Current Protocols in Molecular Biology. Greene Pub. Associates, 1998
- **VILLA-KAMAROFF et al.** *Proc. Natl Acad. Sci. USA*, 1978, vol. 75, 3727-3731
- **DEBOER et al.** *Proc. Natl Acad. Sci. USA*, 1983, vol. 80, 21-25
- Useful proteins from recombinant bacteria. *Scientific American*, 1980, vol. 242, 74-94
- *Nature*, 2020, https://doi.org/10.1038/s42003-020-0939-8
- **BEAUCAGE et al.** *Tet Lett*, 1981, vol. 22, 1859-69
- **MATTHES et al.** *EMBO J.*, 1984, vol. 3, 801-05
- Remington's Pharmaceutical Sciences. Mack Publishing Company
- **CROUCH** ; **OSMOND**. *Comb. Chem. High Throughput Screen*, 2008